# EUROPEAN PATENT APPLICATION

(11) **EP 2 230 300 A1**
(43) Date of publication of application: **22.09.2010**
(21) Application number: 08842029.4
(22) Date of filing: 23.10.2008
(51) Int. Cl.: C12N 15/09, A01K 67/027, A61K 38/00, A61K 39/395, C07K 16/18, C07K 16/24, C07K 16/28, C07K 16/30, C12N 1/15, C12N 1/19, C12N 1/21, C12N 5/10, C12P 21/08

(54) **POLYPEPTIDE HAVING ENHANCED EFFECTOR FUNCTION**

(30) Priority: 24.10.2007 JP 2007276935
(71) Applicant: Otsuka Chemical Holdings Co., Ltd., Osaka-shi, Osaka 540-0021 (JP)
(72) Inventor: TSUJI, Takashi, Nagareyama-shi Chiba 270-0111 (JP); KAJIHARA, Yasuhiro, Yokohama-shi Kanagawa 224-0014 (JP); TEZUKA, Katsunari, Tokushima-shi Tokushima 771-0193 (JP); NAMBU, Yuri, Tokushima-shi Tokushima 771-0193 (JP); FUKAE, Kazuhiro, Tokushima-shi Tokushima 771-0193 (JP); ASAI, Hiroaki, Tokushima-shi Tokushima 771-0193 (JP)
(74) Representative: Flaccus, Rolf-Dieter
(86) International application number: PCT/JP2008/069189
(87) International publication number: WO 2009/054435

(57) **Abstract**

Disclosed is a polypeptide having an enhanced effector function. Specifically disclosed are: a polypeptide having a modified Fc region; a nucleic acid encoding the polypeptide; a vector carrying the nucleic acid; a host cell or a host organism harboring the vector; a pharmaceutical composition comprising the polypeptide; a method for producing the polypeptide; a method for enhancing the effector function of an antibody; and a method for producing a cell capable of producing an antibody having a high effector function.

SEQUENCE LISTING

<110> OTSUKA CHEMICAL CO., LTD.

<120> Polypeptide having enhanced effector functions

<130> PCT2341OC

<140> EP08842029.4
<141> 2008-10-23

<150> JP 2007-276935
<151> 2007-10-24

<160> 153

<170> PatentIn version 3.3

<210> 1
<211> 220
<212> PRT
<213> Artificial

<220>
<223> Glu293Cys Fc amino acid

<400> 1

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Cys Glu Gln Tyr Asn Ser Thr Tyr Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys Gly Gln Pro Arg Glu Pro Gln Val Tyr Thr Leu Pro Pro Ser Arg
        115                 120                 125

Asp Glu Leu Thr Lys Asn Gln Val Ser Leu Thr Cys Leu Val Lys Gly
    130                 135                 140

Phe Tyr Pro Ser Asp Ile Ala Val Glu Trp Glu Ser Asn Gly Gln Pro
145                 150                 155                 160

Glu Asn Asn Tyr Lys Thr Thr Pro Pro Val Leu Asp Ser Asp Gly Ser
                165                 170                 175

Phe Phe Leu Tyr Ser Lys Leu Thr Val Asp Lys Ser Arg Trp Gln Gln
            180                 185                 190

Gly Asn Val Phe Ser Cys Ser Val Met His Glu Ala Leu His Asn His
        195                 200                 205

Tyr Thr Gln Lys Ser Leu Ser Leu Ser Pro Gly Lys
    210                 215                 220

<210>  2
<211>  220
<212>  PRT
<213>  Artificial

<220>
<223>  Glu294Cys Fc amino acid

<400>  2

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Glu Cys Gln Tyr Asn Ser Thr Tyr Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys Gly Gln Pro Arg Glu Pro Gln Val Tyr Thr Leu Pro Pro Ser Arg
        115                 120                 125

Asp Glu Leu Thr Lys Asn Gln Val Ser Leu Thr Cys Leu Val Lys Gly
    130                 135                 140

Phe Tyr Pro Ser Asp Ile Ala Val Glu Trp Glu Ser Asn Gly Gln Pro
145                 150                 155                 160

Glu Asn Asn Tyr Lys Thr Thr Pro Pro Val Leu Asp Ser Asp Gly Ser
                165                 170                 175

Phe Phe Leu Tyr Ser Lys Leu Thr Val Asp Lys Ser Arg Trp Gln Gln
            180                 185                 190

Gly Asn Val Phe Ser Cys Ser Val Met His Glu Ala Leu His Asn His
        195                 200                 205

Tyr Thr Gln Lys Ser Leu Ser Leu Ser Pro Gly Lys
    210                 215                 220

<210>  3
<211>  220
<212>  PRT
<213>  Artificial

<220>
<223>  Tyr296Cys Fc amino acid

<400>  3

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Glu Glu Gln Cys Asn Ser Thr Tyr Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys Gly Gln Pro Arg Glu Pro Gln Val Tyr Thr Leu Pro Pro Ser Arg
        115                 120                 125

Asp Glu Leu Thr Lys Asn Gln Val Ser Leu Thr Cys Leu Val Lys Gly
    130                 135                 140

Phe Tyr Pro Ser Asp Ile Ala Val Glu Trp Glu Ser Asn Gly Gln Pro
145                 150                 155                 160

Glu Asn Asn Tyr Lys Thr Thr Pro Pro Val Leu Asp Ser Asp Gly Ser

165                 170                 175

Phe Phe Leu Tyr Ser Lys Leu Thr Val Asp Lys Ser Arg Trp Gln Gln
            180                 185                 190

Gly Asn Val Phe Ser Cys Ser Val Met His Glu Ala Leu His Asn His
        195                 200                 205

Tyr Thr Gln Lys Ser Leu Ser Leu Ser Pro Gly Lys
    210                 215                 220

<210>  4
<211>  220
<212>  PRT
<213>  Artificial

<220>
<223>  Asn297Cys Fc amino acid

<400>  4

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Glu Glu Gln Tyr Cys Ser Thr Tyr Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys Gly Gln Pro Arg Glu Pro Gln Val Tyr Thr Leu Pro Pro Ser Arg
        115                 120                 125

Asp Glu Leu Thr Lys Asn Gln Val Ser Leu Thr Cys Leu Val Lys Gly
    130                 135                 140

Phe Tyr Pro Ser Asp Ile Ala Val Glu Trp Glu Ser Asn Gly Gln Pro
145                 150                 155                 160

Glu Asn Asn Tyr Lys Thr Thr Pro Pro Val Leu Asp Ser Asp Gly Ser
                165                 170                 175

Phe Phe Leu Tyr Ser Lys Leu Thr Val Asp Lys Ser Arg Trp Gln Gln
            180                 185                 190

Gly Asn Val Phe Ser Cys Ser Val Met His Glu Ala Leu His Asn His
        195                 200                 205

Tyr Thr Gln Lys Ser Leu Ser Leu Ser Pro Gly Lys
    210                 215                 220

<210>  5
<211>  220
<212>  PRT
<213>  Artificial

<220>
<223>  Ser298Cys Fc amino acid

<400>  5

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Glu Glu Gln Tyr Asn Cys Thr Tyr Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys Gly Gln Pro Arg Glu Pro Gln Val Tyr Thr Leu Pro Pro Ser Arg
        115                 120                 125

Asp Glu Leu Thr Lys Asn Gln Val Ser Leu Thr Cys Leu Val Lys Gly
    130                 135                 140

Phe Tyr Pro Ser Asp Ile Ala Val Glu Trp Glu Ser Asn Gly Gln Pro
145                 150                 155                 160

Glu Asn Asn Tyr Lys Thr Thr Pro Pro Val Leu Asp Ser Asp Gly Ser
                165                 170                 175

Phe Phe Leu Tyr Ser Lys Leu Thr Val Asp Lys Ser Arg Trp Gln Gln
            180                 185                 190

Gly Asn Val Phe Ser Cys Ser Val Met His Glu Ala Leu His Asn His
        195                 200                 205

Tyr Thr Gln Lys Ser Leu Ser Leu Ser Pro Gly Lys
    210                 215                 220

<210>  6
<211>  220
<212>  PRT
<213>  Artificial

<220>
<223>  Tyr300Cys Fc amino acid

<400>  6

Pro Cys Pro Ala Pro Glu Leu Leu Gly Gly Pro Ser Val Phe Leu Phe
1               5                   10                  15

Pro Pro Lys Pro Lys Asp Thr Leu Met Ile Ser Arg Thr Pro Glu Val
            20                  25                  30

Thr Cys Val Val Val Asp Val Ser His Glu Asp Pro Glu Val Lys Phe
        35                  40                  45

Asn Trp Tyr Val Asp Gly Val Glu Val His Asn Ala Lys Thr Lys Pro
    50                  55                  60

Arg Glu Glu Gln Tyr Asn Ser Thr Cys Arg Val Val Ser Val Leu Thr
65                  70                  75                  80

Val Leu His Gln Asp Trp Leu Asn Gly Lys Glu Tyr Lys Cys Lys Val
                85                  90                  95

Ser Asn Lys Ala Leu Pro Ala Pro Ile Glu Lys Thr Ile Ser Lys Ala
            100                 105                 110

Lys Gly Gln Pro Arg Glu Pro Gln Val Tyr Thr Leu Pro Pro Ser Arg
        115                 120                 125

Asp Glu Leu Thr Lys Asn Gln Val Ser Leu Thr Cys Leu Val Lys Gly
    130                 135                 140

Phe Tyr Pro Ser Asp Ile Ala Val Glu Trp Glu Ser Asn Gly Gln Pro
145                 150                 155                 160

Glu Asn Asn Tyr Lys Thr Thr Pro Pro Val Leu Asp Ser Asp Gly Ser
                165                 170                 175

Phe Phe Leu Tyr Ser Lys Leu Thr Val Asp Lys Ser Arg Trp Gln Gln
            180                 185                 190

Gly Asn Val Phe Ser Cys Ser Val Met His Glu Ala Leu His Asn His
        195                 200                 205

Tyr Thr Gln Lys Ser Leu Ser Leu Ser Pro Gly Lys
    210                 215                 220

<210>  7
<211>  660
<212>  DNA
<213>  Artificial

<220>
<223>  Glu293Cys Fc nucleotide

<400>  7
ccgtgcccag cacctgaact cctgggggga ccgtcagtct tcctcttccc cccaaaaccc      60

aaggacaccc tcatgatctc ccggacccct gaggtcacat gcgtggtggt ggacgtgagc     120

cacgaagacc ctgaggtcaa gttcaactgg tacgtggacg gcgtggaggt gcataatgcc     180

aagacaaagc cgcggtgcga gcagtacaac agcacgtacc gtgtggtcag cgtcctcacc     240

gtcctgcacc aggactggct gaatggcaag gagtacaagt gcaaggtctc caacaaagcc     300

ctcccagccc ccatcgagaa aaccatctcc aaagccaaag ggcagccccg agaaccacag     360

gtgtacaccc tgcccccatc ccgggatgag ctgaccaaga accaggtcag cctgacctgc     420

ctggtcaaag gcttctatcc cagcgacatc gccgtggagt gggagagcaa tgggcagccg     480

gagaacaact acaagaccac gcctcccgtg ctggactccg acggctcctt cttcctctac     540

agcaagctca ccgtggacaa gagcaggtgg cagcagggga acgtcttctc atgctccgtg     600

atgcatgagg ctctgcacaa ccactacacg cagaagagcc tctccctgtc tccgggtaaa     660

<210>  8
<211>  660
<212>  DNA
<213>  Artificial

<220>
<223>  Glu294Cys Fc nucleotide

<400>  8
ccgtgcccag cacctgaact cctgggggga ccgtcagtct tcctcttccc cccaaaaccc      60

aaggacaccc tcatgatctc ccggacccct gaggtcacat gcgtggtggt ggacgtgagc     120

cacgaagacc ctgaggtcaa gttcaactgg tacgtggacg gcgtggaggt gcataatgcc     180

aagacaaagc cgcgggagtg ccagtacaac agcacgtacc gtgtggtcag cgtcctcacc     240

gtcctgcacc aggactggct gaatggcaag gagtacaagt gcaaggtctc caacaaagcc     300

ctcccagccc ccatcgagaa aaccatctcc aaagccaaag ggcagccccg agaaccacag     360

gtgtacaccc tgcccccatc ccgggatgag ctgaccaaga accaggtcag cctgacctgc     420

ctggtcaaag gcttctatcc cagcgacatc gccgtggagt gggagagcaa tgggcagccg 480

gagaacaact acaagaccac gcctcccgtg ctggactccg acggctcctt cttcctctac 540

agcaagctca ccgtggacaa gagcaggtgg cagcagggga acgtcttctc atgctccgtg 600

atgcatgagg ctctgcacaa ccactacacg cagaagagcc tctccctgtc tccgggtaaa 660

<210> 9
<211> 660
<212> DNA
<213> Artificial

<220>
<223> Tyr296Cys Fc nucleotide

<400> 9
ccgtgcccag cacctgaact cctgggggga ccgtcagtct tcctcttccc cccaaaaccc 60

aaggacaccc tcatgatctc ccggacccct gaggtcacat gcgtggtggt ggacgtgagc 120

cacgaagacc ctgaggtcaa gttcaactgg tacgtggacg gcgtggaggt gcataatgcc 180

aagacaaagc cgcgggagga gcagtgcaac agcacgtacc gtgtggtcag cgtcctcacc 240

gtcctgcacc aggactggct gaatggcaag gagtacaagt gcaaggtctc caacaaagcc 300

ctcccagccc ccatcgagaa aaccatctcc aaagccaaag ggcagccccg agaaccacag 360

gtgtacaccc tgcccccatc ccgggatgag ctgaccaaga accaggtcag cctgacctgc 420

ctggtcaaag gcttctatcc cagcgacatc gccgtggagt gggagagcaa tgggcagccg 480

gagaacaact acaagaccac gcctcccgtg ctggactccg acggctcctt cttcctctac 540

agcaagctca ccgtggacaa gagcaggtgg cagcagggga acgtcttctc atgctccgtg 600

atgcatgagg ctctgcacaa ccactacacg cagaagagcc tctccctgtc tccgggtaaa 660

<210> 10
<211> 660
<212> DNA
<213> Artificial

<220>
<223> Asn297Cys Fc nucleotide

<400> 10
ccgtgcccag cacctgaact cctgggggga ccgtcagtct tcctcttccc cccaaaaccc 60

aaggacaccc tcatgatctc ccggacccct gaggtcacat gcgtggtggt ggacgtgagc 120

cacgaagacc ctgaggtcaa gttcaactgg tacgtggacg gcgtggaggt gcataatgcc 180

aagacaaagc cgcgggagga gcagtactgc agcacgtacc gtgtggtcag cgtcctcacc 240

gtcctgcacc aggactggct gaatggcaag gagtacaagt gcaaggtctc caacaaagcc 300

ctcccagccc ccatcgagaa aaccatctcc aaagccaaag ggcagccccg agaaccacag 360

gtgtacaccc tgcccccatc ccgggatgag ctgaccaaga accaggtcag cctgacctgc 420

ctggtcaaag gcttctatcc cagcgacatc gccgtggagt gggagagcaa tgggcagccg 480

gagaacaact acaagaccac gcctcccgtg ctggactccg acggctcctt cttcctctac 540

agcaagctca ccgtggacaa gagcaggtgg cagcagggga acgtcttctc atgctccgtg 600

atgcatgagg ctctgcacaa ccactacacg cagaagagcc tctccctgtc tccgggtaaa 660

<210> 11
<211> 660
<212> DNA
<213> Artificial

<220>
<223> Ser298Cys Fc nucleotide

<400> 11
ccgtgcccag cacctgaact cctgggggga ccgtcagtct tcctcttccc cccaaaaccc 60

aaggacaccc tcatgatctc ccggacccct gaggtcacat gcgtggtggt ggacgtgagc 120

cacgaagacc ctgaggtcaa gttcaactgg tacgtggacg gcgtggaggt gcataatgcc 180

aagacaaagc cgcgggagga gcagtacaac tgcacgtacc gtgtggtcag cgtcctcacc 240

gtcctgcacc aggactggct gaatggcaag gagtacaagt gcaaggtctc caacaaagcc 300

ctcccagccc ccatcgagaa aaccatctcc aaagccaaag ggcagccccg agaaccacag 360

gtgtacaccc tgcccccatc ccgggatgag ctgaccaaga accaggtcag cctgacctgc 420

ctggtcaaag gcttctatcc cagcgacatc gccgtggagt gggagagcaa tgggcagccg 480

gagaacaact acaagaccac gcctcccgtg ctggactccg acggctcctt cttcctctac 540

agcaagctca ccgtggacaa gagcaggtgg cagcagggga acgtcttctc atgctccgtg 600

atgcatgagg ctctgcacaa ccactacacg cagaagagcc tctccctgtc tccgggtaaa 660

<210> 12
<211> 660
<212> DNA
<213> Artificial

<220>
<223> Tyr300Cys Fc nucleotide

<400> 12
ccgtgcccag cacctgaact cctgggggga ccgtcagtct tcctcttccc cccaaaaccc 60

aaggacaccc tcatgatctc ccggacccct gaggtcacat gcgtggtggt ggacgtgagc 120

cacgaagacc ctgaggtcaa gttcaactgg tacgtggacg gcgtggaggt gcataatgcc 180

aagacaaagc cgcgggagga gcagtacaac agcacgtgcc gtgtggtcag cgtcctcacc 240

gtcctgcacc aggactggct gaatggcaag gagtacaagt gcaaggtctc caacaaagcc 300

ctcccagccc ccatcgagaa aaccatctcc aaagccaaag ggcagccccg agaaccacag 360

gtgtacaccc tgcccccatc ccgggatgag ctgaccaaga accaggtcag cctgacctgc 420

ctggtcaaag gcttctatcc cagcgacatc gccgtggagt gggagagcaa tgggcagccg 480

gagaacaact acaagaccac gcctcccgtg ctggactccg acggctcctt cttcctctac 540

agcaagctca ccgtggacaa gagcaggtgg cagcagggga acgtcttctc atgctccgtg 600

atgcatgagg ctctgcacaa ccactacacg cagaagagcc tctccctgtc tccgggtaaa     660

<210>  13
<211>  330
<212>  PRT
<213>  Artificial

<220>
<223>  Glu293Cys CH amino acid

<400>  13

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Cys
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  14
<211>  330
<212>  PRT
<213>  Artificial

<220>
<223>  Glu294Cys CH amino acid

<400>  14

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Cys Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  15
<211>  330
<212>  PRT
<213>  Artificial

<220>
<223>  Tyr296Cys CH amino acid

<400>  15

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Cys Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  16
<211>  330
<212>  PRT
<213>  Artificial

<220>
<223>  Asn297Cys CH amino acid

<400>  16

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Cys Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  17
<211>  330
<212>  PRT
<213>  Artificial

<220>
<223>  Ser298Cys CH amino acid

<400>  17

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser

50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Cys Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  18
<211>  330
<212>  PRT
<213>  Artificial

<220>
<223>  Tyr300Cys CH amino acid

<400>  18

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Cys Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly

210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  19
<211>  990
<212>  DNA
<213>  Artificial

<220>
<223>  Glu293Cys CH nucleotide

<400>  19
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agcagagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcggtgcga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 20
<211> 990
<212> DNA
<213> Artificial

<220>
<223> Glu294Cys CH nucleotide

<400> 20
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagtg ccagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 21
<211> 990
<212> DNA
<213> Artificial

<220>
<223> Tyr296Cys CH nucleotide

<400> 21
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtgcaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 22
<211> 990
<212> DNA
<213> Artificial

<220>
<223> Asn297Cys CH nucleotide

<400> 22
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtactgc 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 23
<211> 990
<212> DNA
<213> Artificial

<220>
<223> Ser298Cys CH nucleotide

<400> 23
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

tgcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 24
<211> 990
<212> DNA
<213> Artificial

<220>
<223> Tyr300Cys CH nucleotide

<400> 24
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agcagagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtgcc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 25
<211> 452
<212> PRT
<213> Artificial

<220>
<223> Glu293Cys H amino acid

<400> 25

Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Ala Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Cys Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro

385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  26
<211>  452
<212>  PRT
<213>  Artificial

<220>
<223>  Glu294Cys H amino acid

<400>  26

Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Ala Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Cys Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val

420                 425                 430

Met His Glu Ala Leu His Asn His Tyr Thr Gln Lys Ser Leu Ser Leu
        435                 440                 445

Ser Pro Gly Lys
    450

<210>  27
<211>  452
<212>  PRT
<213>  Artificial

<220>
<223>  Tyr296Cys H amino acid

<400>  27

Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Ala Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Cys Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys

    450

<210>  28
<211>  452
<212>  PRT
<213>  Artificial

<220>
<223>  Asn297Cys H amino acid

<400>  28

Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Ala Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Cys Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  29
<211>  452
<212>  PRT
<213>  Artificial

<220>
<223>  Ser298Cys H amino acid

<400>  29

Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Ala Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Cys Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210> 30
<211> 452
<212> PRT
<213> Artificial

<220>
<223> Tyr300Cys H amino acid

<400> 30

Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Val Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Val Val Tyr Tyr Ser Asn Ser Tyr Trp Tyr Phe Asp Val Trp
            100                 105                 110

Gly Thr Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Ala Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Cys Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  31
<211>  1356
<212>  DNA
<213>  Artificial

<220>
<223>  Glu293Cys H nucleotide

<400>  31
caggcttatc tacagcagtc tggggctgag ctggtgaggc ctggggcctc agtgaagatg      60

tcctgcaagg cttctggcta cacatttacc agttacaata tgcactgggt aaagcagaca     120

cctagacagg gcctggaatg gattggagct atttatccag gaaatggtga tacttcctac     180

aatcagaagt tcaagggcaa ggccacactg actgtagaca aatcctccag cacagcctac     240

atgcagctca gcagcctgac atctgaagac tctgcggtct atttctgtgc aagagtggtg     300

tactatagta actcttactg gtacttcgat gtctggggca cagggaccac ggtcaccgtc     360

tcctcagcct ccaccaaggg cccatcggtc ttccccctgg caccctcctc caagagcacc 420

tctgggggca cagcggccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacc 600

cagacctaca tctgcaacgt gaatcacaag cccagcaaca ccaaggtgga caagaaagca 660

gagcccaaat cttgtgacaa aactcacaca tgcccaccgt gcccagcacc tgaactcctg 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg gtgcgagcag 900

tacaacagca cgtaccgtgt ggtcagcgtc ctcaccgtcc tgcaccagga ctggctgaat 960

ggcaaggagt acaagtgcaa ggtctccaac aaagccctcc cagcccccat cgagaaaacc 1020

atctccaaag ccaaagggca gccccgagaa ccacaggtgt acaccctgcc cccatcccgg 1080

gatgagctga ccaagaacca ggtcagcctg acctgcctgg tcaaaggctt ctatcccagc 1140

gacatcgccg tggagtggga gagcaatggg cagccggaga acaactacaa gaccacgcct 1200

cccgtgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 32
<211> 1356
<212> DNA
<213> Artificial

<220>
<223> Glu294Cys H nucleotide

<400> 32
caggcttatc tacagcagtc tggggctgag ctggtgaggc ctggggcctc agtgaagatg 60

tcctgcaagg cttctggcta cacatttacc agttacaata tgcactgggt aaagcagaca 120

cctagacagg gcctggaatg gattggagct atttatccag gaaatggtga tacttcctac 180

aatcagaagt tcaagggcaa ggccacactg actgtagaca aatcctccag cacagcctac 240

atgcagctca gcagcctgac atctgaagac tctgcggtct atttctgtgc aagagtggtg 300

tactatagta actcttactg gtacttcgat gtctggggca cagggaccac ggtcaccgtc 360

tcctcagcct ccaccaaggg cccatcggtc ttccccctgg caccctcctc caagagcacc 420

tctgggggca cagcggccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacc 600

cagacctaca tctgcaacgt gaatcacaag cccagcaaca ccaaggtgga caagaaagca 660

gagcccaaat cttgtgacaa aactcacaca tgcccaccgt gcccagcacc tgaactcctg 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg ggagtgccag 900

tacaacagca cgtaccgtgt ggtcagcgtc ctcaccgtcc tgcaccagga ctggctgaat 960

ggcaaggagt acaagtgcaa ggtctccaac aaagccctcc cagcccccat cgagaaaacc 1020

atctccaaag ccaaagggca gccccgagaa ccacaggtgt acaccctgcc cccatcccgg 1080

gatgagctga ccaagaacca ggtcagcctg acctgcctgg tcaaaggctt ctatcccagc 1140

gacatcgccg tggagtggga gagcaatggg cagccggaga acaactacaa gaccacgcct 1200

cccgtgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 33
<211> 1356
<212> DNA
<213> Artificial

<220>
<223> Tyr296Cys H nucleotide

<400> 33
caggcttatc tacagcagtc tggggctgag ctggtgaggc ctggggcctc agtgaagatg 60

tcctgcaagg cttctggcta cacatttacc agttacaata tgcactgggt aaagcagaca 120

cctagacagg gcctggaatg gattggagct atttatccag gaaatggtga tacttcctac 180

aatcagaagt tcaagggcaa ggccacactg actgtagaca aatcctccag cacagcctac 240

atgcagctca gcagcctgac atctgaagac tctgcggtct atttctgtgc aagagtggtg 300

tactatagta actcttactg gtacttcgat gtctggggca cagggaccac ggtcaccgtc 360

tcctcagcct ccaccaaggg cccatcggtc ttccccctgg caccctcctc caagagcacc 420

tctgggggca cagcggccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacc 600

cagacctaca tctgcaacgt gaatcacaag cccagcaaca ccaaggtgga caagaaagca 660

gagcccaaat cttgtgacaa aactcacaca tgcccaccgt gcccagcacc tgaactcctg 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg ggaggagcag 900

tgcaacagca cgtaccgtgt ggtcagcgtc ctcaccgtcc tgcaccagga ctggctgaat 960

ggcaaggagt acaagtgcaa ggtctccaac aaagccctcc cagcccccat cgagaaaacc 1020

atctccaaag ccaaagggca gccccgagaa ccacaggtgt acaccctgcc cccatcccgg 1080

gatgagctga ccaagaacca ggtcagcctg acctgcctgg tcaaaggctt ctatcccagc 1140

gacatcgccg tggagtggga gagcaatggg cagccggaga acaactacaa gaccacgcct 1200

cccgtgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 34
<211> 1356
<212> DNA
<213> Artificial

<220>
<223> Asn297Cys H nucleotide

<400> 34
caggcttatc tacagcagtc tggggctgag ctggtgaggc ctggggcctc agtgaagatg 60

tcctgcaagg cttctggcta cacatttacc agttacaata tgcactgggt aaagcagaca 120

cctagacagg gcctggaatg gattggagct atttatccag gaaatggtga tacttcctac 180

aatcagaagt tcaagggcaa ggccacactg actgtagaca aatcctccag cacagcctac 240

atgcagctca gcagcctgac atctgaagac tctgcggtct atttctgtgc aagagtggtg 300

tactatagta actcttactg gtacttcgat gtctggggca cagggaccac ggtcaccgtc 360

tcctcagcct ccaccaaggg cccatcggtc ttccccctgg caccctcctc caagagcacc 420

tctgggggca cagcggccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacc 600

cagacctaca tctgcaacgt gaatcacaag cccagcaaca ccaaggtgga caagaaagca 660

gagcccaaat cttgtgacaa aactcacaca tgcccaccgt gcccagcacc tgaactcctg 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg ggaggagcag 900

tactgcagca cgtaccgtgt ggtcagcgtc ctcaccgtcc tgcaccagga ctggctgaat 960

ggcaaggagt acaagtgcaa ggtctccaac aaagccctcc cagcccccat cgagaaaacc 1020

atctccaaag ccaaagggca gccccgagaa ccacaggtgt acaccctgcc cccatcccgg 1080

gatgagctga ccaagaacca ggtcagcctg acctgcctgg tcaaaggctt ctatcccagc 1140

gacatcgccg tggagtggga gagcaatggg cagccggaga acaactacaa gaccacgcct 1200

cccgtgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210>  35
<211>  1356
<212>  DNA
<213>  Artificial

<220>
<223>  Ser298Cys H nucleotide

<400>  35
caggcttatc tacagcagtc tggggctgag ctggtgaggc ctggggcctc agtgaagatg      60

tcctgcaagg cttctggcta cacatttacc agttacaata tgcactgggt aaagcagaca     120

cctagacagg gcctggaatg gattggagct atttatccag gaaatggtga tacttcctac     180

aatcagaagt tcaagggcaa ggccacactg actgtagaca aatcctccag cacagcctac     240

atgcagctca gcagcctgac atctgaagac tctgcggtct atttctgtgc aagagtggtg     300

tactatagta actcttactg gtacttcgat gtctggggca cagggaccac ggtcaccgtc     360

tcctcagcct ccaccaaggg cccatcggtc ttccccctgg caccctcctc caagagcacc     420

tctgggggca cagcggccct gggctgcctg gtcaaggact acttccccga accggtgacg     480

gtgtcgtgga actcaggcgc cctgaccagc ggcgtgcaca ccttcccggc tgtcctacag     540

tcctcaggac tctactccct cagcagcgtg gtgaccgtgc cctccagcag cttgggcacc     600

cagacctaca tctgcaacgt gaatcacaag cccagcaaca ccaaggtgga caagaaagca     660

gagcccaaat cttgtgacaa aactcacaca tgcccaccgt gcccagcacc tgaactcctg     720

gggggaccgt cagtcttcct cttcccccca aaacccaagg acaccctcat gatctcccgg     780

acccctgagg tcacatgcgt ggtggtggac gtgagccacg aagaccctga ggtcaagttc     840

aactggtacg tggacggcgt ggaggtgcat aatgccaaga caaagccgcg ggaggagcag     900

tacaactgca cgtaccgtgt ggtcagcgtc ctcaccgtcc tgcaccagga ctggctgaat     960

ggcaaggagt acaagtgcaa ggtctccaac aaagccctcc cagcccccat cgagaaaacc    1020

atctccaaag ccaaagggca gccccgagaa ccacaggtgt acaccctgcc cccatcccgg    1080

gatgagctga ccaagaacca ggtcagcctg acctgcctgg tcaaaggctt ctatcccagc    1140

gacatcgccg tggagtggga gagcaatggg cagccggaga acaactacaa gaccacgcct    1200

cccgtgctgg actccgacgg ctccttcttc ctctacagca agctcaccgt ggacaagagc    1260

aggtggcagc aggggaacgt cttctcatgc tccgtgatgc atgaggctct gcacaaccac    1320

tacacgcaga agagcctctc cctgtctccg ggtaaa                              1356


<210>  36
<211>  1356
<212>  DNA
<213>  Artificial

<220>
<223>  Tyr300Cys H nucleotide

<400>  36

caggcttatc tacagcagtc tggggctgag ctggtgaggc ctggggcctc agtgaagatg 60

tcctgcaagg cttctggcta cacatttacc agttacaata tgcactgggt aaagcagaca 120

cctagacagg gcctggaatg gattggagct atttatccag gaaatggtga tacttcctac 180

aatcagaagt tcaagggcaa ggccacactg actgtagaca aatcctccag cacagcctac 240

atgcagctca gcagcctgac atctgaagac tctgcggtct atttctgtgc aagagtggtg 300

tactatagta actcttactg gtacttcgat gtctggggca cagggaccac ggtcaccgtc 360

tcctcagcct ccaccaaggg cccatcggtc ttccccctgg caccctcctc caagagcacc 420

tctgggggca cagcggccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacc 600

cagacctaca tctgcaacgt gaatcacaag cccagcaaca ccaaggtgga caagaaagca 660

gagcccaaat cttgtgacaa aactcacaca tgcccaccgt gcccagcacc tgaactcctg 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg ggaggagcag 900

tacaacagca cgtgccgtgt ggtcagcgtc ctcaccgtcc tgcaccagga ctggctgaat 960

ggcaaggagt acaagtgcaa ggtctccaac aaagccctcc cagcccccat cgagaaaacc 1020

atctccaaag ccaaagggca gccccgagaa ccacaggtgt acaccctgcc cccatcccgg 1080

gatgagctga ccaagaacca ggtcagcctg acctgcctgg tcaaaggctt ctatcccagc 1140

gacatcgccg tggagtggga gagcaatggg cagccggaga acaactacaa gaccacgcct 1200

cccgtgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 37
<211> 471
<212> PRT
<213> Artificial

<220>
<223> Glu293Cys H amino acid variant

<400> 37

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Cys Glu Gln Tyr

305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  38
<211>  471
<212>  PRT
<213>  Artificial

<220>
<223>  Glu294Cys H amino acid variant

<400>  38

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Cys Gln Tyr
305                 310                 315                 320

Asn Ser Thr Tyr Arg Val Val Ser Val Leu Thr Val Leu His Gln Asp

325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  39
<211>  471
<212>  PRT
<213>  Artificial

<220>
<223>  Tyr296Cys H amino acid variant

<400>  39

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Cys
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu

340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  40
<211>  471
<212>  PRT
<213>  Artificial

<220>
<223>  Asn297Cys H amino acid variant

<400>  40

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Cys Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg

355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  41
<211>  471
<212>  PRT
<213>  Artificial

<220>
<223>  Ser298Cys H amino acid variant

<400>  41

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Cys Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys

370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  42
<211>  471
<212>  PRT
<213>  Artificial

<220>
<223>  Tyr300Cys H amino acid variant

<400>  42

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Cys Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp

385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  43
<211>  1413
<212>  DNA
<213>  Artificial

<220>
<223>  Glu293Cys H nucleotide variant

<400>  43
atgggttgga gcctcatctt gctcttcctt gtcgctgttg ctacgcgtgt cctgtcccag      60

gcttatctac agcagtctgg ggctgagctg gtgaggcctg gggcctcagt gaagatgtcc     120

tgcaaggctt ctggctacac atttaccagt tacaatatgc actgggtaaa gcagacacct     180

agacagggcc tggaatggat tggagctatt tatccaggaa atggtgatac ttcctacaat     240

cagaagttca agggcaaggc cacactgact gtagacaaat cctccagcac agcctacatg     300

cagctcagca gcctgacatc tgaagactct gcggtctatt tctgtgcaag agtggtgtac     360

tatagtaact cttactggta cttcgatgtc tggggcacag ggaccacggt caccgtctcc     420

tcagcctcca ccaagggccc atcggtcttc cccctggcac cctcctccaa gagcacctct     480

gggggcacag cggccctggg ctgcctggtc aaggactact tccccgaacc ggtgacggtg     540

tcgtggaact caggcgccct gaccagcggc gtgcacacct tcccggctgt cctacagtcc     600

tcaggactct actccctcag cagcgtggtg accgtgccct ccagcagctt gggcacccag     660

acctacatct gcaacgtgaa tcacaagccc agcaacacca aggtggacaa gaaagcagag     720

cccaaatctt gtgacaaaac tcacacatgc ccaccgtgcc cagcacctga actcctgggg     780

ggaccgtcag tcttcctctt ccccccaaaa cccaaggaca ccctcatgat ctcccggacc     840

cctgaggtca catgcgtggt ggtggacgtg agccacgaag accctgaggt caagttcaac     900

tggtacgtgg acggcgtgga ggtgcataat gccaagacaa agccgcggtg cgagcagtac     960

aacagcacgt accgtgtggt cagcgtcctc accgtcctgc accaggactg gctgaatggc    1020

aaggagtaca agtgcaaggt ctccaacaaa gccctcccag cccccatcga gaaaaccatc    1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 44
<211> 1413
<212> DNA
<213> Artificial

<220>
<223> Glu294Cys H nucleotide variant

<400> 44
atgggttgga gcctcatctt gctcttcctt gtcgctgttg ctacgcgtgt cctgtcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcggga gtgccagtac 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 45
<211> 1413
<212> DNA
<213> Artificial

<220>
<223> Tyr296Cys H nucleotide variant

<400> 45
atgggttgga gcctcatctt gctcttcctt gtcgctgttg ctacgcgtgt cctgtcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcggga ggagcagtgc 960

aacagcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 46
<211> 1413
<212> DNA
<213> Artificial

<220>
<223>  Asn297Cys H nucleotide variant

<400>  46
atgggttgga gcctcatctt gctcttcctt gtcgctgttg ctacgcgtgt cctgtcccag      60

gcttatctac agcagtctgg ggctgagctg gtgaggcctg gggcctcagt gaagatgtcc     120

tgcaaggctt ctggctacac atttaccagt tacaatatgc actgggtaaa gcagacacct     180

agacagggcc tggaatggat tggagctatt tatccaggaa atggtgatac ttcctacaat     240

cagaagttca agggcaaggc cacactgact gtagacaaat cctccagcac agcctacatg     300

cagctcagca gcctgacatc tgaagactct gcggtctatt tctgtgcaag agtggtgtac     360

tatagtaact cttactggta cttcgatgtc tggggcacag ggaccacggt caccgtctcc     420

tcagcctcca ccaagggccc atcggtcttc cccctggcac cctcctccaa gagcacctct     480

gggggcacag cggccctggg ctgcctggtc aaggactact tccccgaacc ggtgacggtg     540

tcgtggaact caggcgccct gaccagcggc gtgcacacct tcccggctgt cctacagtcc     600

tcaggactct actccctcag cagcgtggtg accgtgccct ccagcagctt gggcacccag     660

acctacatct gcaacgtgaa tcacaagccc agcaacacca aggtggacaa gaaagcagag     720

cccaaatctt gtgacaaaac tcacacatgc ccaccgtgcc cagcacctga actcctgggg     780

ggaccgtcag tcttcctctt ccccccaaaa cccaaggaca ccctcatgat ctcccggacc     840

cctgaggtca catgcgtggt ggtggacgtg agccacgaag accctgaggt caagttcaac     900

tggtacgtgg acggcgtgga ggtgcataat gccaagacaa agccgcggga ggagcagtac     960

tgcagcacgt accgtgtggt cagcgtcctc accgtcctgc accaggactg gctgaatggc    1020

aaggagtaca agtgcaaggt ctccaacaaa gccctcccag cccccatcga gaaaaccatc    1080

tccaaagcca aagggcagcc ccgagaacca caggtgtaca ccctgccccc atcccgggat    1140

gagctgacca agaaccaggt cagcctgacc tgcctggtca aaggcttcta tcccagcgac    1200

atcgccgtgg agtgggagag caatgggcag ccggagaaca actacaagac cacgcctccc    1260

gtgctggact ccgacggctc cttcttcctc tacagcaagc tcaccgtgga caagagcagg    1320

tggcagcagg ggaacgtctt ctcatgctcc gtgatgcatg aggctctgca caaccactac    1380

acgcagaaga gcctctccct gtctccgggt aaa                                 1413

<210>  47
<211>  1413
<212>  DNA
<213>  Artificial

<220>
<223>  Ser298Cys H nucleotide variant

<400>  47
atgggttgga gcctcatctt gctcttcctt gtcgctgttg ctacgcgtgt cctgtcccag      60

gcttatctac agcagtctgg ggctgagctg gtgaggcctg gggcctcagt gaagatgtcc     120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct 480

gggggcacag cggccctggg ctgcctggtc aaggactact tccccgaacc ggtgacggtg 540

tcgtggaact caggcgccct gaccagcggc gtgcacacct tcccggctgt cctacagtcc 600

tcaggactct actccctcag cagcgtggtg accgtgccct ccagcagctt gggcacccag 660

acctacatct gcaacgtgaa tcacaagccc agcaacacca aggtggacaa gaaagcagag 720

cccaaatctt gtgacaaaac tcacacatgc ccaccgtgcc cagcacctga actcctgggg 780

ggaccgtcag tcttcctctt ccccccaaaa cccaaggaca ccctcatgat ctcccggacc 840

cctgaggtca catgcgtggt ggtggacgtg agccacgaag accctgaggt caagttcaac 900

tggtacgtgg acggcgtgga ggtgcataat gccaagacaa agccgcggga ggagcagtac 960

aactgcacgt accgtgtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 48
<211> 1413
<212> DNA
<213> Artificial

<220>
<223> Tyr300Cys H nucleotide variant

<400> 48
atgggttgga gcctcatctt gctcttcctt gtcgctgttg ctacgcgtgt cctgtcccag 60

gcttatctac agcagtctgg ggctgagctg gtgaggcctg gggcctcagt gaagatgtcc 120

tgcaaggctt ctggctacac atttaccagt tacaatatgc actgggtaaa gcagacacct 180

agacagggcc tggaatggat tggagctatt tatccaggaa atggtgatac ttcctacaat 240

cagaagttca agggcaaggc cacactgact gtagacaaat cctccagcac agcctacatg 300

cagctcagca gcctgacatc tgaagactct gcggtctatt tctgtgcaag agtggtgtac 360

tatagtaact cttactggta cttcgatgtc tggggcacag ggaccacggt caccgtctcc 420

tcagcctcca ccaagggccc atcggtcttc cccctggcac cctcctccaa gagcacctct     480

gggggcacag cggccctggg ctgcctggtc aaggactact tccccgaacc ggtgacggtg     540

tcgtggaact caggcgccct gaccagcggc gtgcacacct tcccggctgt cctacagtcc     600

tcaggactct actccctcag cagcgtggtg accgtgccct ccagcagctt gggcacccag     660

acctacatct gcaacgtgaa tcacaagccc agcaacacca aggtggacaa gaaagcagag     720

cccaaatctt gtgacaaaac tcacacatgc ccaccgtgcc cagcacctga actcctgggg     780

ggaccgtcag tcttcctctt ccccccaaaa cccaaggaca ccctcatgat ctcccggacc     840

cctgaggtca catgcgtggt ggtggacgtg agccacgaag accctgaggt caagttcaac     900

tggtacgtgg acggcgtgga ggtgcataat gccaagacaa agccgcggga ggagcagtac     960

aacagcacgt gccgtgtggt cagcgtcctc accgtcctgc accaggactg gctgaatggc    1020

aaggagtaca agtgcaaggt ctccaacaaa gccctcccag cccccatcga gaaaaccatc    1080

tccaaagcca aagggcagcc ccgagaacca caggtgtaca ccctgccccc atcccgggat    1140

gagctgacca agaaccaggt cagcctgacc tgcctggtca aaggcttcta tcccagcgac    1200

atcgccgtgg agtgggagag caatgggcag ccggagaaca actacaagac cacgcctccc    1260

gtgctggact ccgacggctc cttcttcctc tacagcaagc tcaccgtgga caagagcagg    1320

tggcagcagg ggaacgtctt ctcatgctcc gtgatgcatg aggctctgca caaccactac    1380

acgcagaaga gcctctccct gtctccgggt aaa                                 1413

<210>  49
<211>  471
<212>  PRT
<213>  Homo sapiens

<400>  49

Met Gly Trp Ser Leu Ile Leu Leu Phe Leu Val Ala Val Ala Thr Arg
1               5                   10                  15

Val Leu Ser Gln Ala Tyr Leu Gln Gln Ser Gly Ala Glu Leu Val Arg
            20                  25                  30

Pro Gly Ala Ser Val Lys Met Ser Cys Lys Ala Ser Gly Tyr Thr Phe
        35                  40                  45

Thr Ser Tyr Asn Met His Trp Val Lys Gln Thr Pro Arg Gln Gly Leu
    50                  55                  60

Glu Trp Ile Gly Ala Ile Tyr Pro Gly Asn Gly Asp Thr Ser Tyr Asn
65                  70                  75                  80

Gln Lys Phe Lys Gly Lys Ala Thr Leu Thr Val Asp Lys Ser Ser Ser
                85                  90                  95

Thr Ala Tyr Met Gln Leu Ser Ser Leu Thr Ser Glu Asp Ser Ala Val
            100                 105                 110

Tyr Phe Cys Ala Arg Val Val Tyr Tyr Ser Asn Ser Tyr Trp Tyr Phe
        115                 120                 125

Asp Val Trp Gly Thr Gly Thr Thr Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Lys Ala Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350

Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Asp Glu Leu Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470

<210>  50
<211>  1413
<212>  DNA
<213>  Homo sapiens

<400>  50
atgggttgga gcctcatctt gctcttcctt gtcgctgttg ctacgcgtgt cctgtcccag      60

gcttatctac agcagtctgg ggctgagctg gtgaggcctg gggcctcagt gaagatgtcc     120

tgcaaggctt ctggctacac atttaccagt tacaatatgc actgggtaaa gcagacacct     180

agacagggcc tggaatggat tggagctatt tatccaggaa atggtgatac ttcctacaat     240

cagaagttca agggcaaggc cacactgact gtagacaaat cctccagcac agcctacatg     300

cagctcagca gcctgacatc tgaagactct gcggtctatt tctgtgcaag agtggtgtac     360

tatagtaact cttactggta cttcgatgtc tggggcacag ggaccacggt caccgtctcc     420

tcagcctcca ccaagggccc atcggtcttc cccctggcac cctcctccaa gagcacctct     480

gggggcacag cggccctggg ctgcctggtc aaggactact tccccgaacc ggtgacggtg     540

tcgtggaact caggcgccct gaccagcggc gtgcacacct tcccggctgt cctacagtcc     600

tcaggactct actccctcag cagcgtggtg accgtgccct ccagcagctt gggcacccag     660

acctacatct gcaacgtgaa tcacaagccc agcaacacca aggtggacaa gaaagcagag     720

cccaaatctt gtgacaaaac tcacacatgc ccaccgtgcc cagcacctga actcctgggg     780

ggaccgtcag tcttcctctt ccccccaaaa cccaaggaca ccctcatgat ctcccggacc     840

cctgaggtca catgcgtggt ggtggacgtg agccacgaag accctgaggt caagttcaac     900

tggtacgtgg acggcgtgga ggtgcataat gccaagacaa agccgcggga ggagcagtac     960

aacagcacgt accgggtggt cagcgtcctc accgtcctgc accaggactg gctgaatggc 1020

aaggagtaca agtgcaaggt ctccaacaaa gccctcccag cccccatcga gaaaaccatc 1080

tccaaagcca aagggcagcc ccgagaacca caggtgtaca ccctgccccc atcccgggat 1140

gagctgacca agaaccaggt cagcctgacc tgcctggtca aaggcttcta tcccagcgac 1200

atcgccgtgg agtgggagag caatgggcag ccggagaaca actacaagac cacgcctccc 1260

gtgctggact ccgacggctc cttcttcctc tacagcaagc tcaccgtgga caagagcagg 1320

tggcagcagg ggaacgtctt ctcatgctcc gtgatgcatg aggctctgca caaccactac 1380

acgcagaaga gcctctccct gtctccgggt aaa 1413

<210> 51
<211> 5
<212> PRT
<213> Homo sapiens

<400> 51

Ser Tyr Asn Met His
1               5

<210> 52
<211> 15
<212> DNA
<213> Homo sapiens

<400> 52
agttacaata tgcac 15

<210> 53
<211> 17
<212> PRT
<213> Homo sapiens

<400> 53

Ala Ile Tyr Pro Gly Asn Gly Asp Thr Ser Tyr Asn Gln Lys Phe Lys
1               5                   10                  15

Gly

<210> 54
<211> 51
<212> DNA
<213> Homo sapiens

<400> 54
gctatttatc caggaaatgg tgatacttcc tacaatcaga agttcaaggg c 51

<210> 55
<211> 13
<212> PRT
<213> Homo sapiens

<400> 55

Val Val Tyr Tyr Ser Asn Ser Tyr Trp Tyr Phe Asp Val
1               5                   10

<210>  56
<211>  39
<212>  DNA
<213>  Homo sapiens

<400>  56
gtggtgtact atagtaactc ttactggtac ttcgatgtc                              39

<210>  57
<211>  330
<212>  PRT
<213>  Homo sapiens

<400>  57

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Ala Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  58
<211>  990
<212>  DNA
<213>  Homo sapiens

<400>  58
gcctccacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agcagagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgtgagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 59
<211> 326
<212> PRT
<213> Homo sapiens

<400> 59

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  60
<211>  978
<212>  DNA
<213>  Homo sapiens

<400>  60
gcctccacca agggcccatc ggtcttcccc ctggcgccct gctccaggag cacctccgag      60

agcacagccg ccctgggctg cctggtcaag gactacttcc ccgaaccggt gacggtgtcg     120

tggaactcag gcgctctgac cagcggcgtg cacaccttcc cagctgtcct acagtcctca     180

ggactctact ccctcagcag cgtggtgacc gtgccctcca gcaacttcgg cacccagacc     240

tacacctgca acgtagatca caagcccagc aacaccaagg tggacaagac agttgagcgc     300

aaatgttgtg tcgagtgccc accgtgccca gcaccacctg tggcaggacc gtcagtcttc     360

ctcttccccc caaaacccaa ggacaccctc atgatctccc ggacccctga ggtcacgtgc     420

gtggtggtgg acgtgagcca cgaagacccc gaggtccagt tcaactggta cgtggacggc     480

gtggaggtgc ataatgccaa gacaaagcca cgggaggagc agttcaacag cacgttccgt     540

gtggtcagcg tcctcaccgt tgtgcaccag gactggctga acggcaagga gtacaagtgc     600

aaggtctcca acaaaggcct cccagccccc atcgagaaaa ccatctccaa aaccaaaggg     660

cagccccgag aaccacaggt gtacaccctg cccccatccc gggaggagat gaccaagaac     720

caggtcagcc tgacctgcct ggtcaaaggc ttctacccca gcgacatcgc cgtggagtgg     780

gagagcaatg ggcagccgga gaacaactac aagaccacac ctcccatgct ggactccgac 840

ggctccttct tcctctacag caagctcacc gtggacaaga gcaggtggca gcaggggaac 900

gtcttctcat gctccgtgat gcatgaggct ctgcacaacc actacacgca gaagagcctc 960

tccctgtctc cgggtaaa 978

<210> 61
<211> 377
<212> PRT
<213> Homo sapiens

<400> 61

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Lys Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210>  62
<211>  1131
<212>  DNA
<213>  Homo sapiens

<400>  62
gcttccacca agggcccatc ggtcttcccc ctggcgccct gctccaggag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacacctgca acgtgaatca caagcccagc aacaccaagg tggacaagag agttgagctc     300

aaaaccccac ttggtgacac aactcacaca tgcccacggt gcccagagcc caaatcttgt     360

gacacacctc ccccgtgccc acggtgccca gagcccaaat cttgtgacac acctccccca     420

tgcccacggt gcccagagcc caaatcttgt gacacacctc ccccgtgccc aaggtgccca     480

gcacctgaac tcctgggagg accgtcagtc ttcctcttcc ccccaaaacc caaggatacc 540

cttatgattt cccggacccc tgaggtcacg tgcgtggtgg tggacgtgag ccacgaagac 600

cccgaggtcc agttcaagtg gtacgtggac ggcgtggagg tgcataatgc caagacaaag 660

ccgcgggagg agcagtacaa cagcacgttc cgtgtggtca gcgtcctcac cgtcctgcac 720

caggactggc tgaacggcaa ggagtacaag tgcaaggtct ccaacaaagc cctcccagcc 780

cccatcgaga aaaccatctc caaaaccaaa ggacagcccc gagaaccaca ggtgtacacc 840

ctgcccccat cccgggagga gatgaccaag aaccaggtca gcctgacctg cctggtcaaa 900

ggcttctacc ccagcgacat cgccgtggag tgggagagca gcgggcagcc ggagaacaac 960

tacaagacca cgcctcccat gctggactcc gacggctcct tcttcctcta cagcaagctc 1020

accgtggaca agagcaggtg gcagcagggg aacatcttct catgctccgt gatgcatgag 1080

gctctgcaca accgcttcac gcagaagagc ctctccctgt ctccgggtaa a 1131

<210> 63
<211> 327
<212> PRT
<213> Homo sapiens

<400> 63

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  64
<211>  981
<212>  DNA
<213>  Homo sapiens

<400>  64
gcttccacca agggcccatc cgtcttcccc ctggcgccct gctccaggag cacctccgag      60

agcacagccg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacgaagacc     240

tacacctgca acgtagatca caagcccagc aacaccaagg tggacaagag agttgagtcc     300

aaatatggtc ccccatgccc atcatgccca gcacctgagt tcctgggggg accatcagtc     360

ttcctgttcc ccccaaaacc caaggacact ctcatgatct cccggacccc tgaggtcacg     420

tgcgtggtgg tggacgtgag ccaggaagac cccgaggtcc agttcaactg gtacgtggat 480

ggcgtggagg tgcataatgc caagacaaag ccgcgggagg agcagttcaa cagcacgtac 540

cgtgtggtca gcgtcctcac cgtcctgcac caggactggc tgaacggcaa ggagtacaag 600

tgcaaggtct ccaacaaagg cctcccgtcc tccatcgaga aaaccatctc caaagccaaa 660

gggcagcccc gagagccaca ggtgtacacc ctgcccccat cccaggagga gatgaccaag 720

aaccaggtca gcctgacctg cctggtcaaa ggcttctacc ccagcgacat cgccgtggag 780

tgggagagca atgggcagcc ggagaacaac tacaagacca cgcctcccgt gctggactcc 840

gacggctcct tcttcctcta cagcaggcta accgtggaca agagcaggtg gcaggagggg 900

aatgtcttct catgctccgt gatgcatgag gctctgcaca accactacac acagaagagc 960

ctctccctgt ctctgggtaa a 981

<210> 65
<211> 234
<212> PRT
<213> Homo sapiens

<400> 65

Met Asp Phe Gln Val Gln Ile Ile Ser Phe Leu Leu Ile Ser Ala Ser
1               5                   10                  15

Val Ile Met Ser Arg Gly Gln Ile Val Leu Ser Gln Ser Pro Ala Ile
            20                  25                  30

Leu Ser Ala Ser Pro Gly Glu Lys Val Thr Met Thr Cys Arg Ala Ser
        35                  40                  45

Ser Ser Val Ser Tyr Met His Trp Tyr Gln Gln Lys Pro Gly Ser Ser
    50                  55                  60

Pro Lys Pro Trp Ile Tyr Ala Pro Ser Asn Leu Ala Ser Gly Val Pro
65                  70                  75                  80

Ala Arg Phe Ser Gly Ser Gly Ser Gly Thr Ser Tyr Ser Leu Thr Ile
                85                  90                  95

Ser Arg Val Glu Ala Glu Asp Ala Ala Asn Tyr Tyr Cys Gln Gln Trp
            100                 105                 110

Ser Phe Asn Pro Pro Thr Phe Gly Ala Gly Thr Lys Leu Glu Leu Lys
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210>  66
<211>  702
<212>  DNA
<213>  Homo sapiens

<400>  66
atggattttc aggtgcagat tatcagcttc ctgctaatca gtgcttcagt cataatgtcc      60

agaggacaaa ttgttctctc ccagtctcca gcaatcctgt ctgcatctcc aggggagaag     120

gtcacaatga cttgcagggc cagctcaagt gtaagttaca tgcactggta ccagcagaag     180

ccaggatcct cccccaaacc ctggatttat gccccatcca acctggcttc tggagtccct     240

gctcgcttca gtggcagtgg gtctgggacc tcttactctc tcacaatcag cagagtggag     300

gctgaagatg ctgccaatta ttactgccag cagtggagtt ttaacccacc cacgttcggt     360

gctgggacca agctggagct gaaaactgtg gctgcaccat ctgtcttcat cttcccgcca     420

tctgatgagc agttgaaatc tggaactgcc tctgttgtgt gcctgctgaa taacttctat     480

cccagagagg ccaaagtaca gtggaaggtg gataacgccc tccaatcggg taactcccag     540

gagagtgtca cagagcagga cagcaaggac agcacctaca gcctcagcag caccctgacg     600

ctgagcaaag cagactacga gaaacacaaa gtctacgcct gcgaagtcac ccatcagggc     660

ctgagctcgc ccgtcacaaa gagcttcaac aggggagagt gt                        702

<210>  67
<211>  5
<212>  PRT
<213>  Homo sapiens

<400>  67

Ser Tyr Asn Met His
1               5

<210>  68
<211>  30
<212>  DNA

<213>  Homo sapiens

<400>  68
agggccagct caagtgtaag ttacatgcac                                        30


<210>  69
<211>  17
<212>  PRT
<213>  Homo sapiens

<400>  69

Ala Ile Tyr Pro Gly Asn Gly Asp Thr Ser Tyr Asn Gln Lys Phe Lys
1               5                   10                  15


Gly




<210>  70
<211>  21
<212>  DNA
<213>  Homo sapiens

<400>  70
gccccatcca acctggcttc t                                                 21


<210>  71
<211>  13
<212>  PRT
<213>  Homo sapiens

<400>  71

Val Val Tyr Tyr Ser Asn Ser Tyr Trp Tyr Phe Asp Val
1               5                   10


<210>  72
<211>  27
<212>  DNA
<213>  Homo sapiens

<400>  72
cagcagtgga gttttaaccc acccacg                                           27


<210>  73
<211>  106
<212>  PRT
<213>  Homo sapiens

<400>  73

Thr Val Ala Ala Pro Ser Val Phe Ile Phe Pro Pro Ser Asp Glu Gln
1               5                   10                  15


Leu Lys Ser Gly Thr Ala Ser Val Val Cys Leu Leu Asn Asn Phe Tyr
            20                  25                  30


Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  74
<211>  318
<212>  DNA
<213>  Homo sapiens

<400>  74
actgtggctg caccatctgt cttcatcttc ccgccatctg atgagcagtt gaaatctgga      60

actgcctctg ttgtgtgcct gctgaataac ttctatccca gagaggccaa agtacagtgg     120

aaggtggata acgccctcca atcgggtaac tcccaggaga gtgtcacaga gcaggacagc     180

aaggacagca cctacagcct cagcagcacc ctgacgctga gcaaagcaga ctacgagaaa     240

cacaaagtct acgcctgcga agtcacccat cagggcctga gctcgcccgt cacaaagagc     300

ttcaacaggg gagagtgt                                                   318

<210>  75
<211>  375
<212>  PRT
<213>  Artificial

<220>
<223>  AILIM Glu221Cys amino acid

<400>  75

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro
145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Cys Glu Gln Tyr
    210                 215                 220

Asn Ser Thr Tyr Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg
            260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys
    370                 375

<210>  76
<211>  375
<212>  PRT
<213>  Artificial

<220>
<223>  AILIM Glu222Cys amino acid

<400>  76

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro
145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Glu Cys Gln Tyr
    210                 215                 220

Asn Ser Thr Tyr Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg
            260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys
    370                 375

<210>  77
<211>  375
<212>  PRT
<213>  Artificial

<220>
<223>  AILIM Tyr224Cys amino acid

<400>  77

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val

35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro
145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Glu Glu Gln Tyr
    210                 215                 220

Cys Ser Thr Tyr Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg
            260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys
    370                 375

<210>  78
<211>  375
<212>  PRT
<213>  Artificial

<220>
<223>  AILIM Asn225Cys amino acid

<400>  78

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro

145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Glu Glu Gln Cys
    210                 215                 220

Asn Ser Thr Tyr Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg
            260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys
    370                 375

<210>  79
<211>  375
<212>  PRT
<213>  Artificial

<220>
<223>  AILIM Ser226Cys amino acid

<400>  79

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro
145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Glu Glu Gln Tyr
    210                 215                 220

Asn Cys Thr Tyr Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg

260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys
    370                 375

<210>  80
<211>  375
<212>  PRT
<213>  Artificial

<220>
<223>  AILIM Tyr228Cys amino acid

<400>  80

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro
145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Glu Glu Gln Tyr
    210                 215                 220

Asn Ser Thr Cys Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg
            260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys

370                 375

<210>  81
<211>  1125
<212>  DNA
<213>  Artificial

<220>
<223>  AILIM Glu221Cys nucleotide

<400>  81
atgaagtcag gcctctggta tttctttctc ttctgcttgc gcattaaagt tttaacagga      60

gaaatcaatg gttctgccaa ttatgagatg tttatatttc acaacggagg tgtacaaatt     120

ttatgcaaat atcctgacat tgtccagcaa tttaaaatgc agttgctgaa aggggggcaa      180

atactctgcg atctcactaa gacaaaagga agtggaaaca cagtgtccat taagagtctg     240

aaattctgcc attctcagtt atccaacaac agtgtctctt tttttctata caacttggac     300

cattctcatg ccaactatta cttctgcaac ctatcaattt ttgatcctcc tccttttaaa     360

gtaactctta caggaggata tttgcatatt tatgaatcac aactttgttg ccagctgaag     420

gatcccgagg agcccaaatc ttgtgacaaa actcacacat gcccaccgtg cccagcacct     480

gaactcctgg ggggaccgtc agtcttcctc ttccccccaa aacccaagga caccctcatg     540

atctcccgga cccctgaggt cacatgcgtg gtggtggacg tgagccacga agaccctgag     600

gtcaagttca actggtacgt ggacggcgtg gaggtgcata atgccaagac aaagccgcgg     660

tgcgagcagt acaacagcac gtaccgtgtg gtcagcgtcc tcaccgtcct gcaccaggac     720

tggctgaatg gcaaggagta caagtgcaag gtctccaaca aagccctccc agcccccatc     780

gagaaaacca tctccaaagc caaagggcag ccccgagaac cacaggtgta caccctgccc     840

ccatcccggg atgagctgac caagaaccag gtcagcctga cctgcctggt caaaggcttc     900

tatcccagcg acatcgccgt ggagtgggag agcaatgggc agccggagaa caactacaag     960

accacgcctc ccgtgctgga ctccgacggc tccttcttcc tctacagcaa gctcaccgtg    1020

gacaagagca ggtggcagca ggggaacgtc ttctcatgct ccgtgatgca tgaggctctg    1080

cacaaccact acacgcagaa gagcctctcc ctgtctccgg gtaaa                    1125

<210>  82
<211>  1125
<212>  DNA
<213>  Artificial

<220>
<223>  AILIM Glu222Cys nucleotide

<400>  82
atgaagtcag gcctctggta tttctttctc ttctgcttgc gcattaaagt tttaacagga      60

gaaatcaatg gttctgccaa ttatgagatg tttatatttc acaacggagg tgtacaaatt     120

ttatgcaaat atcctgacat tgtccagcaa tttaaaatgc agttgctgaa aggggggcaa      180

atactctgcg atctcactaa gacaaaagga agtggaaaca cagtgtccat taagagtctg     240

aaattctgcc attctcagtt atccaacaac agtgtctctt tttttctata caacttggac 300

cattctcatg ccaactatta cttctgcaac ctatcaattt ttgatcctcc tccttttaaa 360

gtaactctta caggaggata tttgcatatt tatgaatcac aactttgttg ccagctgaag 420

gatcccgagg agcccaaatc ttgtgacaaa actcacacat gcccaccgtg cccagcacct 480

gaactcctgg ggggaccgtc agtcttcctc ttccccccaa aacccaagga caccctcatg 540

atctcccgga cccctgaggt cacatgcgtg gtggtggacg tgagccacga agaccctgag 600

gtcaagttca actggtacgt ggacggcgtg gaggtgcata atgccaagac aaagccgcgg 660

gagtgccagt acaacagcac gtaccgtgtg gtcagcgtcc tcaccgtcct gcaccaggac 720

tggctgaatg gcaaggagta caagtgcaag gtctccaaca aagccctccc agcccccatc 780

gagaaaacca tctccaaagc caaagggcag ccccgagaac cacaggtgta caccctgccc 840

ccatcccggg atgagctgac caagaaccag gtcagcctga cctgcctggt caaaggcttc 900

tatcccagcg acatcgccgt ggagtgggag agcaatgggc agccggagaa caactacaag 960

accacgcctc ccgtgctgga ctccgacggc tccttcttcc tctacagcaa gctcaccgtg 1020

gacaagagca ggtggcagca ggggaacgtc ttctcatgct ccgtgatgca tgaggctctg 1080

cacaaccact acacgcagaa gagcctctcc ctgtctccgg gtaaa 1125

<210> 83
<211> 1125
<212> DNA
<213> Artificial

<220>
<223> AILIM Tyr224Cys nucleotide

<400> 83
atgaagtcag gcctctggta tttctttctc ttctgcttgc gcattaaagt tttaacagga 60

gaaatcaatg gttctgccaa ttatgagatg tttatatttc acaacggagg tgtacaaatt 120

ttatgcaaat atcctgacat tgtccagcaa tttaaaatgc agttgctgaa aggggggcaa 180

atactctgcg atctcactaa gacaaaagga agtggaaaca cagtgtccat taagagtctg 240

aaattctgcc attctcagtt atccaacaac agtgtctctt tttttctata caacttggac 300

cattctcatg ccaactatta cttctgcaac ctatcaattt ttgatcctcc tccttttaaa 360

gtaactctta caggaggata tttgcatatt tatgaatcac aactttgttg ccagctgaag 420

gatcccgagg agcccaaatc ttgtgacaaa actcacacat gcccaccgtg cccagcacct 480

gaactcctgg ggggaccgtc agtcttcctc ttccccccaa aacccaagga caccctcatg 540

atctcccgga cccctgaggt cacatgcgtg gtggtggacg tgagccacga agaccctgag 600

gtcaagttca actggtacgt ggacggcgtg gaggtgcata atgccaagac aaagccgcgg 660

gaggagcagt gcaacagcac gtaccgtgtg gtcagcgtcc tcaccgtcct gcaccaggac 720

tggctgaatg gcaaggagta caagtgcaag gtctccaaca aagccctccc agcccccatc 780

gagaaaacca tctccaaagc caaagggcag ccccgagaac cacaggtgta caccctgccc 840

ccatcccggg atgagctgac caagaaccag gtcagcctga cctgcctggt caaaggcttc 900

tatcccagcg acatcgccgt ggagtgggag agcaatgggc agccggagaa caactacaag 960

accacgcctc ccgtgctgga ctccgacggc tccttcttcc tctacagcaa gctcaccgtg 1020

gacaagagca ggtggcagca ggggaacgtc ttctcatgct ccgtgatgca tgaggctctg 1080

cacaaccact acacgcagaa gagcctctcc ctgtctccgg gtaaa 1125

<210> 84
<211> 1125
<212> DNA
<213> Artificial

<220>
<223> AILIM Asn225Cys nucleotide

<400> 84
atgaagtcag gcctctggta tttctttctc ttctgcttgc gcattaaagt tttaacagga 60

gaaatcaatg gttctgccaa ttatgagatg tttatatttc acaacggagg tgtacaaatt 120

ttatgcaaat atcctgacat tgtccagcaa tttaaaatgc agttgctgaa aggggggcaa 180

atactctgcg atctcactaa gacaaaagga agtggaaaca cagtgtccat taagagtctg 240

aaattctgcc attctcagtt atccaacaac agtgtctctt tttttctata caacttggac 300

cattctcatg ccaactatta cttctgcaac ctatcaattt ttgatcctcc tccttttaaa 360

gtaactctta caggaggata tttgcatatt tatgaatcac aactttgttg ccagctgaag 420

gatcccgagg agcccaaatc ttgtgacaaa actcacacat gcccaccgtg cccagcacct 480

gaactcctgg ggggaccgtc agtcttcctc ttccccccaa aacccaagga caccctcatg 540

atctcccgga cccctgaggt cacatgcgtg gtggtggacg tgagccacga agaccctgag 600

gtcaagttca actggtacgt ggacggcgtg gaggtgcata atgccaagac aaagccgcgg 660

gaggagcagt actgcagcac gtaccgtgtg gtcagcgtcc tcaccgtcct gcaccaggac 720

tggctgaatg gcaaggagta caagtgcaag gtctccaaca aagccctccc agcccccatc 780

gagaaaacca tctccaaagc caaagggcag ccccgagaac cacaggtgta caccctgccc 840

ccatcccggg atgagctgac caagaaccag gtcagcctga cctgcctggt caaaggcttc 900

tatcccagcg acatcgccgt ggagtgggag agcaatgggc agccggagaa caactacaag 960

accacgcctc ccgtgctgga ctccgacggc tccttcttcc tctacagcaa gctcaccgtg 1020

gacaagagca ggtggcagca ggggaacgtc ttctcatgct ccgtgatgca tgaggctctg 1080

cacaaccact acacgcagaa gagcctctcc ctgtctccgg gtaaa 1125

<210> 85
<211> 1125
<212> DNA
<213> Artificial

<220>

<223>  AILIM Ser226Cys nucleotide

<400>  85
atgaagtcag gcctctggta tttctttctc ttctgcttgc gcattaaagt tttaacagga      60

gaaatcaatg gttctgccaa ttatgagatg tttatatttc acaacggagg tgtacaaatt     120

ttatgcaaat atcctgacat tgtccagcaa tttaaaatgc agttgctgaa aggggggcaa     180

atactctgcg atctcactaa gacaaaagga agtggaaaca cagtgtccat taagagtctg     240

aaattctgcc attctcagtt atccaacaac agtgtctctt tttttctata caacttggac     300

cattctcatg ccaactatta cttctgcaac ctatcaattt ttgatcctcc tccttttaaa     360

gtaactctta caggaggata tttgcatatt tatgaatcac aactttgttg ccagctgaag     420

gatcccgagg agcccaaatc ttgtgacaaa actcacacat gcccaccgtg cccagcacct     480

gaactcctgg ggggaccgtc agtcttcctc ttccccccaa aacccaagga caccctcatg     540

atctcccgga cccctgaggt cacatgcgtg gtggtggacg tgagccacga agaccctgag     600

gtcaagttca actggtacgt ggacggcgtg gaggtgcata atgccaagac aaagccgcgg     660

gaggagcagt acaactgcac gtaccgtgtg gtcagcgtcc tcaccgtcct gcaccaggac     720

tggctgaatg gcaaggagta caagtgcaag gtctccaaca aagccctccc agcccccatc     780

gagaaaacca tctccaaagc caaagggcag ccccgagaac cacaggtgta caccctgccc     840

ccatcccggg atgagctgac caagaaccag gtcagcctga cctgcctggt caaaggcttc     900

tatcccagcg acatcgccgt ggagtgggag agcaatgggc agccggagaa caactacaag     960

accacgcctc ccgtgctgga ctccgacggc tccttcttcc tctacagcaa gctcaccgtg    1020

gacaagagca ggtggcagca ggggaacgtc ttctcatgct ccgtgatgca tgaggctctg    1080

cacaaccact acacgcagaa gagcctctcc ctgtctccgg gtaaa                    1125

<210>  86
<211>  1125
<212>  DNA
<213>  Artificial

<220>
<223>  AILIM Tyr228Cys nucleotide

<400>  86
atgaagtcag gcctctggta tttctttctc ttctgcttgc gcattaaagt tttaacagga      60

gaaatcaatg gttctgccaa ttatgagatg tttatatttc acaacggagg tgtacaaatt     120

ttatgcaaat atcctgacat tgtccagcaa tttaaaatgc agttgctgaa aggggggcaa     180

atactctgcg atctcactaa gacaaaagga agtggaaaca cagtgtccat taagagtctg     240

aaattctgcc attctcagtt atccaacaac agtgtctctt tttttctata caacttggac     300

cattctcatg ccaactatta cttctgcaac ctatcaattt ttgatcctcc tccttttaaa     360

gtaactctta caggaggata tttgcatatt tatgaatcac aactttgttg ccagctgaag     420

gatcccgagg agcccaaatc ttgtgacaaa actcacacat gcccaccgtg cccagcacct     480

gaactcctgg ggggaccgtc agtcttcctc ttccccccaa aacccaagga caccctcatg 540

atctcccgga cccctgaggt cacatgcgtg gtggtggacg tgagccacga agaccctgag 600

gtcaagttca actggtacgt ggacggcgtg gaggtgcata atgccaagac aaagccgcgg 660

gaggagcagt acaacagcac gtgccgtgtg gtcagcgtcc tcaccgtcct gcaccaggac 720

tggctgaatg gcaaggagta caagtgcaag gtctccaaca aagccctccc agcccccatc 780

gagaaaacca tctccaaagc caaagggcag ccccgagaac cacaggtgta caccctgccc 840

ccatcccggg atgagctgac caagaaccag gtcagcctga cctgcctggt caaaggcttc 900

tatcccagcg acatcgccgt ggagtgggag agcaatgggc agccggagaa caactacaag 960

accacgcctc ccgtgctgga ctccgacggc tccttcttcc tctacagcaa gctcaccgtg 1020

gacaagagca ggtggcagca ggggaacgtc ttctcatgct ccgtgatgca tgaggctctg 1080

cacaaccact acacgcagaa gagcctctcc ctgtctccgg gtaaa 1125

<210> 87
<211> 375
<212> PRT
<213> Homo sapiens

<400> 87

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu Lys Asp Pro Glu Glu
    130                 135                 140

Pro Lys Ser Cys Asp Lys Thr His Thr Cys Pro Pro Cys Pro Ala Pro

145                 150                 155                 160

Glu Leu Leu Gly Gly Pro Ser Val Phe Leu Phe Pro Pro Lys Pro Lys
                165                 170                 175

Asp Thr Leu Met Ile Ser Arg Thr Pro Glu Val Thr Cys Val Val Val
            180                 185                 190

Asp Val Ser His Glu Asp Pro Glu Val Lys Phe Asn Trp Tyr Val Asp
        195                 200                 205

Gly Val Glu Val His Asn Ala Lys Thr Lys Pro Arg Glu Glu Gln Tyr
    210                 215                 220

Asn Ser Thr Tyr Arg Val Val Ser Val Leu Thr Val Leu His Gln Asp
225                 230                 235                 240

Trp Leu Asn Gly Lys Glu Tyr Lys Cys Lys Val Ser Asn Lys Ala Leu
                245                 250                 255

Pro Ala Pro Ile Glu Lys Thr Ile Ser Lys Ala Lys Gly Gln Pro Arg
            260                 265                 270

Glu Pro Gln Val Tyr Thr Leu Pro Pro Ser Arg Asp Glu Leu Thr Lys
        275                 280                 285

Asn Gln Val Ser Leu Thr Cys Leu Val Lys Gly Phe Tyr Pro Ser Asp
    290                 295                 300

Ile Ala Val Glu Trp Glu Ser Asn Gly Gln Pro Glu Asn Asn Tyr Lys
305                 310                 315                 320

Thr Thr Pro Pro Val Leu Asp Ser Asp Gly Ser Phe Phe Leu Tyr Ser
                325                 330                 335

Lys Leu Thr Val Asp Lys Ser Arg Trp Gln Gln Gly Asn Val Phe Ser
            340                 345                 350

Cys Ser Val Met His Glu Ala Leu His Asn His Tyr Thr Gln Lys Ser
        355                 360                 365

Leu Ser Leu Ser Pro Gly Lys
    370                 375

<210>  88
<211>  1125
<212>  DNA
<213>  Homo sapiens

<400>  88
atgaagtcag gcctctggta tttctttctc ttctgcttgc gcattaaagt tttaacagga      60

gaaatcaatg gttctgccaa ttatgagatg tttatatttc acaacggagg tgtacaaatt 120

ttatgcaaat atcctgacat tgtccagcaa tttaaaatgc agttgctgaa aggggggcaa 180

atactctgcg atctcactaa gacaaaagga agtggaaaca cagtgtccat taagagtctg 240

aaattctgcc attctcagtt atccaacaac agtgtctctt tttttctata caacttggac 300

cattctcatg ccaactatta cttctgcaac ctatcaattt ttgatcctcc tccttttaaa 360

gtaactctta caggaggata tttgcatatt tatgaatcac aactttgttg ccagctgaag 420

gatcccgagg agcccaaatc ttgtgacaaa actcacacat gcccaccgtg cccagcacct 480

gaactcctgg ggggaccgtc agtcttcctc ttccccccaa aacccaagga caccctcatg 540

atctcccgga cccctgaggt cacatgcgtg gtggtggacg tgagccacga agaccctgag 600

gtcaagttca actggtacgt ggacggcgtg gaggtgcata atgccaagac aaagccgcgg 660

gaggagcagt acaacagcac gtaccgggtg gtcagcgtcc tcaccgtcct gcaccaggac 720

tggctgaatg gcaaggagta caagtgcaag gtctccaaca aagccctccc agcccccatc 780

gagaaaacca tctccaaagc caaagggcag ccccgagaac cacaggtgta caccctgccc 840

ccatcccggg atgagctgac caagaaccag gtcagcctga cctgcctggt caaaggcttc 900

tatcccagcg acatcgccgt ggagtgggag agcaatgggc agccggagaa caactacaag 960

accacgcctc ccgtgctgga ctccgacggc tccttcttcc tctacagcaa gctcaccgtg 1020

gacaagagca ggtggcagca ggggaacgtc ttctcatgct ccgtgatgca tgaggctctg 1080

cacaaccact acacgcagaa gagcctctcc ctgtctccgg gtaaa 1125

<210> 89
<211> 139
<212> PRT
<213> Artificial

<220>
<223> AILIM/ICOS amino acid

<400> 89

Met Lys Ser Gly Leu Trp Tyr Phe Phe Leu Phe Cys Leu Arg Ile Lys
1               5                   10                  15

Val Leu Thr Gly Glu Ile Asn Gly Ser Ala Asn Tyr Glu Met Phe Ile
            20                  25                  30

Phe His Asn Gly Gly Val Gln Ile Leu Cys Lys Tyr Pro Asp Ile Val
        35                  40                  45

Gln Gln Phe Lys Met Gln Leu Leu Lys Gly Gly Gln Ile Leu Cys Asp
    50                  55                  60

Leu Thr Lys Thr Lys Gly Ser Gly Asn Thr Val Ser Ile Lys Ser Leu
65                  70                  75                  80

Lys Phe Cys His Ser Gln Leu Ser Asn Asn Ser Val Ser Phe Phe Leu
                85                  90                  95

Tyr Asn Leu Asp His Ser His Ala Asn Tyr Tyr Phe Cys Asn Leu Ser
            100                 105                 110

Ile Phe Asp Pro Pro Pro Phe Lys Val Thr Leu Thr Gly Gly Tyr Leu
        115                 120                 125

His Ile Tyr Glu Ser Gln Leu Cys Cys Gln Leu
    130                 135

<210>  90
<211>  417
<212>  DNA
<213>  Artificial

<220>
<223>  AILIM/ICOS nucleotide

<400>  90
atgaagtcag gcctctggta tttctttctc ttctgcttgc gcattaaagt tttaacagga      60

gaaatcaatg gttctgccaa ttatgagatg tttatatttc acaacggagg tgtacaaatt     120

ttatgcaaat atcctgacat tgtccagcaa tttaaaatgc agttgctgaa aggggggcaa      180

atactctgcg atctcactaa gacaaaagga agtggaaaca cagtgtccat taagagtctg     240

aaattctgcc attctcagtt atccaacaac agtgtctctt tttttctata caacttggac     300

cattctcatg ccaactatta cttctgcaac ctatcaattt ttgatcctcc tccttttaaa     360

gtaactctta caggaggata tttgcatatt tatgaatcac aactttgttg ccagctg        417

<210>  91
<211>  4
<212>  PRT
<213>  Artificial

<220>
<223>  AILIM linker amino acid

<400>  91

Lys Asp Pro Glu
1

<210>  92
<211>  12
<212>  DNA
<213>  Artificial

<220>
<223>  AILIM linker nucleotide

<400>  92
aaggatcccg ag                                                          12

<210>  93

<211>  12
<212>  PRT
<213>  Artificial

<220>
<223>  AILIM hinge amino acid

<400>  93

Glu Pro Lys Ser Cys Asp Lys Thr His Thr Cys Pro
1               5                   10


<210>  94
<211>  36
<212>  DNA
<213>  Artificial

<220>
<223>  AILIM hinge nucleotide

<400>  94
gagcccaaat cttgtgacaa aactcacaca tgccca                                36


<210>  95
<211>  30
<212>  DNA
<213>  Artificial

<220>
<223>  MKV1 primer

<400>  95
atgaagttgc ctgttaggct gttggtgctg                                       30


<210>  96
<211>  30
<212>  DNA
<213>  Artificial

<220>
<223>  MKV2 primer

<400>  96
atggagwcag acacactcct gytatgggtg                                       30


<210>  97
<211>  30
<212>  DNA
<213>  Artificial

<220>
<223>  MKV3 primer

<400>  97
atgagtgtgc tcactcaggt cctggsgttg                                       30


<210>  98
<211>  33
<212>  DNA
<213>  Artificial

<220>

<223>  MKV4 primer

<400>  98
atgaggrccc ctgctcagwt tyttggmwtc ttg                                    33


<210>  99
<211>  30
<212>  DNA
<213>  Artificial

<220>
<223>  MKV5 primer

<400>  99
atggatttwc aggtgcagat twtcagcttc                                        30


<210>  100
<211>  27
<212>  DNA
<213>  Artificial

<220>
<223>  MKV6 primer

<400>  100
atgaggtkcy ytgytsagyt yctgrgg                                           27


<210>  101
<211>  31
<212>  DNA
<213>  Artificial

<220>
<223>  MKV7 primer

<400>  101
atgggcwtca agatggagtc acakwyycwg g                                      31


<210>  102
<211>  31
<212>  DNA
<213>  Artificial

<220>
<223>  MKV8 primer

<400>  102
atgtggggay ctktttycmm tttttcaatt g                                      31


<210>  103
<211>  25
<212>  DNA
<213>  Artificial

<220>
<223>  MKV9 primer

<400>  103
atggtrtccw casctcagtt ccttg                                             25


<210>  104

<211>  27
<212>  DNA
<213>  Artificial

<220>
<223>  MKV10 primer

<400>  104
atgtatatat gtttgttgtc tatttct                                          27


<210>  105
<211>  28
<212>  DNA
<213>  Artificial

<220>
<223>  MKV11 primer

<400>  105
atggaagccc cagctcagct tctcttcc                                         28


<210>  106
<211>  20
<212>  DNA
<213>  Artificial

<220>
<223>  MKC primer

<400>  106
actggatggt gggaagatgg                                                  20


<210>  107
<211>  27
<212>  DNA
<213>  Artificial

<220>
<223>  MHV1 primer

<400>  107
atgaaatgca gctggggcat sttcttc                                          27


<210>  108
<211>  26
<212>  DNA
<213>  Artificial

<220>
<223>  MHV2 primer

<400>  108
atgggatgga gctrtatcat sytctt                                           26


<210>  109
<211>  27
<212>  DNA
<213>  Artificial

<220>
<223>  MHV3 primer

<400>  109
atgaagwtgt ggttaaactg ggttttt                                          27


<210>  110
<211>  25
<212>  DNA
<213>  Artificial

<220>
<223>  MHV4 primer

<400>  110
atgractttg ggytcagctt grttt                                            25


<210>  111
<211>  30
<212>  DNA
<213>  Artificial

<220>
<223>  MHV5 primer

<400>  111
atggactcca ggctcaattt agttttcctt                                       30


<210>  112
<211>  27
<212>  DNA
<213>  Artificial

<220>
<223>  MHV6 primer

<400>  112
atggctgtcy trgsgctrct cttctgc                                          27


<210>  113
<211>  26
<212>  DNA
<213>  Artificial

<220>
<223>  MHV7 primer

<400>  113
atggratgga gckggrtctt tmtctt                                           26


<210>  114
<211>  23
<212>  DNA
<213>  Artificial

<220>
<223>  MHV8 primer

<400>  114
atgagagtgc tgattctttt gtg                                              23


<210>  115
<211>  30
<212>  DNA

<213>  Artificial

<220>
<223>  MHV9 primer

<400>  115
atggmttggg tgtggamctt gctattcctg                                        30


<210>  116
<211>  27
<212>  DNA
<213>  Artificial

<220>
<223>  MHV10 primer

<400>  116
atgggcagac ttacattctc attcctg                                           27


<210>  117
<211>  28
<212>  DNA
<213>  Artificial

<220>
<223>  MHV11 primer

<400>  117
atggattttg ggctgatttt ttttattg                                          28


<210>  118
<211>  27
<212>  DNA
<213>  Artificial

<220>
<223>  MHV11 primer

<400>  118
atgatggtgt taagtcttct gtacctg                                           27


<210>  119
<211>  21
<212>  DNA
<213>  Artificial

<220>
<223>  MHCG2b primer

<400>  119
cagtggatag actgatgggg g                                                 21


<210>  120
<211>  24
<212>  DNA
<213>  Artificial

<220>
<223>  hIgG1 LCF primer

<400>  120
actgtggctg caccatctgt cttc                                              24

<210>  121
<211>  27
<212>  DNA
<213>  Artificial

<220>
<223>  hIgG1 LCR primer

<400>  121
ttaacactct cccctgttga agctctt                                          27


<210>  122
<211>  24
<212>  DNA
<213>  Artificial

<220>
<223>  hIgG1 HCF primer

<400>  122
gcctccacca agggcccatc ggtc                                             24


<210>  123
<211>  27
<212>  DNA
<213>  Artificial

<220>
<223>  hIgG1 HCR primer

<400>  123
ttatttaccc ggagacaggg agaggct                                          27


<210>  124
<211>  37
<212>  DNA
<213>  Artificial

<220>
<223>  L1 primer

<400>  124
accgctcgag atggattttc aggtgcagat tatcagc                               37


<210>  125
<211>  27
<212>  DNA
<213>  Artificial

<220>
<223>  L2 primer

<400>  125
tttcagctcc agcttggtcc cagcacc                                          27


<210>  126
<211>  27
<212>  DNA
<213>  Artificial

<220>
<223>  L3 primer

<400>  126
actgtggctg caccatctgt cttcatc                                          27


<210>  127
<211>  46
<212>  DNA
<213>  Artificial

<220>
<223>  L4 primer

<400>  127
atagtttagc ggccgcttaa cactctcccc tgttgaagct ctttgt                     46


<210>  128
<211>  37
<212>  DNA
<213>  Artificial

<220>
<223>  H1 primer

<400>  128
accgctcgag atgggatgga gctgggtctt tctcttc                               37


<210>  129
<211>  24
<212>  DNA
<213>  Artificial

<220>
<223>  H2 primer

<400>  129
tgaggagacg gtgaccgtgg tccc                                             24


<210>  130
<211>  24
<212>  DNA
<213>  Artificial

<220>
<223>  H3 primer

<400>  130
gcctccacca agggcccatc ggtc                                             24


<210>  131
<211>  46
<212>  DNA
<213>  Artificial

<220>
<223>  H4 primer

<400>  131
atagtttagc ggccgcttat ttacccggag acagggagag gctctt                     46

<210>  132
<211>  51
<212>  DNA
<213>  Artificial

<220>
<223>  Glu293Cys sense primer

<400>  132
gccaagacaa agccgcggtg cgagcagtac aacagcacgt accgggtggt c                51


<210>  133
<211>  51
<212>  DNA
<213>  Artificial

<220>
<223>  Glu293Cys anti-sense primer

<400>  133
gaccacccgg tacgtgctgt tgtactgctc gcaccgcggc tttgtcttgg c                51


<210>  134
<211>  46
<212>  DNA
<213>  Artificial

<220>
<223>  Glu294Cys sense primer

<400>  134
gacaaagccg cgggagtgcc agtacaacag cacgtaccgg gtggtc                      46


<210>  135
<211>  46
<212>  DNA
<213>  Artificial

<220>
<223>  Glu294Cys anti-sense primer

<400>  135
gacaaagccg cgggagtgcc agtacaacag cacgtaccgg gtggtc                      46


<210>  136
<211>  38
<212>  DNA
<213>  Artificial

<220>
<223>  Tyr296Cys sense primer

<400>  136
aagccgcggg aggagcagtg caacagcacg taccgggt                               38


<210>  137
<211>  38
<212>  DNA
<213>  Artificial

<220>
<223>  Tyr296Cys anti-sense primer

<400>  137
acccggtacg tgctgttgca ctgctcctcc cgcggctt                              38


<210>  138
<211>  28
<212>  DNA
<213>  Artificial

<220>
<223>  Asn297Cys sense primer

<400>  138
aggagcagta ctgcagcacg taccgggt                                         28


<210>  139
<211>  28
<212>  DNA
<213>  Artificial

<220>
<223>  Asn297Cys anti-sense primer

<400>  139
acccggtacg tgctgcagta ctgctcct                                         28


<210>  140
<211>  31
<212>  DNA
<213>  Artificial

<220>
<223>  Ser298Cys sense primer

<400>  140
gaggagcagt acaactgcac gtaccgggtg g                                     31


<210>  141
<211>  31
<212>  DNA
<213>  Artificial

<220>
<223>  Ser298Cys anti-sense primer

<400>  141
ccacccggta cgtgcagttg tactgctcct c                                     31


<210>  142
<211>  34
<212>  DNA
<213>  Artificial

<220>
<223>  Tyr300Cys sense primer

<400>  142
agcagtacaa cagcacgtgc cgggtggtca gcgt                                  34


<210>  143
<211>  34

<212>  DNA
<213>  Artificial

<220>
<223>  Tyr300Cys anti-sense primer

<400>  143
acgctgacca cccggcacgt gctgttgtac tgct                                34


<210>  144
<211>  60
<212>  DNA
<213>  Artificial

<220>
<223>  FUT8 siRNA Sense primer

<400>  144
gatccccgct gagtctctcc gaatacttca agagagtatt cggagagact cagcttttta     60


<210>  145
<211>  60
<212>  DNA
<213>  Artificial

<220>
<223>  FUT8 siRNA anti-sense primer

<400>  145
tcgataaaaa gctgagtctc tccgaatact ctcttgaagt attcggagag actcagcggg     60


<210>  146
<211>  23
<212>  DNA
<213>  Artificial

<220>
<223>  FUT-8 expression analysis sense primer

<400>  146
tggtctactg cttcatgatt gca                                            23


<210>  147
<211>  21
<212>  DNA
<213>  Artificial

<220>
<223>  FUT-8 expression analysis anti-sense primer

<400>  147
gcatagcgcc aattctgaga t                                              21


<210>  148
<211>  29
<212>  DNA
<213>  Artificial

<220>
<223>  AILIM/ICOS sense primer

<400>  148

tgttgctagc aaacatgaag tcaggcctc 29

<210> 149
<211> 26
<212> DNA
<213> Artificial

<220>.
<223> AILIM/ICOS anti-sense primer

<400> 149
aacggatcct tcagctggca acaaag 26

<210> 150
<211> 34
<212> DNA
<213> Artificial

<220>
<223> AILIM/ICOS sense primer

<400> 150
tgaaggatcc cgaggagccc aaatcttgtg acaa 34

<210> 151
<211> 39
<212> DNA
<213> Artificial

<220>
<223> AILIM/ICOS anti-sense primer

<400> 151
gaagcggccg ctcatttacc cggagacagg gagaggctc 39

<210> 152
<211> 26
<212> DNA
<213> Artificial

<220>
<223> CD16 sense primer

<400> 152
tttgaattca tgtggcagct gctcct 26

<210> 153
<211> 31
<212> DNA
<213> Artificial

<220>
<223> CD16 anti-sense primer

<400> 153
aaagcggccg cccagtctct tgttgagctt c 31

## Description

### Technical Field

The present invention relates to a polypeptide having an enhanced effector function, a nucleic acid coding therefor, a vector containing the nucleic acid, a host cell or host organism having the vector, a pharmaceutical composition containing the polypeptide, a method for producing the polypeptide, a method for enhancing an effector function of an antibody, a method for preparing a high effector function antibody-producing cell, etc.

### Background Art

An antibody has a variable region involved in binding to an antigen and a constant region involved in bioactivity, and the C terminal side of a heavy chain constant region is formed from an Fc region consisting of CH2 and CH3. The Fc region is not directly involved in binding to an antigen but, through binding to an Fc receptor (FcR), can make a cell having such an Fc receptor molecule on the surface exhibit various effector functions. Specifically, binding an Fc receptor binding site on the Fc region to an FcR present on the effector cell surface causes various important biological reactions including phagocytosis and rupture of an antibody-coated particle, purification of an immunocomplex, lysis of an antibody-coated target cell by an activated effector cell (antibody-dependent cell-mediated cytotoxicity (ADCC)), release of an inflammatory mediator, placental transfer, and control of immunoglobulin production.
In recent years, antibody therapies that utilize such properties of an antibody in the treatment of a disease have been attempted. However, when producing an antibody, preparation of a cell that produces an antibody that recognizes a target antigen requires time and effort; moreover, in order to obtain the necessary antibody activity, since it is necessary to carry out production using an animal cell the resulting increase in cost of antibody production is one of the concerns in antibody medicine. Therefore, for the efficient development of an antibody medicine, there is a demand for an inexpensive method for producing an antibody and for a highly active antibody in order to cut costs, and research into such a production method and antibody has been carried out.

It has been suggested that, among effector functions possessed by an antibody, expression of ADCC induction activity requires binding between an Fc region of IgG and an FcR present on the surface of an effector cell, and some amino acid residues in the second domain (hereinafter referred to as the CH2 domain) of the constant region and the hinge region of the antibody are important for the above binding (ref. e.g. Non-Patent Documents 1 to 3). Furthermore, it has also been shown that a sugar chain binding to the CH2 domain is important for binding between an Fc region and an FcR (ref. e.g. Non-Patent Documents 3 and 4), and it has also been reported that an antibody having a high effector function is prepared by optimizing sugar chain structure (ref. e.g. Patent Document 1)).

Upon receipt of such reports, research into amino acid substitution of the Fc region has been actively carried out. Presta et al. have found that, by comprehensively carrying out alanine substitution of Fc region amino acids, some amino acid substitutions (S298A, E333A, K334A) increase the ability to bind to FcγRIIIa and increase ADCC activity (ref. Patent Document 2). Furthermore, Lazar et al. have presented data showing that by simultaneous substitution of 3 amino acids, that is, S239D/A330L/I332E, the ADCC activities of different antibodies are likewise increased (ref. Patent Document 3). Similar attempts are also described in Patent Documents 4 to 9.
However, an antibody having a satisfactory effector function has not yet been developed, and there has been a desire for further technical improvement.

Patent Document 1: JP, A, 2005-224240
Patent Document 2: WO 00/42072
Patent Document 3: US, A, 2005/0054832
Patent Document 4: WO 2004/063351 A2
Patent Document 5: WO 2004/074455 A2
Patent Document 6: WO 2005/070963 A1
Patent Document 7: WO 2006/019447 A1
Patent Document 8: WO 2006/104989 A2
Patent Document 9: WO 2006/105062 A2
Non-Patent Document 1: Eur. J. Immunol., 23, 1098 (1993)
Non-Patent Document 2: Immunology, 86, 319 (1995)
Non-Patent Document 3: Chemical Immunology, 65, 88 (1997)
Non-Patent Document 4: Abstracts of The 3rd Symposium of Japanese Consortium for Glycobiology and Glycotechnology p30 (2005)

### Disclosure of Invention

### Problems to be Solved by the Invention

The object of the present invention is to provide a polypeptide having an enhanced effector function.

### Means for Solving the Problems

While carrying out an intensive investigation in order to attain the above-mentioned object, the present inventors have found that, by substituting a specific site of the Fc region with a specific amino acid residue, an effector function can be outstandingly improved, and the present invention has thus been accomplished. That is, the present invention relates to the following. <1> (1) A polypeptide having an effector function, the polypeptide containing a polypeptide consisting of an amino acid sequence represented by any one of SEQ ID Nos: 1 to 6 as an Fc receptor binding portion, or (2) a polypeptide having an enhanced effector function and containing an Fc receptor binding portion selected from the group consisting of (i) a polypeptide that has one or more mutations in a polypeptide consisting of an amino acid sequence represented by any one of SEQ ID Nos: 1 to 6, (ii) a polypeptide that is coded by a nucleic acid having one or more mutations in the base sequence of a nucleic acid having a base sequence represented by any one of SEQ ID Nos: 7 to 12 or of a nucleic acid coding for the same polypeptide as that coded by the above nucleic acid, (iii) a polypeptide that is coded by a nucleic acid hybridizing under stringent conditions with a complementary strand, or a fragment thereof, of a nucleic acid coding for a polypeptide consisting of an amino acid sequence represented by any one of SEQ ID Nos: 1 to 6 or mutant (i) or (ii), and (iv) a polypeptide that is coded by a nucleic acid having at least 60% homology to a base sequence represented by any one of SEQ ID Nos: 7 to 12.

<2> The polypeptide according to <1> above, wherein the Fc receptor binding portion is an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue.
<3> The polypeptide according to <1> or <2> above, further including a cell binding portion.
<4> The polypeptide according to <3> above, wherein the cell binding portion recognizes or binds to at least one molecule selected from the group consisting of a cytokine receptor, a cell adhesion molecule, a cancer cell surface molecule, a cancer stem cell surface molecule, a blood cell surface molecule, and a virus-infected cell surface molecule.

<5> The polypeptide according to <4> above, wherein the cell binding portion recognizes or binds to at least one molecule selected from the group consisting of antigens CD3, CD11a, CD20, CD22, CD25, CD28, CD33, CD52, Her2/neu, EGF receptor, EpCAM, MUC1, GD3, CEA, CA125, HLA-DR, TNFα receptor, VEGF receptor, CTLA-4, AILIM/ICOS, and an integrin molecule.
<6> An isolated nucleic acid coding for the polypeptide according to any one of <1> to <5> above.
<7> A vector containing the nucleic acid according to <6> above.
<8> A host cell or host organism having the vector according to <7> above.
<9> A method for producing a polypeptide, the method including culturing the host cell or host organism according to <8> above so as to express the polypeptide coded by the nucleic acid.

<10> A method for producing in vitro an Fc region-containing polypeptide having a high effector function, the method including a step of subjecting an amino acid in the Fc region of the Fc region-containing polypeptide to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid.
<11> A polypeptide produced by the method according to <10> above.
<12> An in vitro method for enhancing an effector function of an antibody, the method including a step of subjecting an amino acid in an Fc region of the antibody to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid.
<13> An antibody obtained by the method according to <12> above.

<14> A method for preparing in vitro a high effector function antibody-producing cell, the method including a step of mutating a gene that codes for an Fc region of the antibody-producing cell so as to replace an amino acid in the Fc region by a cysteine residue, the amino acid being selected from the group consisting of, as Kabat EU index numbers, the 293rd amino acid, 294th amino acid, 296th amino acid, 297th amino acid, 298th amino acid, and 300th amino acid.
<15> An antibody-producing cell prepared by the method according to <14> above.
<16> A pharmaceutical composition containing the polypeptide according to any one of <1> to <5> and <11> above, the antibody according to <13> above, the nucleic acid according to <6> above, the vector according to <7> above, the host cell or host organism according to <8> above, and/or the antibody-producing cell according to <15> above.

### Effects of the Invention

The polypeptide of the present invention has an outstandingly enhanced effector function, and when it is used in antibody therapy, etc., since the amount of drug used can be reduced, it is economical and side effects can be suppressed.
Furthermore, since the method for enhancing an effector function of an antibody of the present invention can be applied to any existing antibody, the range of applications is wide, and enormous contributions to various fields including medicine and veterinary medicine can be anticipated.

### Brief Description of Drawings

[FIG. 1] A graph showing the results of evaluation of CD20 binding reactivity of an anti-CD20 chimera antibody and polypeptides of the present invention.
[FIG. 2] A graph showing the results of evaluation of CD16 binding reactivity of an anti-CD20 chimera antibody and polypeptides of the present invention.
[FIG. 3] A graph showing the results of evaluation of ADCC induction activity of an anti-CD20 chimera antibody and polypeptides of the present invention.

### Best Mode for Carrying Out the Invention

The present invention relates to
(1) a polypeptide having an effector function, the polypeptide containing a polypeptide consisting of an amino acid sequence represented by any one of SEQ ID Nos: 1 to 6 as an Fc receptor binding portion, or
(2) a polypeptide having an enhanced effector function and containing an Fc receptor binding portion selected from the group consisting of
   (i) a polypeptide that has one or more, preferably one or a few, mutations in a polypeptide consisting of an amino acid sequence represented by any one of SEQ ID Nos: 1 to 6,
   (ii) a polypeptide that is coded by a nucleic acid having one or more, preferably one or a few, mutations in the base sequence of a nucleic acid having a base sequence represented by any one of SEQ ID Nos: 7 to 12 or of a nucleic acid coding for the same polypeptide as that coded by the above nucleic acid,
   (iii) a polypeptide that is coded by a nucleic acid hybridizing under stringent conditions with a complementary strand, or a fragment thereof, of a nucleic acid coding for a polypeptide consisting of an amino acid sequence represented by any one of SEQ ID Nos: 1 to 6 or a mutant of (i) or (ii),
      and
   (iv) a polypeptide that is coded by a nucleic acid having at least 60%, preferably at least 70%, more preferably at least 80%, yet more preferably at least 90%, and particularly preferably at least 95% homology to a base sequence represented by any one of SEQ ID Nos: 7 to 12.
      In a preferred embodiment of the polypeptide of the present invention, the Fc receptor binding portion is an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue.

In the present invention, 'having an enhanced effector function' means that an effector function of the polypeptide of the present invention is enhanced compared with a polypeptide having a wild type Fc region, such as a wild type antibody. Specifically, this means, but is not limited to, for example, the FcR-binding capability of the polypeptide of the present invention being higher than a polypeptide having a wild type Fc region (with, for example, a concentration that exhibits the same effect being used as an index) by at least 1.5 times, preferably at least 2 times, more preferably at least 3 times, yet more preferably at least 4 times, and particularly preferably at least 5 times, for example, at least 6 times, 8 times, 10 times, 12 times, or 15 times, or compared with a polypeptide having a wild type Fc region (with, for example, a concentration that exhibits the same effect being used as an index), the ADCC induction activity of the polypeptide of the present invention being higher by at least 10 times, preferably at least 25 times, more preferably at least 50 times, yet more preferably at least 75 times, and particularly preferably at least 100 times, for example, at least 150 times, 200 times, 300 times, 400 times, or 500 times. The FcR-binding activity or effector function of a polypeptide may be determined as appropriate by any known technique, as described below.

The term 'stringent conditions' used in the present description is a well-known parameter in the present technical field, and is described in standard protocols such as, for example, Sambrook et al., Molecular Cloning: A Laboratory Manual, 3d ed., Cold Spring Harbor Press (2001) and Ausubel et al., Current Protocols in Molecular Biology, Greene Publishing Associates (1992).
The stringent conditions in the present invention denote, for example, hybridization at 65°C by a hybridization buffer containing 3.5×SSC (0.15 M sodium chloride/0.15 M sodium citrate, pH 7), Ficoll 0.02%, polyvinyl pyrrolidone 0.02%, bovine serum albumin 0.02%, NaH₂PO₄ 25 mM (pH 7), SDS 0.05%, and EDTA 2 mM. After hybridization, a membrane to which DNA is transferred is washed with 2xSSC at room temperature and subsequently with 0.1 to 0.5×SSC/0.1×SDS at a temperature up to 68°C. Alternatively, stringent hybridization may be carried out using a commercial hybridization buffer such as ExpressHyb® Hybridization Solution (Clontech), etc. under hybridization and washing conditions stated by the manufacturer.
There are other conditions, reagents, etc. that can be employed and result in the same level of stringency, but since it is expected that a person skilled in the art will be conversant with such conditions, they are not specially described in the present description. However, it is possible to modify the conditions so that a homologue or allele of a nucleic acid coding for a mutant of CSBP can be clearly identified.

As another embodiment of the polypeptide of the present invention, there can be cited a polypeptide having an effector function, the polypeptide containing an Fc receptor binding portion selected from the group consisting of
(1) a polypeptide consisting of an amino acid sequence represented by any one of SEQ ID Nos: 1 to 6,
(2) a polypeptide that has one or more, preferably one or a few, mutations in polypeptide (1) but has the same function as the above polypeptide,
(3) a polypeptide that has the same function as polypeptide (1) and is coded by a nucleic acid having one or more, preferably one or a few, mutations in the base sequence of a nucleic acid having a base sequence represented by any one of SEQ ID Nos: 7 to 12 or of a nucleic acid coding for the same polypeptide as that coded by the above nucleic acid,
(4) a polypeptide that has the same function as polypeptide (1) and is coded by a nucleic acid hybridizing under stringent conditions with a complementary strand, or a fragment thereof, of a nucleic acid coding for polypeptide (1) or mutant (2) or (3), and
(5) a polypeptide that has the same function as polypeptide (1) and is coded by a nucleic acid having at least 60%, preferably at least 70%, more preferably at least 80%, yet more preferably at least 90%, and particularly preferably at least 95% homology to a base sequence represented by any one of SEQ ID Nos: 7 to 12.

Whether or not polypeptides (2) to (5) above have the same function as polypeptide (1) may be determined by evaluating for example the activity of binding to an FcR or, in an embodiment in which a cell binding site is further contained, an effector function such as ADCC induction activity. Examples of a polypeptide that has the same function as polypeptide (1) include one for which the FcR-binding activity is enhanced compared with wild type Fc region and, in an embodiment in which a cell binding site is further contained, one for which an effector function such as ADCC induction activity is enhanced compared with wild type antibody. The 'same' referred to here includes a case in which the level of enhancement of the FcR-binding activity or the effector function in an embodiment in which a cell binding site is further contained is the same level as polypeptide (1), but is not limited thereto, and the level of enhancement may be smaller or larger than polypeptide (1) as long as the FcR-binding activity or the effector function is enhanced compared with wild type Fc region or wild type antibody. The FcR-binding activity or the effector function may be determined as appropriate by any known technique, as described below.

The polypeptide of the present invention has a remarkably high FcR-binding capability and an outstandingly enhanced effector function. Examples of the effector function include phagocytosis and rupture of an antibody-coated particle, purification of an immunocomplex, lysis of an antibody-coated target cell by an activated effector cell ((ADCC)), cell membrane rupture by a complement protein (complement-dependent cytotoxicity (CDC)), release of an inflammatory mediator, plancental transfer, and control of immunoglobulin production. Among them, for the polypeptide of the present invention, ADCC and CDC activities are enhanced, and ADCC activity is particularly enhanced.
The polypeptide of the present invention can mediate ADCC more effectively in the presence of a human effector cell. ADCC means a reaction mediated by a cell, and is a reaction in which an effector cell recognizes a target cell via an antibody and causes lysis of the target cell. Examples of the effector cell include a killer T cell, a natural killer (NK) cell, a neutrophil, a monocyte, and a macrophage. These effector cells usually have an FcR expressed on the cell surface and, depending on the structure of the Fc region, a cell that expresses a type of FcR that can bind thereto is recognized, thus enabling cytotoxic activity to be exhibited.
The polypeptide of the present invention recognizes an effector cell via an Fc receptor binding portion, thus inducing and promoting ADCC. In the present invention, probably due to the Fc receptor binding portion easily binding to a surface molecule (FcγR, etc.) on the effector cell so that activation can be efficiently carried out, high ADCC activity can be rapidly induced with a small amount of polypeptide.

CDC means a reaction mediated by a complement protein, and is a reaction in which the complement protein recognizes a target cell via an antibody and causes cell membrane rupture of the target cell. The complement protein is activated by binding to an Fc receptor binding portion of a polypeptide. In the present invention, probably due to the Fc receptor binding portion easily binding to a complement protein (C1q, etc.) so that activation can be efficiently carried out, high CDC activity can be rapidly induced with a small amount of polypeptide.
Furthermore, it is known that there is a possibility of an effector function being affected by polymorphism of an FcR. For example, it has been reported that the binding properties of the Fc region of an antibody are affected by the 158th amino acid of human CD16 being either valine or phenylalanine (ref. e.g. Koene HR et al., Blood. 1997; 90 (3): 1109-14). Because of this, the polypeptide of the present invention can also exhibit special reactivity depending on the polymorphism. Therefore, the polypeptide of the present invention in one embodiment exhibits a particularly high effector function for a person who has valine for the 158th amino acid of CD16. Furthermore, the polypeptide of the present invention in another embodiment exhibits a particularly high effector function for a person who has phenylalanine for the 158th amino acid of CD16.

The Fc receptor binding portion in the present invention preferably has a structure that can mediate an effector function (in particular, ADCC, CDC, etc.) effectively. From such a viewpoint, as the Fc receptor binding portion, those based on the Fc region of IgG may be used. Among them, the Fc region of human IgG is preferable, and the Fc region of human IgG1, human IgG2, human IgG3, or human IgG4, etc. is more preferably used. In the present invention, the Fc region of human IgG1 or human IgG3, which inherently have ADCC activity, is suitably used. Furthermore, CDC activity has different cidal effects depending on the subclass, and the Fc region of human IgG1, human IgG4, etc. may be used. The base sequences of wild type human Cγ1, Cγ2, Cγ3, and Cγ4 are shown in SEQ ID Nos: 58, 60, 62, and 64, and the amino acid sequences thereof are shown in SEQ ID Nos: 57, 59, 61, and 63. In addition, in the same way as for other proteins possessed by a living body, since polymorphism is also present in the Fc region of an antibody, wild type human Cγ1, Cγ2, Cγ3, or Cγ4 may have a base sequence and/or an amino acid sequence that is different from the above, and the Fc receptor binding portion of the present invention may be based on an Fc region having such polymorphism.
In addition, in the present description, in order to show a specific amino acid in an antibody constant region, a Kabat EU index might be referred to, and this is always based on (Sequence of Proteins of Immunological Interest, 5th Ed. Public Health Service, National Institutes of Health, Bethesda, MD (1991)). However, the above-mentioned index is used only for convenience in identifying a specific amino acid, and the amino acid sequence of the polypeptide of the present invention is not limited to those described in the above publication.

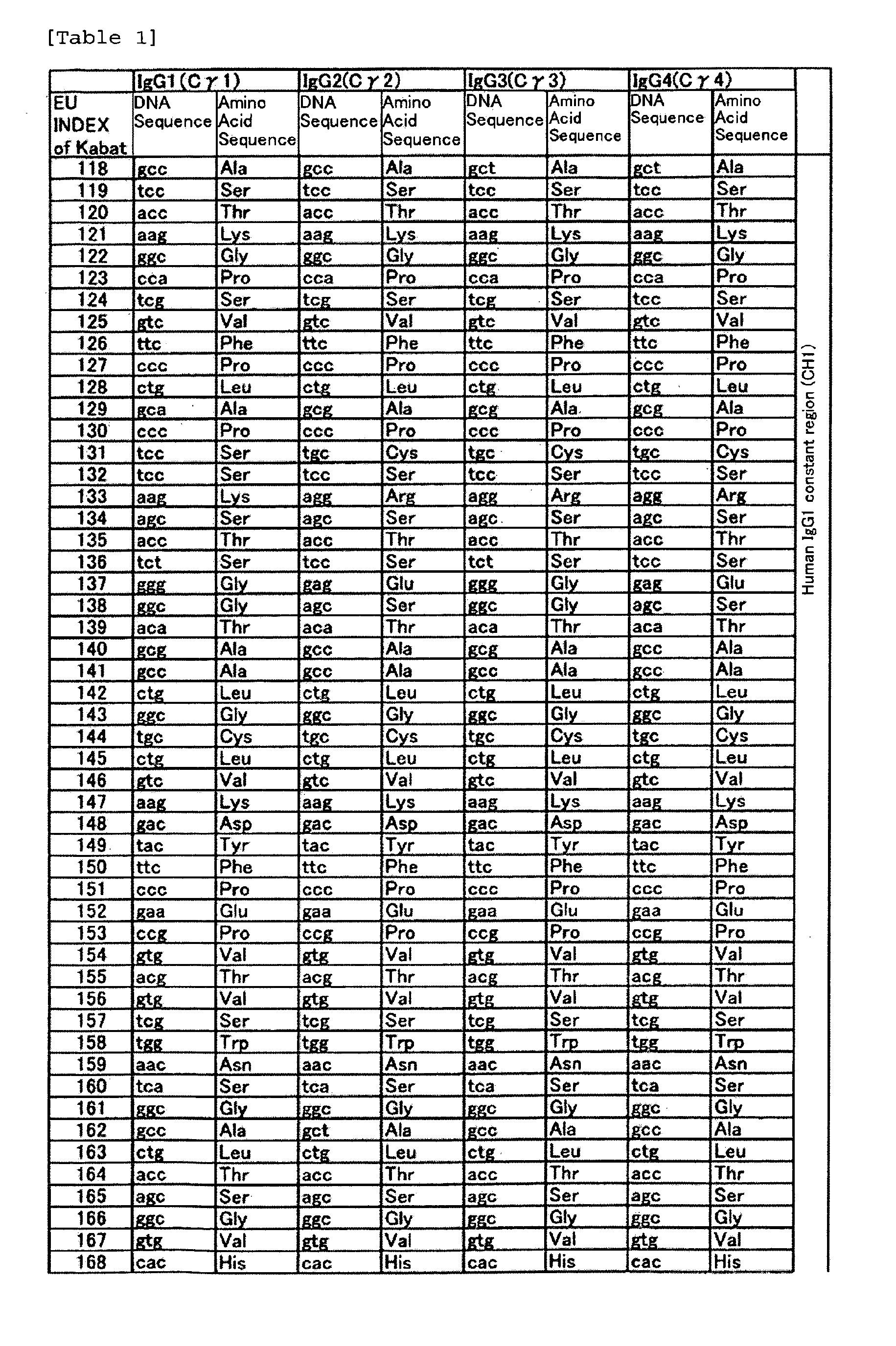

**Table 2**

| | | | | | | | | |
|---|---|---|---|---|---|---|---|---|
| 169 | acc | Thr | acc | Thr | acc | Thr | acc | Thr |
| 170 | ttc | Phe | ttc | Phe | ttc | Phe | ttc | Phe |
| 171 | ccg | Pro | cca | Pro | ccg | Pro | ccg | Pro |
| 172 | gct | Ala | gct | Ala | gct | Ala | gct | Ala |
| 173 | gtc | Val | gtc | Val | gtc | Val | gtc | Val |
| 174 | cta | Leu | cta | Leu | cta | Leu | cta | Leu |
| 175 | cag | Gln | cag | Gln | cag | Gln | cag | Gln |
| 176 | tcc | Ser | tcc | Ser | tcc | Ser | tcc | Ser |
| 177 | tca | Ser | tca | Ser | tca | Ser | tca | Ser |
| 178 | gga | Gly | gga | Gly | gga | Gly | gga | Gly |
| 179 | ctc | Leu | ctc | Leu | ctc | Leu | ctc | Leu |
| 180 | tac | Tyr | tac | Tyr | tac | Tyr | tac | Tyr |
| 181 | tcc | Ser | tcc | Ser | tcc | Ser | tcc | Ser |
| 182 | ctc | Leu | ctc | Leu | ctc | Leu | ctc | Leu |
| 183 | agc | Ser | agc | Ser | agc | Ser | agc | Ser |
| 184 | agc | Ser | agc | Ser | agc | Ser | agc | Ser |
| 185 | gtg | Val | gtg | Val | gtg | Val | gtg | Val |
| 186 | gtg | Val | gtg | Val | gtg | Val | gtg | Val |
| 187 | acc | Thr | acc | Thr | acc | Thr | acc | Thr |
| 188 | gtg | Val | gtg | Val | gtg | Val | gtg | Val |
| 189 | ccc | Pro | ccc | Pro | ccc | Pro | ccc | Pro |
| 190 | tcc | Ser | tcc | Ser | tcc | Ser | tcc | Ser |
| 191 | agc | Ser | age | Ser | agc | Ser | agc | Ser |
| 192 | agc | Ser | aac | Asn | agc | Ser | agc | Ser |
| 193 | ttg | Leu | ttc | Phe | ttg | Leu | ttg | Leu |
| 194 | ggc | Gly | ggc | Gly | ggc | Gly | ggc | Gly |
| 195 | acc | Thr | acc | Thr | acc | Thr | acg | Thr |
| 196 | cag | Gln | cag | Gln | cag | Gln | aag | Lys |
| 197 | acc | Thr | acc | Thr | acc | Thr | acc | Thr |
| 198 | tac | Tyr | tac | Tyr | tac | Tyr | tac | Tyr |
| 199 | atc | Ile | acc | Thr | acc | Thr | acc | Thr |
| 200 | tgc | Cys | tgc | Cys | tgc | Cys | tgc | Cys |
| 201 | aac | Asn | aac | Asn | aac | Asn | aac | Asn |
| 202 | gtg | Val | gta | Val | gtg | Val | gta | Val |
| 203 | aat | Asn | gat | Asp | aat | Asn | gat | Asp |
| 204 | cac | His | cac | His | cac | His | cac | His |
| 205 | aag | Lys | aag | Lys | aag | Lys | aag | Lys |
| 206 | ccc | Pro | ccc | Pro | ccc | Pro | ccc | Pro |
| 207 | agc | Ser | agc | Ser | agc | Ser | agc | Ser |
| 208 | aac | Asn | aac | Asn | aac | Asn | aac | Asn |
| 209 | acc | Thr | acc | Thr | acc | Thr | acc | Thr |
| 210 | aag | Lys | aag | Lys | aag | Lys | aag | Lys |
| 211 | gtg | Val | gtg | Val | gtg | Val | gtg | Val |
| 212 | gac | Asp | gac | Asp | gac | Asp | gac | Asp |
| 213 | aag | Lys | aag | Lys | aag | Lys | aag | Lys |
| 214 | aaa | Lys | aca | Thr | aga | Arg | aga | Arg |
| | | | gtt | Val | gtt | Val | gtt | Val |
| 215 | gca | Ala | | | | | | |
| 216 | gag | Glu | gag | Glu | gag | Glu | gag | Glu |
| 217 | ccc | Pro | cgc | Arg | ctc | Leu | tcc | Ser |
| 218 | aaa | Lys | aaa | Lys | aaa | Lys | aaa | Lys |
| 219 | tct | Ser | | | acc | Thr | tat | Tyr |
| 220 | tgt | Cys | | | cca | Pro | ggt | Gly |
| 221 | gac | Asp | tgt | Cys | ctt | Leu | | |
| | | | tgt | Cys | ggt | Gly | | |

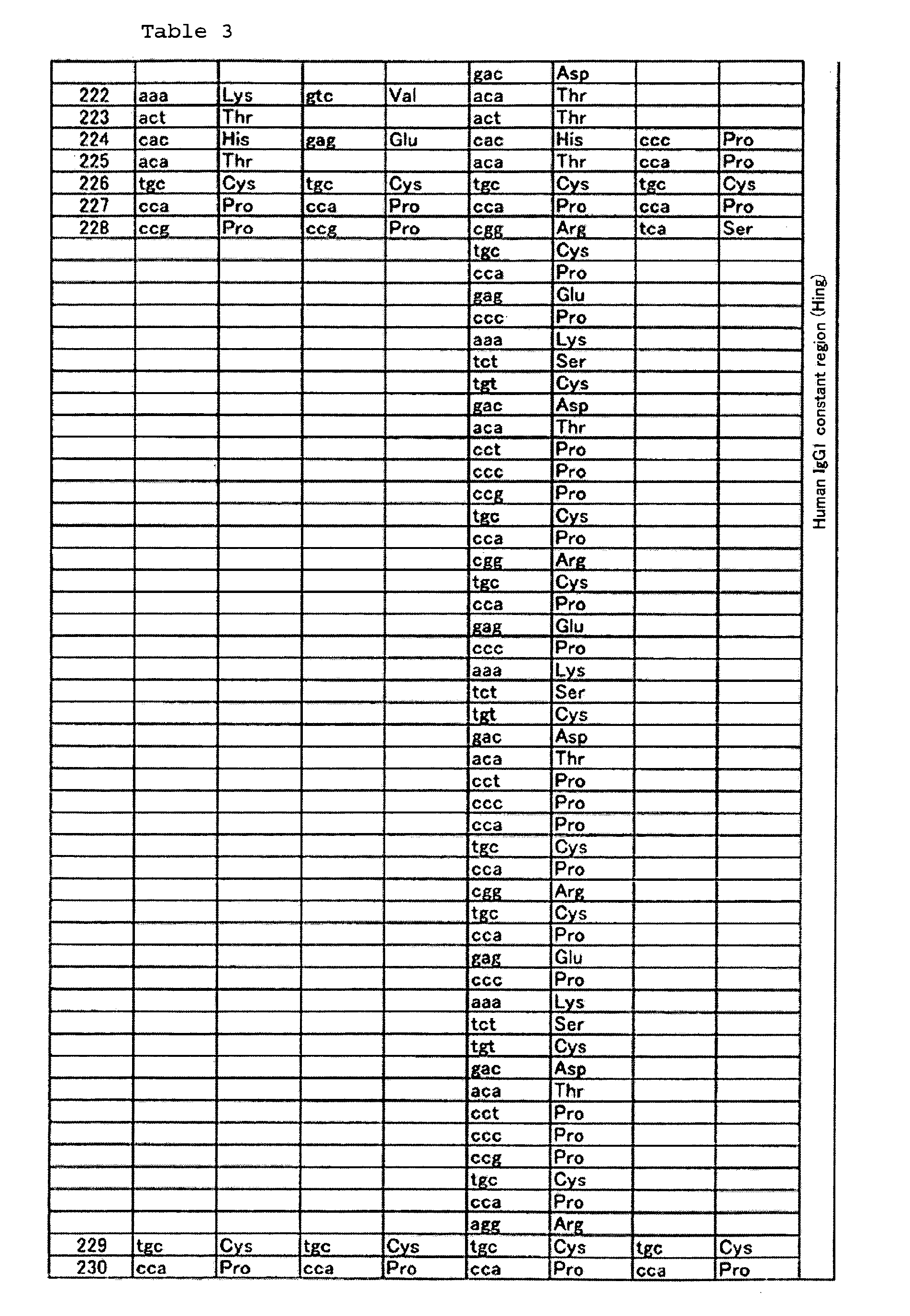

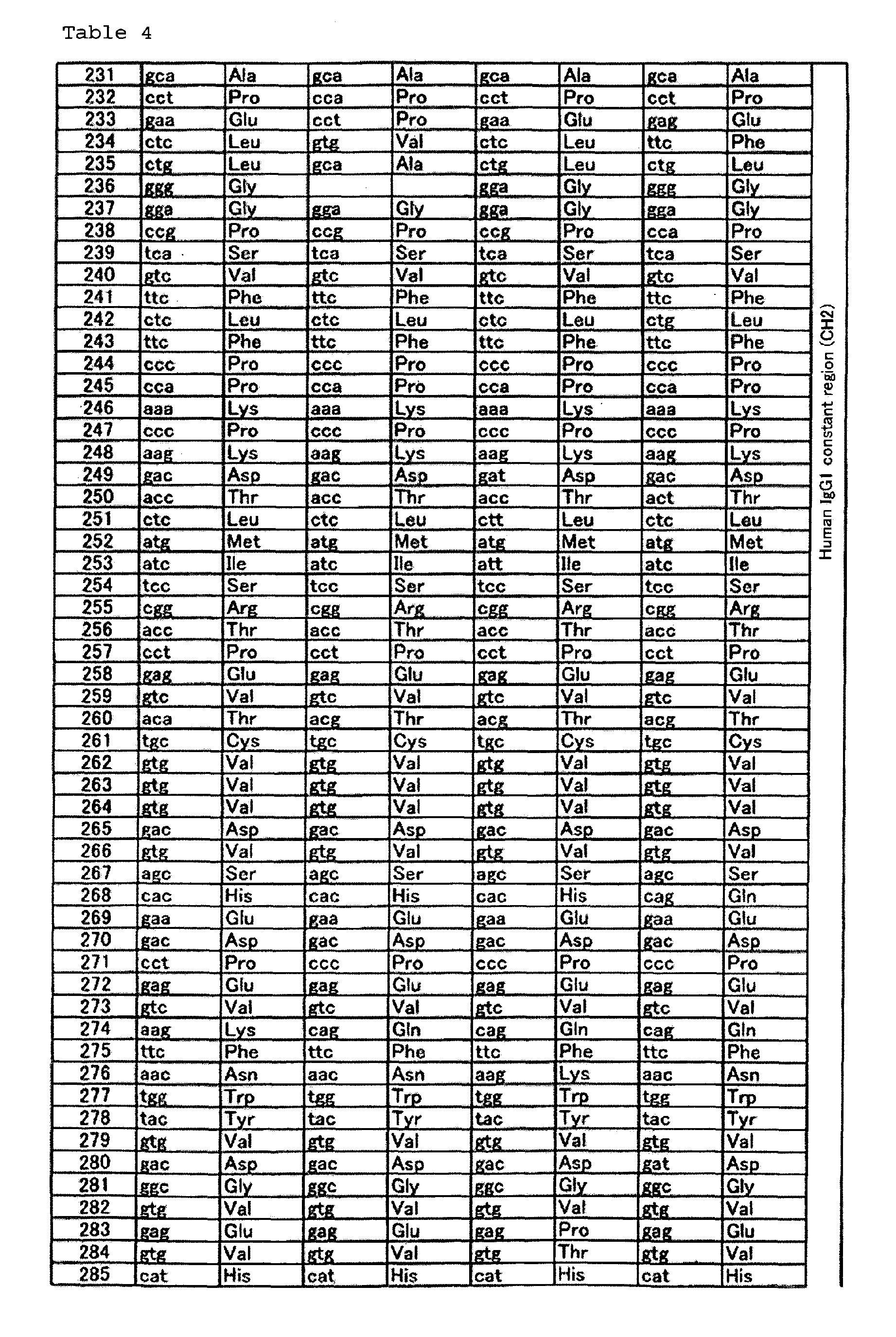

**Table 5**

| | | | | | | | | |
|---|---|---|---|---|---|---|---|---|
| 286 | aat | Asn | aat | Asn | aat | Asn | aat | Asn |
| 287 | gcc | Ala | gcc | Ala | gcc | Ala | gcc | Ala |
| 288 | aag | Lys | aag | Lys | aag | Lys | aag | Lys |
| 289 | aca | Thr | aca | Thr | aca | Thr | aca | Thr |
| 290 | aag | Lys | aag | Lys | aag | Lys | aag | Lys |
| 291 | ccg | Pro | cca | Pro | ccg | Pro | ccg | Pro |
| 292 | cgt | Arg | cgg | Arg | cgg | Arg | cgg | Arg |
| 293 | gag | Glu | gag | Glu | gag | Glu | gag | Glu |
| 294 | gag | Glu | gag | Glu | gag | Glu | gag | Glu |
| 295 | cag | Gln | cag | Gln | cag | Gln | cag | Gln |
| 296 | tac | Tyr | ttc | Phe | tac | Tyr | ttc | Phe |
| 297 | aac | Asn | aac | Asn | aac | Asn | aac | Asn |
| 298 | agc | Ser | agc | Ser | agc | Ser | agc | Ser |
| 299 | acg | Thr | acg | Thr | acg | Thr | acg | Thr |
| 300 | tac | Tyr | ttc | Phe | ttc | Phe | tac | Tyr |
| 301 | cgt | Arg | cgt | Arg | cgt | Arg | cgt | Arg |
| 302 | gtg | Val | gtg | Val | gtg | Val | gtg | Val |
| 303 | gtc | Val | gtc | Val | gtc | Val | gtc | Val |
| 304 | agc | Ser | agc | Ser | agc | Ser | agc | Ser |
| 305 | gtc | Val | gtc | Val | gtc | Val | gtc | Val |
| 306 | ctc | Leu | ctc | Leu | ctc | Leu | ctc | Leu |
| 307 | acc | Thr | acc | Thr | acc | Thr | acc | Thr |
| 308 | gtc | Val | gtt | Val | gtc | Val | gtc | Val |
| 309 | ctg | Leu | gtg | Val | ctg | Leu | ctg | Leu |
| 310 | cac | His | cac | His | cac | His | cac | His |
| 311 | cag | Gln | cag | Gln | cag | Gln | cag | Gln |
| 312 | gac | Asp | gac | Asp | gac | Asp | gac | Asp |
| 313 | tgg | Trp | tgg | Trp | tgg | Trp | tgg | Trp |
| 314 | ctg | Leu | ctg | Leu | ctg | Leu | ctg | Leu |
| 315 | aat | Asn | aac | Asn | aac | Asn | aac | Asn |
| 316 | ggc | Gly | ggc | Gly | ggc | Gly | ggc | Gly |
| 317 | aag | Lys | aag | Lys | aag | Lys | aag | Lys |
| 318 | gag | Glu | gag | Glu | gag | Glu | gag | Glu |
| 319 | tac | Tyr | tac | Tyr | tac | Tyr | tac | Tyr |
| 320 | aag | Lys | aag | Lys | aag | Lys | aag | Lys |
| 321 | tgc | Cys | tgc | Cys | tgc | Cys | tgc | Cys |
| 322 | aag | Lys | aag | Lys | aag | Lys | aag | Lys |
| 323 | gtc | Val | gtc | Val | gtc | Val | gtc | Val |
| 324 | tcc | Ser | tcc | Ser | tcc | Ser | tcc | Ser |
| 325 | aac | Asn | aac | Asn | aac | Asn | aac | Asn |
| 326 | aaa | Lys | aaa | Lys | aaa | Lys | aaa | Lys |
| 327 | gcc | Ala | ggc | Gly | gcc | Ala | ggc | Gly |
| 328 | ctc | Leu | ctc | Leu | ctc | Leu | ctc | Leu |
| 329 | cca | Pro | cca | Pro | cca | Pro | ccg | Pro |
| 330 | gcc | Ala | gcc | Ala | gcc | Ala | tcc | Ser |
| 331 | ccc | Pro | ccc | Pro | ccc | Pro | tcc | Ser |
| 332 | atc | Ile | atc | Ile | atc | Ile | atc | Ile |
| 333 | gag | Glu | gag | Glu | gag | Glu | gag | Glu |
| 334 | aaa | Lys | aaa | Lys | aaa | Lys | aaa | Lys |
| 335 | acc | Thr | acc | Thr | acc | Thr | acc | Thr |
| 336 | atc | Ile | atc | Ile | atc | Ile | atc | Ile |
| 337 | tcc | Ser | tcc | Ser | tcc | Ser | tcc | Ser |
| 338 | aaa | Lys | aaa | Lys | aaa | Lys | aaa | Lys |
| 339 | gcc | Ala | acc | Thr | gcc | Thr | gcc | Ala |
| 340 | aaa | Lys | aaa | Lys | aaa | Lys | aaa | Lys |

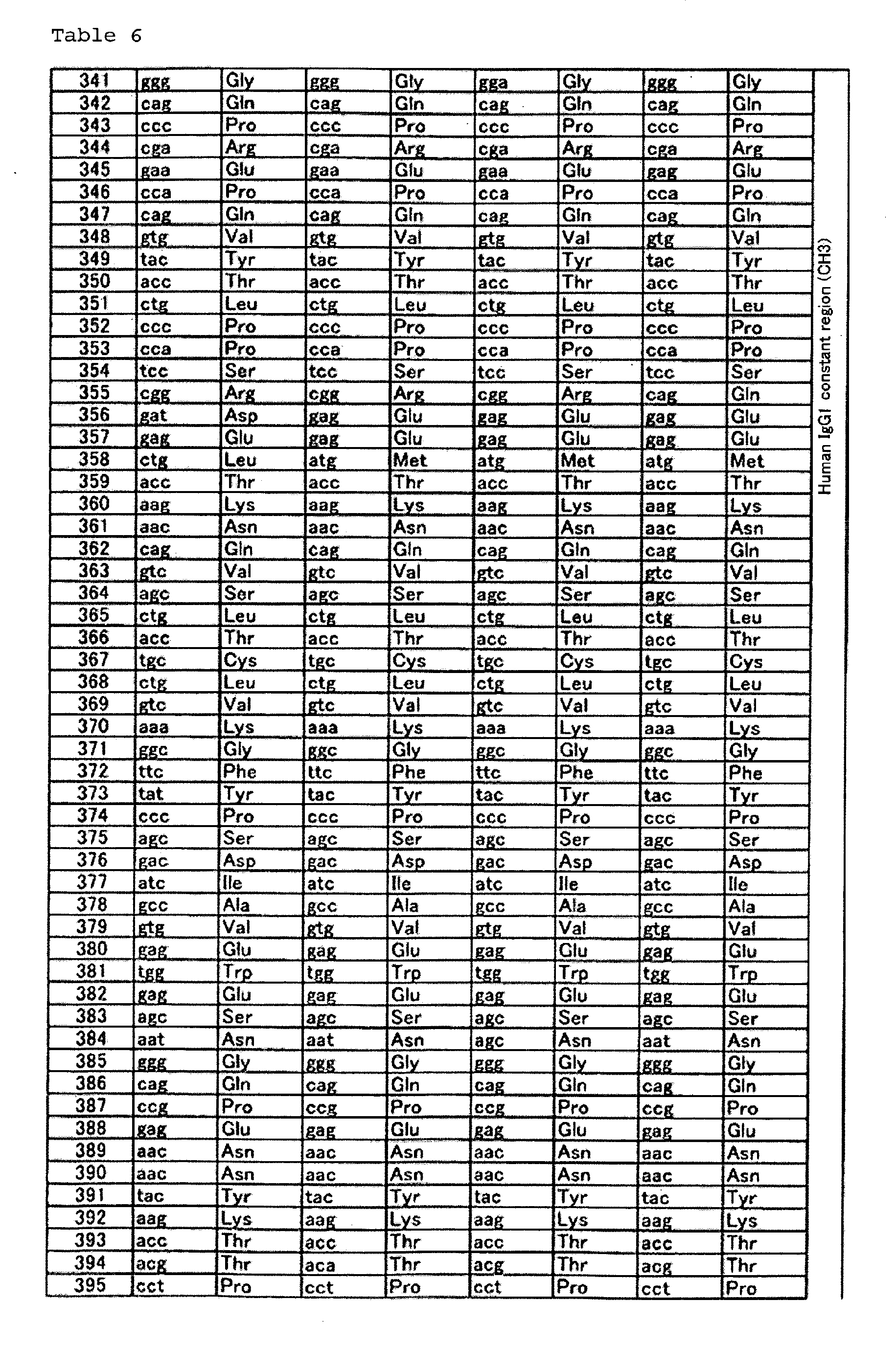

**Table 7**

| | | | | | | | | |
|---|---|---|---|---|---|---|---|---|
| 396 | ccc | Pro | ccc | Pro | ccc | Pro | ccc | Pro |
| 397 | gtg | Val | atg | Met | atg | Met | gtg | Val |
| 398 | ctg | Leu | ctg | Leu | ctg | Leu | ctg | Leu |
| 399 | gac | Asp | gac | Asp | gac | Asp | gac | Asp |
| 400 | tcc | Ser | tcc | Ser | tcc | Ser | tcc | Ser |
| 401 | gac | Asp | gac | Asp | gac | Asp | gac | Asp |
| 402 | ggc | Gly | ggc | Gly | ggc | Gly | ggc | Gly |
| 403 | tcc | Ser | tcc | Ser | tcc | Ser | tcc | Ser |
| 404 | ttc | Phe | ttc | Phe | ttc | Phe | ttc | Phe |
| 405 | ttc | Phe | ttc | Phe | ttc | Phe | ttc | Phe |
| 406 | ctc | Leu | ctc | Leu | ctc | Leu | ctc | Leu |
| 407 | tac | Tyr | tac | Tyr | tac | Tyr | tac | Tyr |
| 408 | agc | Ser | agc | Ser | agc | Ser | agc | Ser |
| 409 | aag | Lys | aag | Lys | aag | Lys | agg | Arg |
| 410 | ctc | Leu | ctc | Leu | ctc | Leu | cta | Leu |
| 411 | acc | Thr | acc | Thr | acc | Thr | acc | Thr |
| 412 | gtg | Val | gtg | Val | gtg | Val | gtg | Val |
| 413 | gac | Asp | gac | Asp | gac | Asp | gac | Asp |
| 414 | aag | Lys | aag | Lys | aag | Lys | aag | Lys |
| 415 | agc | Ser | agc | Ser | agc | Ser | agc | Ser |
| 416 | agg | Arg | agg | Arg | agg | Arg | agg | Arg |
| 417 | tgg | Trp | tgg | Trp | tgg | Trp | tgg | Trp |
| 418 | cag | Gln | cag | Gln | cag | Gln | cag | Gln |
| 419 | cag | Gln | cag | Gln | cag | Gln | gag | Glu |
| 420 | ggg | Gly | ggg | Gly | ggg | Gly | ggg | Gly |
| 421 | aac | Asn | aac | Asn | aac | Asn | aat | Asn |
| 422 | gtc | Val | gtc | Val | atc | Ile | gtc | Val |
| 423 | ttc | Phe | ttc | Phe | ttc | Phe | ttc | Phe |
| 424 | tca | Ser | tca | Ser | tca | Ser | tca | Ser |
| 425 | tgc | Cys | tgc | Cys | tgc | Cys | tgc | Cys |
| 426 | tcc | Ser | tcc | Ser | tcc | Ser | tcc | Ser |
| 427 | gtg | Val | gtg | Val | gtg | Val | gtg | Val |
| 428 | atg | Met | atg | Met | atg | Met | atg | Met |
| 429 | cat | His | cat | His | cat | His | cat | His |
| 430 | gag | Glu | gag | Glu | gag | Glu | gag | Glu |
| 431 | gct | Ala | gct | Ala | gct | Ala | gct | Ala |
| 432 | ctg | Leu | ctg | Leu | ctg | Leu | ctg | Leu |
| 433 | cac | His | cac | His | cac | His | cac | His |
| 434 | aac | Asn | aac | Asn | aac | Asn | aac | Asn |
| 435 | cac | His | cac | His | cgc | Arg | cac | His |
| 436 | tac | Tyr | tac | Tyr | ttc | Phe | tac | Tyr |
| 437 | acg | Thr | acg | Thr | acg | Thr | aca | Thr |
| 438 | cag | Gln | cag | Gln | cag | Gin | cag | Gln |
| 439 | aag | Lys | aag | Lys | aag | Lys | aag | Lys |
| 440 | agc | Ser | agc | Ser | agc | Ser | agc | Ser |
| 441 | etc | Leu | ctc | Leu | ctc | Leu | ctc | Leu |
| 442 | tcc | Ser | tcc | Ser | tcc | Ser | tcc | Ser |
| 443 | ctg | Leu | ctg | Leu | ctg | Leu | ctg | Leu |
| 444 | tct | Ser | tct | Ser | tct | Ser | tct | Ser |
| 445 | ccg | Pro | ccg | Pro | ccg | Pro | ctg | Leu |
| 446 | ggt | Gly | ggt | Gly | ggt | Gly | ggt | Gly |
| 447 | aaa | Lys | aaa | Lys | aaa | Lys | aaa | Lys |

The Fc region used as a base for an Fc receptor binding portion in the present invention is not limited to Fc regions of naturally occurring antibodies, and may be one formed by subjecting part of an amino acid sequence of these Fc regions to a modification such as deletion, addition, or substitution or may be a synthetic polypeptide consisting of a corresponding amino acid sequence. Substitution of an amino acid residue in an Fc region as a base by a cysteine residue may be carried out by a known method employing gene recombination technology, for example, a site-directed mutagenesis method.

When the Fc receptor binding portion in the present invention contains an Fc region mutant, this typically contains a sugar chain binding site. It is not always necessary for the sugar chain binding site to have a sugar chain binding thereto, but in terms of promoting ADCC activity it is preferable for it to have at least one sugar chain binding thereto. The sugar chain is not particularly limited as long as it is a sugar chain formed from at least one monosaccharide, and it may be any sugar chain, for example, a sugar chain binding to an Fc region of an antibody heavy chain or a glycoform thereof, or a synthetic sugar chain. For example, the sugar chain represented by the formula below is an example of a sugar chain binding to a sugar chain binding site in an Fc region of IgG1 antibody heavy chain.

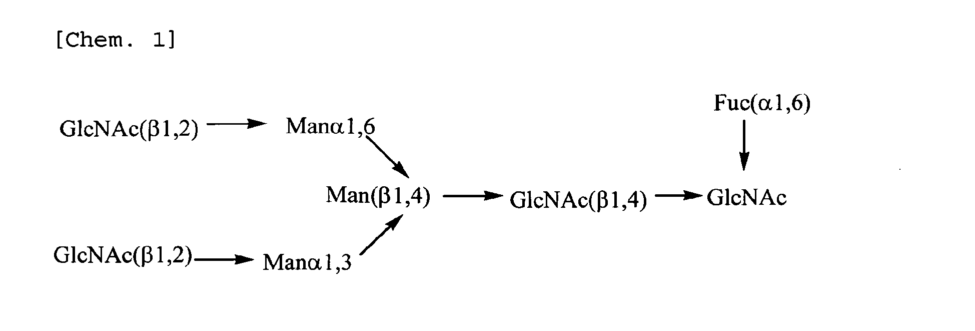

It is generally known that the structure of a sugar chain binding to an Fc region is intimately involved in an effector function, and for the purpose of enhancing the function it is also preferable to use a sugar chain formed by subjecting a natural type sugar chain to appropriate modification. Examples of a sugar chain formed by subjecting a natural type sugar chain to modification include a sugar chain having a structure obtained by subjecting fucose (Fuc (α1,6)) binding to N-acetylglucosamine (GlcNAc) in the above formula to a modification such as addition, deletion, or substitution. A sugar chain binding to a glycosylation site may be either an O-linked type or an N-linked type, but a sugar chain formed from an N-linked type sugar chain is preferably used.

In general, a sugar chain of an Fc region is very important to the ADCC activity of an IgG antibody, and it is known that the mere presence of a sugar chain or the sugar chain structure thereof is intimately involved in ADCC activity. Furthermore, binding of a sugar chain to a glycosylation site (in particular, expression of an N type sugar chain) generally requires an Asn-X-Ser/Thr amino acid sequence (X being an any amino acid residue).

The polypeptide of the present invention preferably has, as a polypeptide site other than an Fc receptor binding portion, a site (cell binding portion) that recognizes a cell surface molecule. The cell surface molecule may be a molecule that is present on the surface of either a human cell or a nonhuman cell (mouse cell, etc.), but a human cell surface molecule is suitable.

Examples of the human cell surface molecule include a cytokine receptor, a cell adhesion molecule, a cancer cell surface molecule, a cancer stem cell surface molecule, a blood cell surface molecule, and a virus-infected cell surface molecule. Specific examples of such human cell surface molecules include CD3, CD11a, CD20, CD22, CD25, CD28, CD33, CD52, Her2/neu, an EGF receptor, EpCAM, MUC1, GD3, CEA, CA125, HLA-DR, a TNFα receptor, a VEGF receptor, CTLA-4, AILIM/ICOS, and an integrin molecule.

The polypeptide of the present invention may be constituted from, for example, a mutant of an antibody containing an Fc region in IgG or a mutant of a polypeptide (antibody-like molecule) such as a chimera molecule of an Fc region in IgG and a polypeptide molecule that recognizes a cell surface molecule (human cell surface molecule in particular). The mutants of an antibody and an antibody-like molecule may be used singly or in a combination thereof.
More specifically, the polypeptide of the present invention includes an antibody mutant in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue, and a chimera molecule mutant containing a cell binding portion and an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue. Compared with the antibody or chimera molecule before substitution, the polypeptide of the present invention has improved FcR-binding capability and/or effector function. In the polypeptide of the present invention, examples of the Fc region include the Fc region of human IgG, and more preferably the Fc region of human IgG1, human IgG2, human IgG3, or human IgG4. The Fc region of the present invention preferably has an N-linked type sugar chain on a glycosylation site as described above.

The antibody referred to in the present invention is not particularly limited as long as it is a molecule that has an antigen binding site and can induce an effector function (ADCC in particular), and examples thereof include natural type antibodies such as a human antibody and a nonhuman antibody such as a mouse antibody, and derivatives thereof, and recombinant antibodies that can induce an antigen-antibody reaction such as a chimera antibody, a humanized antibody, and a single chain antibody. They may be either monoclonal antibodies or polyclonal antibodies, and may be used singly or in a combination of two or more types. The derivatives of the natural type antibodies mean antibodies formed by introducing a mutation such as deletion, addition, or substitution to part of an amino acid sequence of the natural type antibody, and such mutation may be caused naturally or artificially. The antigen binding site includes VH and VL variable sites (hypervariable sites CDR1 to 3 in particular) in the Fab region of a natural type antibody and a region consisting of a sequence that is homologous thereto.

Among them, from the viewpoint of good ADCC activity being exhibited and easy application to antibody medicine, a recombinant antibody, in particular a chimera antibody, a humanized antibody, etc. is preferably used. The chimera antibody means an antibody formed from at least two heterologous or allogeneic fusion proteins, and examples thereof include an antibody formed from the Fc region of human IgG and the Fab region of a nonhuman IgG (mouse IgG, etc.). Examples of the humanized antibody include an antibody in which a hypervariable region of a nonhuman IgG (mouse IgG, etc.) is fused with a human IgG-derived Fc region-containing remaining region.

As the antibody, antibodies to various types of antigens may be used. Antigens that are recognized by these antibodies include polypeptides such as a receptor like a transmembrane molecule and a ligand like a growth factor, and non-polypeptide antigens described in US Patent No. 5091178, such as a tumor-related glycolipid antigen. Specific examples of the antigen include renin; a growth hormone such as human growth hormone or bovine growth hormone; growth hormone releasing factor; parathyroid hormone; thyroid stimulating hormone; lipoprotein; α-1-antitrypsin; insulin A-chain; insulin B-chain; proinsulin; follicle stimulating hormone; calcitonin; luteinizing hormone; glucagon; a coagulation factor such as factor VIIIC, factor IX, tissue factor, or von Willebrand factor; an anti-coagulation factor such as protein C; atrial natriuretic factor; a pulmonary surfactant; a plasminogen activator such as urokinase, or human urinary or tissue-type plasminogen activator (t-PA); bombesin; thrombin; hematopoietic growth factor; tumor necrosis factor-α and -β; enkephalinase; RANTES (regulated on activation normally T-cell expressed and secreted); human macrophage inflammation protein (MIP-1-α); a serum albumin such as human serum albumin; a Muellerian inhibiting substance; relaxin A-chain; relaxin B-chain; prorelaxin; a mouse gonadotropin-related peptide; a microbial protein such as beta-lactamase; DNASE; IgE; a cytotoxic T lymphocyte-related antigen (CTLA) such as CTLA-4; inhibin; activin; vascular endothelium growth factor (VEGF); a receptor for a hormone or a growth factor; protein A or D; rheumatoid factor; a brain-derived neurotrophic factor (BDNF), a neurotrophin-3, -4, -5, or -6 (NT-3, NT-4, NT-5, or NT-6), or nerve growth factor such as NGF-β; platelet derived growth factor (PDGF); a fibroblast growth factor such as aFGF or bFGF; epithelial growth factor (EGF); a transforming growth factor (TGF) such as TGF-α or TGF-β (TGF-β1, TGF-β2, TGF-β3, TGF-β4, TGF-β5, etc.); insulin-like growth factor-I and -II (IGF-I and IGF-II); des(1-3)-IGF-I (brain IGF-I), insulin-like growth factor binding protein; a CD protein such as CD3, CD4, CD8, CD19, or CD20; erythropoietin; bone inducing factor; immunotoxin; bone morphogenetic protein (BMP); an interferon such as interferon-α, -β, or -γ; a colony stimulating factor (CSF) such as M-CSF, GM-SCF, or G-CSF; an interleukin (IL) such as IL-1 to IL-10; super oxide dismutase; T cell receptor; surface membrane protein; decay-accelerating factor; a viral antigen such as part of the AIDS envelope; transport protein; homing receptor; addressin (addressin); regulatory protein; an integrin such as CD11a, CD11b, CD11c, CD18, ICAM, VLA-4, or VCAM; a tumor-related antigen such as HER2, HER3, or HER4 receptor; and fragments of these polypeptides.

Examples of suitable molecular targets for the antibody include a CD protein such as CD3, CD4, CD8, CD19, CD20, or CD34; the ErbB receptor family such as HER2, HER3, or HER4 receptor; a cell adhesion molecule such as LFA-1, Mac1, p150.95, VLA-4, ICAM-1, VCAM, and αv/β3 integrin containing any one of an α or β subunit thereof (e.g. anti-CD11a, anti-CD18, or anti-CD11b antibody); a growth factor such as VEGF; IgE; a blood type antigen; an flk2/flt3 receptor; an obesity (OB) receptor; an mpl receptor; CTLA-4; and protein C.

Among the antigens cited as examples above, a soluble antigen and a fragment thereof may be used as an immunogen for producing an antibody. For example, with respect to a transmembrane molecule such as a receptor, for example, a fragment of an extracellular domain of a receptor may be used as an immunogen. Alternatively, a cell that expresses a transmembrane molecule may be used as an immunogen. Such a cell may be extracted from a natural source (e.g. tumor cell line), or may be a cell that has been transformed so as to express an antigen recognition region of a transmembrane molecule, etc. using a recombinant vector.

These antibodies may have, other than the antigen binding site, a site that can bind to a cell surface molecule. In accordance with an antibody having such a constitution, since the efficiency with which a target cell is recognized can be improved, it is possible for a high effector function to be exhibited with a small amount of antibody.

The present invention provides, but is not limited to, a mutant of for example an anti-CD20 antibody described in any one of (1) to (4) below.
(1) An antibody mutant containing an H chain having the amino acid sequence described in SEQ ID No: 51 as CDR1, the amino acid sequence described in SEQ ID No: 53 as CDR2, the amino acid sequence described in SEQ ID No: 55 as CDR3, and an amino acid sequence described in any one of SEQ ID Nos: 13 to 18 as CH,
(2) an antibody mutant containing an H chain having an amino acid sequence described in any one of SEQ ID Nos: 25 to 30 (amino acid sequence of H chain full length),
(3) an antibody mutant containing an H chain having the amino acid sequence described in SEQ ID No: 51 as CDR1, the amino acid sequence described in SEQ ID No: 53 as CDR2, the amino acid sequence described in SEQ ID No: 55 as CDR3, and an amino acid sequence described in any one of SEQ ID Nos: 1 to 6 as the Fc region,
(4) an antibody mutant containing an H chain described in any one of (1), (2), or (3) above, and an L chain described in (i) or (ii) below,
   (i) an L chain having the amino acid sequence described in SEQ ID No: 67 as CDR1, the amino acid sequence described in SEQ ID No: 69 as CDR2, the amino acid sequence described in SEQ ID No: 71 as CDR3, and the amino acid sequence described in SEQ ID No: 73 as CL,
   (ii) an L chain having the amino acid sequence described in SEQ ID No: 65 (amino acid sequence of full length L chain).

Furthermore, the present invention also provides a mutant of for example an anti-CD20 antibody described in any one of (1) to (4) below.
(1) an antibody mutant containing an H chain having CDR1 coded by the base sequence described in SEQ ID No: 52, CDR2 coded by the base sequence described in SEQ ID No: 54, CDR3 coded by the base sequence described in SEQ ID No: 56, and CH coded by a base sequence described in any one of SEQ ID Nos: 19 to 24,
(2) an antibody mutant containing an H chain coded by a base sequence described in any one of SEQ ID Nos: 31 to 36,
(3) an antibody mutant containing an H chain having CDR1 coded by the base sequence described in SEQ ID No: 52, CDR2 coded by the base sequence described in SEQ ID No: 54, CDR3 coded by the base sequence described in SEQ ID No: 56, and an Fc region coded by a base sequence described in any one of SEQ ID Nos: 7 to 12,
(4) an antibody mutant having as a pair an H chain described in any one of (1), (2), and (3) above and an L chain described in (i) or (ii) below,
   (i) an L chain having CDR1 coded by the base sequence described in SEQ ID No: 68, CDR2 coded by the base sequence described in SEQ ID No: 70, CDR3 coded by the base sequence described in SEQ ID No: 72, and CL coded by the base sequence described in SEQ ID No: 74,
   (ii) an L chain coded by the base sequence described in SEQ ID No: 66.

The antibody-like molecule referred to in the present invention is different from an antibody in terms of not having an antigen binding site, but has a cell binding portion in the molecule and has a similar function to that of an antibody in terms of being capable of contributing to exhibition of an effector function via binding to a cell surface molecule at said portion.

As the antibody-like molecule in the present invention, a recombinant molecule such as a chimera molecule containing a cell binding portion and an Fc region, etc. may be used. That is, the present invention provides a chimera molecule mutant containing a cell binding portion and an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue. The chimera molecule mutant of the present invention may contain, as long as it has an effector function, a peptide region between the cell binding portion and the Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue. As the peptide region, (1) to (3) below can be cited as examples but should not be construed as being limited thereto. (1) A linker, (2) an antibody hinge region, and (3) a linker and an antibody hinge region. The 'antibody hinge region' includes not only the full length of an antibody hinge region, but also part of an antibody hinge region as long as it contributes to an effector function (or does not inhibit an effector function).

Examples of the cell surface molecule are the same as those described above. That is, the present invention provides a chimera molecule mutant that binds to at least one human cell surface molecule selected from the group consisting of a cytokine receptor, a cell adhesion molecule, a cancer cell surface molecule, a cancer stem cell surface molecule, a blood cell surface molecule, and a virus-infected cell surface molecule. The cell binding portion may be formed from, for example, a binding domain of an adhesion protein. Furthermore, the cell binding portion may be formed from, for example, a variable region of an antibody H chain and/or L chain.
The technique of the present invention is a technique that can be applied as long as it is a molecule that contains an antibody Fc region and a site having the function of binding to an antigen in the same manner as for an antigen recognition site of an antibody, and that has an effector function. Therefore, a target to which the technique of the present invention is applied is not limited to an antibody molecule. The technique of the present invention can be applied in general to a chimera molecule containing a cell binding portion (e.g. a binding domain of an adhesion protein, an adhesion molecule, a signal molecule, etc.) and an antibody Fc region. The adhesion protein referred to here is for example a molecule that mediates a cell-to-cell interaction and a molecule that has as a cell recognition site an extracellular region within the structure. Since the antibody-like molecule in the present invention utilizes the extracellular region structure as a binding domain, it can recognize a cell surface molecule to which this molecule inherently binds.

Examples of the adhesion protein include the immunoglobulin superfamily (IgSF), a receptor for cytokines such as a receptor having tyrosine kinase activity (receptor tyrosine kinase), erythropoietin and nerve growth factor receptor superfamily, an extracellular domain of a cell surface molecule such as integrin, cadherin, or (E-, L-, and P-) selectin, and a polypeptide molecule that has affinity therewith (can bind thereto). The IgSF includes, in addition to a transmembrane type cell adhesion molecule like NCAM or L1, a GPI-anchor type cell adhesion molecule having no transmembrane portion.
More specifically, the present invention provides a chimera molecule mutant that binds to at least one human cell surface molecule selected from the group consisting of CD3, CD11a, CD20, CD22, CD25, CD28, CD33, CD52, Her2/neu, EGF receptor, EpCAM, MUC1, GD3, CEA, CA125, HLA-DR, TNFα receptor, VEGF receptor, AILIM/ICOS, CTLA-4, B7h, CD80, CD86, and an integrin molecule.

The antibody-like molecule and mutant thereof in the present invention preferably have a structure having a binding domain (in the present description, this might be expressed as a 'cell binding portion' or a 'site that recognizes a cell surface molecule') on an N terminal side and an Fc region on a C terminal side, but are not limited thereto, and may have a structure having an Fc region on an N terminal side and a binding domain on a C terminal side. The Fc region forming an antibody-like molecule and the mutant thereof preferably retains CH2 and CH3 domains, and the binding domain forming the antibody-like molecule and mutant thereof is preferably disposed in a site corresponding to CH1 of an antibody heavy chain and/or an antibody light chain.

In the present invention, chimera molecule mutants described in for example (1) to (3) below are provided.
(1) A chimera molecule mutant containing the amino acid sequence described in SEQ ID No: 89 as a cell binding portion, and an amino acid sequence described in any one of SEQ ID Nos: 1 to 6 as an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue,
(2) a chimera molecule mutant containing the amino acid sequence described in SEQ ID No: 89 as a cell binding portion, the amino acid sequence described in SEQ ID No: 93 as an antibody hinge region, and an amino acid sequence described in any one of SEQ ID Nos: 1 to 6 as an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue, and
(3) a chimera molecule mutant consisting of an amino acid sequence described in any one of SEQ ID Nos: 75 to 80.

Furthermore, the present invention provides chimera molecule mutants described in for example (1) to (3) below.
(1) A chimera molecule mutant containing a cell binding portion coded by the base sequence described in SEQ ID No: 90, and an Fc region, coded by a base sequence described in any one of SEQ ID Nos: 7 to 12, in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue,
(2) a chimera molecule mutant containing a cell binding portion coded by the base sequence described in SEQ ID No: 90, an antibody hinge region coded by the base sequence described in SEQ ID No: 94, and an Fc region, coded by a base sequence described in any one of SEQ ID Nos: 7 to 12, in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue, and (3) a chimera molecule mutant coded by a base sequence described in any one of SEQ ID Nos: 81 to 86.

The chimera molecule mutant of the present invention may further have a linker sequence between the cell binding portion and the antibody hinge region. The linker sequence is not particularly limited as long as it contributes to an effector function (or does not inhibit an effector function), and examples thereof include a linker sequence consisting of the amino acid sequence described in SEQ ID No: 91. Examples of DNA coding for the amino acid sequence described in SEQ ID No: 91 include DNA having the base sequence described in SEQ ID No: 92, but are not limited thereto. The 'antibody hinge region' of the present invention includes not only the full length of an antibody hinge region but also part of an antibody hinge region as long as it contributes to an effector function (or does not inhibit an effector function).

The polypeptide of the present invention may be produced by a known method employing gene recombination technology, etc. The polypeptide of the present invention may be produced by a method in which an isolated nucleic acid that codes for the polypeptide of the present invention is used, a vector containing said nucleic acid is prepared, a host cell or a host organism having said vector is obtained, and the host cell or the host organism is cultured so as to express the polypeptide coded by the nucleic acid.

When the polypeptide is formed from an antibody, a normal method for producing an antibody may be employed. A method for producing an antibody mutant may employ a method described in Molecular Cloning Second Edition, Current Protocols In Molecular Biology, Antibodies, A Laboratory manual, Cold Spring Harbor Laboratory, 1988, Monoclonal Antibodies: Principles and Practice, Third Edition, Acad. Press, 1993, Antibody Engineering, A Practical Approach, IRL Press at Oxford University Press, 1996, etc., and for example it may be obtained by expression in a host cell as described below. Furthermore, a similar method may be employed in the production of an antibody-like molecule.

Production of the polypeptide includes for example a step of isolating a nucleic acid consisting of a sequence that has been substituted so as to code for a cysteine residue instead of an amino acid selected from the group consisting of, as Kabat EU index numbers, the 293rd amino acid, 294th amino acid, 296th amino acid, 297th amino acid, 298th amino acid, and 300th amino acid in a nucleic acid coding for a polypeptide containing an Fc region as a base for an Fc receptor binding portion; a step of producing a replicable recombinant vector by incorporating the isolated nucleic acid coding for the polypeptide into an expression vector containing an appropriate promoter; a step of transforming a host cell or a host organism using the recombinant vector thus obtained; and a step of culturing the transformed host cell or host organism and producing the polypeptide.

The polypeptide containing an Fc region as a base may have a modification such as deletion, addition, or substitution in an amino acid other than an amino acid selected from the group consisting of, as Kabat EU index numbers, the 293rd amino acid, 294th amino acid, 296th amino acid, 297th amino acid, 298th amino acid, and 300th amino acid, or in a sugar chain binding to a glycosylation site, etc.

The nucleic acid coding for the polypeptide of the present invention may be obtained by a commonly used method for introducing a mutation to a base sequence, for example, it may be prepared by PCR using as a template cDNA containing an Fc region as a base by the use of a synthetic oligonucleotide containing a nucleic acid sequence in which the nucleic acid sequence coding for the amino acid residue to be substituted in the Fc region as a base is replaced by 'TGC' or 'TCT' coding for cysteine. As long as an effector function is not inhibited, the polypeptide of the present invention may be subjected to another modification during the step of introducing the cysteine substitution mutation or after the introduction, for example, a modification such as deletion, addition, or substitution of an amino acid sequence or part of a sugar chain binding to a sugar chain binding site. In the present invention in particular, it is preferable to subject a sugar chain binding to a sugar chain binding site of an Fc region to a known modification since an effect in improving an effector function can be obtained.

The replicable recombinant vectors may be prepared by inserting a nucleic acid coding for the polypeptide of the present invention (DNA fragment or full length cDNA) downstream of a promoter of an appropriate expression vector. The expression vector may be appropriately selected according to the type of host cell or host organism into which it is introduced, and one that is autonomously replicable in a host cell or can be inserted into a chromosome, and contains a promoter that can transcribe a nucleic acid coding for a target polypeptide may be used. These recombinant vectors may be used singly or in a combination of two or more types.

In the present invention, two or more recombinant vectors may be used in combination. As recombinant vectors used in combination, for example, an expression vector containing an appropriate promoter and a nucleic acid coding for a complementary antibody light chain or heavy chain, or a binding domain of a desired receptor or a ligand, a nucleic acid coding for a desired glycosylation enzyme, etc. may be used. The promoter is preferably operably linked to the nucleic acid. In accordance with the use of such recombinant vectors in combination, a polypeptide that can exhibit a higher effector function can be obtained. When two or more recombinant vectors are used, the host cells or host organisms may be identical or different.

As the host cell, any one such as a yeast, animal cell, insect cell, or plant cell may be used as long as a target gene can be expressed. Furthermore, in the present invention, as a host cell, a cell for which the activity of a modifying enzyme on a sugar chain binding to a sugar chain binding site that affects an effector function is degraded or eliminated may be selected, or a cell for which same activity is degraded or eliminated by an artificial method may be used. Such enzymes include enzymes involved in the modification of an N-glycoside binding sugar chain, and specific examples thereof include an enzyme involved in synthesis of intracellular sugar nucleotide GDP-fucose, and an enzyme involved in glycosylation in which the 1-position of fucose α-binds to the 6-position of N-acetylglucosamine, which is an N-glycoside binding complex type sugar chain reducing terminal, for example, a fucose transfer enzyme such as fucosyltransferase (FUT). Examples of a host organism include an animal individual, a plant individual, a bacterium, and a virus.

When a yeast is used as a host cell, as an expression vector, for example, YEP13 (ATCC37115), YEp24 (ATCC37051), YCp50 (ATCC37419), etc. may be used. As a promoter, any promoter may be used as long as it can be expressed in a yeast strain, and examples thereof include glycolytic pathway gene promoters such as hexose kinase, PHO5 promoter, PGK promoter, GAP promoter, ADH promoter, gal1 promoter, gal10 promoter, heat shock protein promoter, MFα1 promoter, and CUP1 promoter. As the host cell, microorganisms belonging to the Saccharomyces genus, the Schizosaccharomyces genus, the Kluyveromyces genus, the Trichosporon genus, the Schwanniomyces genus, etc. can be cited, and examples thereof include Saccharomyces cerevisiae, Schizosaccharomyces pombe, Kluyveromyces lactis, Trichosporon pullulans, and Schwanniomyces alluvius.

As a method for introducing a recombinant vector into a yeast, any method may be used as long as it is a method for introducing DNA into yeast, and examples thereof include an electroporation method (Methods. Enzymol., 194, 182 (1990)), a spheroplast method (Proc. Natl. Acad. Sci. USA, 75, 1929 (1978)), a lithium acetate method (J. Bacteriology, 153, 163 (1983)), and a method described in Proc. Natl. Acad. Sci. USA, 75, 1929 (1978).

When an animal cell is used as a host, examples of expression vectors include pcDNAI, pcDM8 (Funakoshi Corporation), pAGE107 (JP, A, 3-22979; Cytotechnology, 3, 133, (1990)), pAS3-3 (JP, A, 2-227075), pCDM8 (Nature, 329, 840, (1987)), pcDNAI/Amp (Invitrogen), pREP4 (Invitrogen), pAGE103 (J. Biochemistry, 101, 1307 (1987)), and pAGE210. As a promoter, any promoter may be used as long as it can be expressed in an animal cell, and examples thereof include cytomegalovirus (CMV) IE (immediate early) gene promoter, SV40 early promoter, retrovirus promoter, metallothionein promoter, heat shock promoter, and SRα promoter. Furthermore, human CMV IE gene enhancer may be used together with the promoter. Examples of the host cell include Namalwa (Namalwa) cells, which are human cells, COS cells, which are monkey cells, CHO cells, which are Chinese hamster cells, HBT5637 (JP, A, 63-299), rat myeloma cells, mouse myeloma cells, Syrian hamster kidney-derived cells, embryonic stem cells, and fertilized egg cells.

As a method for introducing a recombinant vector into an animal cell, any method may be used as long as it is a method for introducing DNA into an animal cell, and examples thereof include an electroporation method (Cytotechnology, 3, 133 (1990)), a calcium phosphate method (JP, A, 2-227075), a lipofection method (Proc. Natl. Acad. Sci. USA, 84, 7413 (1987)), an injection method (Manipulating the Mouse Embryo: A Laboratory Manual), a method employing a particle gun (gene gun) (Japanese Patent Nos. 2606856 and 2517813), a DEAE-dextran method (Biomanual Series 4-Gene Transfer and Expression/Analysis Method (Yodosha) Ed. By Takashi Yokota and Kenichi Arai (1994)), and a virus vector method (Manipulating the Mouse Embryo Second Edition).

When an insect cell is used as a host, a protein may be expressed by a method described in, for example, Current Protocols In Molecular Biology, Baculovirus Expression Vectors, A Laboratory Manual, W. H. Freeman and Company, New York (1992), Bio/Technology, 6, 47 (1988), etc. That is, a recombinant gene transfer vector and a baculovirus are cointroduced into an insect cell to thus obtain a recombinant virus in an insect cell culture supernatant, and the insect cell is then infected with the recombinant virus, thus enabling a protein to be expressed. Examples of gene transfer vectors used in said method include pVL1392, pVL1393, and pBlueBacIII (all from Invitrogen).

As the baculovirus, for example, Autographa californica nuclear polyhedrosis virus, which is a virus that infects insects of the Noctuidae family, etc. may be used. As insect cells, Sf9 or Sf21 (Current Protocols In Molecular Biology, Baculovirus Expression Vectors, A Laboratory Manual, W. H. Freeman and Company, New York (1992)), which are Spodopterafrugiperda ovary cells, High5 (Invitrogen), which are Trichoplusiani ovary cells, etc. may be used.

Examples of a method for cointroducing the recombinant gene transfer vector and the baculovirus into an insect cell in order to prepare a recombinant virus include a calcium phosphate method (JP, A, 2-227075) and a lipofection method (Proc. Natl. Acad. Sci. USA, 84, 7413 (1987)).

When a plant cell is used as a host cell, examples of expression vectors include Ti plasmid and tobacco mosaic virus vector. As a promoter, any promoter may be used as long as it can be expressed in a plant cell, and examples thereof include cauliflower mosaic virus (CaMV) 35S promoter and rice actin 1 promoter. Examples of the host cell include tobacco, potato, tomato, carrot, soya bean, oilseed rape, alfalfa, rice, wheat, barley, etc. plant cells.

As a method for introducing a recombinant vector into a plant cell, any method may be used as long as it is a method for introducing DNA into a plant cell, and examples thereof include a method employing Agrobacterium (Agrobacterium) (JP, A, 59-140885, JP, A, 60-70080, WO94/00977), an electroporation method (JP, A, 60-251887), and a method employing a particle gun (gene gun) (Japanese Patent Nos. 2606856 and 2517813). As a gene expression method, other than direct expression, secretory production, expression of a fusion protein of the Fc region and another protein, etc. may be carried out in accordance with a method described in Molecular Cloning Second Edition above, etc.

A transformant into which one or more recombinant vectors have been introduced as above can form and accumulate a desired polypeptide in a culture by culturing in accordance with a known method using a medium appropriate to the host.

As a medium for culturing a transformant obtained by use of a prokaryotic organism such as E. coli or an eukaryotic organism such as a yeast as a host, either a natural medium or a synthetic medium may be used as long as it is a medium that contains a carbon source, a nitrogen source, an inorganic salt, etc. that the organism can utilize, and enables efficient culturing of the transformant to be carried out. As the carbon source, any carbon source may be used as long as the organism can utilize it, and glucose, fructose, sucrose, molasses containing same, a carbohydrate such as starch or starch hydrolysate, an organic acid such as acetic acid or propionic acid, an alcohol such as ethanol or propanol, etc. may be used. As the nitrogen source, ammonia, an ammonium salt of an inorganic acid or an organic acid such as ammonium chloride, ammonium sulfate, ammonium acetate, or ammonium phosphate, another nitrogen-containing compound, peptone, meat extract, yeast extract, corn steep liquor, casein hydrolysate, soybean cake, soybean cake hydrolysate, various types of fermentation microbes and digests thereof, etc. may be used. As the inorganic salt, monopotassium phosphate, dipotassium phosphate, magnesium phosphate, magnesium sulfate, sodium chloride, ferrous sulfate, manganese sulfate, copper sulfate, calcium carbonate, etc. may be used.

Culturing of a transformant using as a host a prokaryote such as E. coli or an eukaryote such as a yeast is usually carried out under aerobic conditions such as shaking culture or deep aeration stirring culture. The culture temperature may be 15°C to 40°C, and the culture time is usually 16 hours to 7 days. The pH during culturing is maintained at 3.0 to 9.0. Adjustment of pH is carried out using an inorganic or organic acid, an alkali solution, urea, calcium carbonate, ammonia, etc. Furthermore, an antibiotic such as ampicillin or tetracycline may be added to the medium as necessary during culturing. When a microorganism that has been transformed by a recombinant vector using an inducible promoter as a promoter is cultured, an inducer may be added to the medium as necessary. For example, when culturing a microorganism that has been transformed by a recombinant vector using lac promoter, isopropyl-β-D-thiogalactopyranoside, etc. may be added to the medium, and when culturing a microorganism that has been transformed by a recombinant vector using trp promoter, indole acrylic acid, etc. may be added to the medium.

As a medium for culturing a transformant that has been obtained using an animal cell as a host, a generally used medium such as RPMI1640 medium (The Journal of the American Medical Association, 199, 519 (1967)), Eagle MEM medium (Science, 122, 501 (1952)), Dulbecco-modified MEM medium (Virology, 8, 396 (1959)), 199 medium (Proceeding of the Society for the Biological Medicine, 73, 1 (1950)), Whitten medium (Developmental Engineering Experimental Manual-How to Make a Transgenic Mouse (Kodansha) Ed. by Motoya Katsuki (1987)), or a medium formed by adding fetal calf serum to the above medium may be used. Culturing is usually carried out under conditions of a pH of 6 to 8 at 30°C to 40°C in the presence of 5% CO₂, etc. for 1 to 7 days. Furthermore, an antibiotic such as kanamycin or penicillin may be added to the medium as necessary during culturing.

As a medium for culturing a transformant that has been obtained using an insect cell as a host, a generally used medium such as TNM-FH medium (Pharmingen), Sf-900IISFM medium (Life Technologies), ExCell400, ExCell405 (both from JRH Biosciences), or Grace's Insect Medium (Nature, 195, 788 (1962)) may be used. Culturing is usually carried out under conditions of a pH of 6 to 7 at 25°C to 30°C, etc. for 1 to 5 days. Furthermore, an antibiotic such as gentamicin may be added to the medium as necessary during culturing.

A transformant that has been obtained using a plant cell as a host may be cultured as a cell or by differentiating into a plant cell or organ. As a medium for culturing the transformant, a normally used medium such as Murashige and Skoog (MS) medium, White's medium, and a medium formed by adding plant hormones such as auxin, cytokinin, etc., to the above medium may be used. Culturing is usually carried out under conditions of a pH of 5 to 9 at 20°C to 40°C for 3 to 60 days. Furthermore, an antibiotic such as kanamycin or hygromycin may be added to a medium as necessary during culturing.

While being cultured, a transformant expresses a polypeptide naturally or by induction, and produces and accumulates it in the culture. As a method for expressing a polypeptide, other than direct expression, for example, secretory production, fusion protein expression, etc. may be carried out in accordance with a method described in the above Molecular Cloning Second Edition. As a method for producing a polypeptide, there is a method in which it is produced within a host cell, a method in which it is secreted outside a host cell, or a method in which it is produced on a host cell outer membrane, and the method may be selected by changing the host cell used or the structure of the polypeptide that is produced.

When a polypeptide is produced within a host cell or on a host cell outer membrane, the polypeptide may be actively secreted outside the host cell in accordance with a method by Paulson et al. (J. Biol. Chem., 264, 17619 (1989)), a method by Lowe et al. (Proc. Natl. Acad. Sci. USA, 86, 8227 (1989); Genes Develop., 4, 1288 (1990)), or a method described in JP, A, 05-336963, JP, A, 06-823021, etc.

That is, by the use of a gene recombination technique, by inserting into an expression vector DNA coding for a polypeptide and DNA coding for a signal peptide appropriate for expression of the polypeptide, introducing the expression vector into a host cell, and then expressing the polypeptide, a target polypeptide may be secreted actively outside the host cell. Furthermore, in accordance with a method described in JP, A, 2-227075, the amount produced may be increased by utilizing a gene amplification system using dihydrofolate reductase gene, etc. Moreover, by redifferentiating an animal or plant cell into which a gene has been introduced, an animal individual (transgenic nonhuman animal) or plant individual (transgenic plant) into which the gene has been introduced is created, and a polypeptide may be produced by the use of these individuals.

When the transformant is an animal individual or a plant individual, it is raised or cultivated in accordance with a usual method, a polypeptide is formed and accumulated, the polypeptide is collected from the animal individual or plant individual, and the polypeptide may thus be produced. As a method for producing a polypeptide using an animal individual, a method in which a target polypeptide is produced in an animal obtained by introducing a gene in accordance with for example a known method (American Journal of Clinical Nutrition, 63, 639S (1996); American Journal of Clinical Nutrition, 63, 627S (1996); Bio/Technology, 9, 830 (1991)) can be cited.

In the case of an animal individual, for example, a transgenic nonhuman animal into which DNA coding for a polypeptide has been introduced is raised, the polypeptide is formed and accumulated in the animal, and the polypeptide is collected from the animal, thus producing the polypeptide. As a location for formation and accumulation in the animal, for example, milk (JP, A, 63-309192), an egg, etc. of the animal can be cited. As a promoter used here, any promoter may be used as long as it can be expressed in an animal and, for example, α casein promoter, β casein promoter, β lactoglobulin promoter, whey acidic protein promoter, etc., which are mammary gland cell specific promoters, may be suitably used.

As a method for producing a polypeptide using a plant individual, there can be cited a method for producing a polypeptide in which, for example, a transgenic plant into which DNA coding for an antibody molecule has been introduced is cultivated in accordance with a known method (Soshiki Baiyo (Tissue Culture), 20 (1994); Soshiki Baiyo, 21 (1995); Trends in Biotechnology, 15, 45 (1997)), the polypeptide is formed and accumulated in the plant, and the polypeptide is collected from the plant. With regard to a polypeptide produced by a transformant into which a gene coding for the polypeptide has been introduced, for example, when a polypeptide is expressed within a cell in a dissolved state, after culturing is completed, cells are recovered by centrifugation, suspended in an aqueous buffer, and then disrupted by means of an ultrasonic homogenizer, a French press, a Manton-Gaulin homogenizer, a Dyno-Mill, etc., thus giving a cell-free extract. A purified preparation of the polypeptide may be obtained by subjecting a supernatant that has been obtained by centrifuging the cell-free extract to, singly or in combination, usual enzyme isolation and purification methods, that is, a solvent extraction method, a salting out method using ammonium sulfate, etc., a desalting method, a precipitation method using an organic solvent, an anion exchange chromatographic method using a resin such as diethylaminoethyl (DEAE)-Sepharose or DIAION HPA-75 (Mitsubishi Chemical Corporation), a cation exchange chromatographic method using a resin such as S-Sepharose FF (Pharmacia), a hydrophobic chromatographic method using a resin such as butyl Sepharose or phenyl Sepharose, a gel filtration method using a molecular sieve, an affinity chromatographic method, a chromatofocusing method, an electrophoresis method such as isoelectric focusing, etc.

Furthermore, when a polypeptide is expressed by forming an insoluble substance within a cell, cells are recovered, then disrupted in the same manner, and centrifuged, thus recovering an insoluble polypeptide substance as a precipitated fraction. The insoluble polypeptide substance thus recovered is solubilized using a protein denaturant. After this solubilized liquid is diluted or dialyzed to thus convert the polypeptide into the normal tertiary structure, a purified preparation of the polypeptide may be obtained by the same isolation and purification method as above.

When a polypeptide is secreted outside a cell, the polypeptide or a derivative thereof may be recovered in the culture supernatant. That is, a soluble fraction is obtained by treating the culture by a method such as centrifuging in the same manner as above, and a purified preparation of the polypeptide may be obtained from the soluble fraction by the same isolation and purification method as above.

Examples of polypeptides thus obtained include an antibody mutant, a fragment of an antibody mutant, and a fusion protein having an Fc region of an antibody mutant (a recombinant antibody for which an antigen antibody reaction can be induced such as a chimera antibody, a humanized antibody, or a single chain antibody; an antibody-like molecule such as a chimera molecule). That is, the present invention provides a method for producing an antibody mutant having an improved effector function, the method including a step of subjecting an amino acid residue of an antibody having an effector function to substitution with a cysteine residue, the amino acid residue being selected from the group consisting of, as Kabat EU index numbers, the 293rd amino acid, 294th amino acid, 296th amino acid, 297th amino acid, 298th amino acid, and 300th amino acid. More specifically, the present invention provides a method for producing an antibody mutant having an improved effector function, the method including the steps below.
(1) A step of subjecting an amino acid residue of an antibody having an effector function to substitution with a cysteine residue, the amino acid residue being selected from the group consisting of, as Kabat EU index numbers, the 293rd amino acid, 294th amino acid, 296th amino acid, 297th amino acid, 298th amino acid, and 300th amino acid,
(2) a step of introducing DNA coding for an L chain and DNA coding for an H chain having an Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue into a host cell or a host organism, thus expressing the DNAs, and
(3) a step of recovering an expression product.

As one embodiment of the present invention, first, an amino acid residue, of an antibody having an effector function known to a person skilled in the art, selected from the group consisting of, as Kabat EU index numbers, the 293rd amino acid, 294th amino acid, 296th amino acid, 297th amino acid, 298th amino acid, and 300th amino acid is replaced by a cysteine residue. In another embodiment of the present invention, first, an antibody having an effector function is produced, and subsequently an amino acid residue, of the antibody thus produced, selected from the group consisting of, as Kabat EU index numbers, the 293rd amino acid, 294th amino acid, 296th amino acid, 297th amino acid, 298th amino acid, and 300th amino acid is replaced by a cysteine residue. For example, first, an antibody that binds to a desired antigen is obtained by the method described below, and whether or not the antibody thus obtained has an effector function is then determined by a method known to a person skilled in the art, thus enabling an antibody having an effector function to be produced. Examples of methods for determining an effector function include a method described in Examples.

In the present invention, a method for subjecting an amino acid residue to substitution with a cysteine residue is not particularly limited and, for example, it may be carried out by a site-directed mutagenesis method (Oligonucleotide-directed mutagenesis using M13-derived vector: an efficient and general procedure for the production of point mutations in any fragment of DNA, Mark J. Zoller and Michael Smith, Nucleic Acids Research, Volume 10 Number 20, 6487-6500, 1982; Directed evolution of green fluorescent protein by a new versatile PCR strategy for site-directed and semi-random mutagenesis Asako Sawano and Atsushi Miyawaki, Nucleic Acids Research, Volume 28, Number 16, e78, 2000).

In the present invention, DNA coding for an L chain and DNA coding for an H chain having an Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue is subsequently introduced into a host cell or a host organism by a method known to a person skilled in the art, and the DNA is expressed. Subsequently, an expression product (an antibody mutant) may be recovered by utilizing a method known to a person skilled in the art.

DNA coding for an H chain having an Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue may be produced by dividing into partial DNAs. As a combination of partial DNAs, for example, DNA coding for a variable region and DNA coding for a constant region, or DNA coding for a Fab region and DNA coding for an Fc region can be cited, but the combination is not limited thereto. DNA coding for an L chain may also be produced by dividing into partial DNAs in the same manner.

A method for obtaining an antibody that binds to a desired antigen is explained below.
As a gene coding for an H chain or an L chain of an antibody, a known sequence may be used, or it may be obtained by a method known to a person skilled in the art. For example, it may be obtained from an antibody library or may be obtained by cloning a gene coding for an antibody from a hybridoma that produces a monoclonal antibody.
With regard to the antibody library, a large number of antibody libraries are already known, methods for preparing an antibody library are also known, and a person skilled in the art can therefore obtain an antibody library as appropriate. For example, with regard to an antibody phage library, publications such as Clackson et al., Nature 1991, 352: 624-8, Marks et al., J. Mol. Biol. 1991, 222: 581-97, Waterhouses et al., Nucleic Acids Res. 1993, 21: 2265-6, Griffiths et al., EMBO J. 1994, 13: 3245-60, Vaughan et al., Nature Biotechnology 1996, 14: 309-14, and JP, A, (PCT) 20-504970 may be referred to. Alternatively, a known method such as a method using an eukaryotic cell as a library (WO95/15393) or a ribosome display method may be used. Furthermore, a technique of acquiring a human antibody by panning using a human antibody library is also known. For example, a variable region of a human antibody is expressed on the surface of a phage as a single chain antibody (scFv) by a phage display method, and a phage that binds to an antigen may be selected. By analyzing the gene of the phage thus selected, a DNA sequence coding for a variable region of a human antibody that binds to an antigen may be determined. When the DNA sequence of an scFv that binds to an antigen is clarified, an appropriate expression vector may be prepared based on this sequence, and a human antibody may be obtained. These methods are already well-known, and may be referred to in WO92/01047, WO92/20791, WO93/06213, WO93/11236, WO93/19172, WO95/01438, or WO95/15388.

With regard to a method for acquiring a gene coding for an antibody from a hybridoma, known techniques are basically used; a desired antigen or a cell that expresses a desired antigen is used as a sensitizing antigen, immunization is carried out with this in accordance with a standard immunization method, the immune cell thus obtained is fused with a known parent cell by a standard cell fusion method, a monoclonal antibody-producing cell (hybridoma) is screened by a standard screening method, cDNA of a variable region (V region) of an antibody is synthesized from mRNA of the hybridoma thus obtained using reverse transcriptase, and this is linked to DNA coding for a desired antibody constant region (C region).

More specifically, although not particularly limited to the examples below, a sensitizing antigen for obtaining an antibody gene coding for an H chain and an L chain of the present invention includes both a complete antigen having immunogenicity and an incomplete antigen including a hapten that does not exhibit immunogenicity. For example, a full length protein, a partial peptide, etc. of a target protein may be used. Preparation of an antigen may be carried out by a method known to a person skilled in the art; for example, it may be carried out in accordance with a method using a baculovirus (e.g. WO98/46777, etc.), etc. Preparation of a hybridoma may be carried out in accordance with for example a method by Milstein et al., (G. Kohler and C. Milstein, Methods Enzymol. 1981, 73: 3-46), etc. When the immunogenicity of an antigen is low, immunization may be carried out by binding to a macromolecule having immunogenicity such as albumin. Furthermore, as necessary an antigen may be bound to another molecule to thus make a soluble antigen. When a transmembrane molecule such as a receptor is used as an antigen, an extracellular region portion of the receptor may be used as a fragment, or a cell that expresses the transmembrane molecule on the cell surface may be used as an immunogen.

An antibody-producing cell may be obtained by immunization of an animal using the above-mentioned appropriate sensitizing antigen. Alternatively, an antibody-producing cell may be formed by in vitro immunization of a lymphocyte that can produce an antibody. With regard to the animal that is subjected to immunization, various types of mammals may be used, and a rodent, Lagomorpha, or primate animal is generally used. Examples thereof include animals such as rodents such as mouse, rat, or hamster, Lagomorpha such as rabbit, and primates such as monkeys, for example Macaca fascicularis, Macaca mulatta, Papio hamadryas, or chimpanzee. In addition, a transgenic animal having the full human antibody gene repertoire is also known, and a human antibody may be obtained using such an animal (ref. WO96/34096; Mendez et al., Nat. Genet. 1997, 15: 146-56). Instead of using such a transgenic animal, for example, a human lymphocyte is sensitized in vitro with a desired antigen or a cell that expresses a desired antigen to thus fuse a sensitizing lymphocyte with a human myeloma cell, for example, U266, thus giving a desired human antibody having activity in binding to an antigen (ref. JP, B, 1-59878). Moreover, by immunizing a transgenic animal having the full human antibody gene repertoire with a desired antigen, a desired human antibody may be obtained (ref. WO93/12227, WO92/03918, WO94/02602, WO96/34096, WO96/33735).

Immunization of an animal may be carried out by, for example, appropriately diluting a sensitizing antigen with phosphate buffered physiological saline (PBS), physiological saline, etc., suspending, mixing with an adjuvant as necessary so as to emulsify it, and then injecting an animal therewith intraperitoneally or subcutaneously. Following this, a sensitizing antigen mixed with Freund's incomplete adjuvant is preferably administered every 4 to 21 days several times. Confirmation of production of an antibody may be carried out by measuring the titer of a target antibody in serum of the animal by a commonly used method.

A hybridoma may be prepared by fusing an antibody-producing cell, which has been obtained from an animal or lymphocyte immunized with a desired antigen, with a myeloma cell using a commonly used fusing agent (e.g. polyethylene glycol) (Goding, Monoclonal Antibodies: Principles and Practice, Academic Press, 1986, 59-103). Hybridoma cells are cultured and grown as necessary, and binding specificity of an antibody produced by the hybridoma is measured by a known analytical method such as immunoprecipitation, radioimmunoassay (RIA), or enzyme-linked immunosorbent assay (ELISA). Following this, a hybridoma that produces an antibody for which a target specificity, affinity, or activity has been measured may be subcloned as necessary by a method such as a limiting dilution method.

Subsequently, a gene coding for the antibody thus selected may be cloned from a hybridoma or antibody-producing cell (sensitizing lymphocyte, etc.) using a probe that can specifically bind to the antibody (e.g. an oligonucleotide that is complementary to a sequence coding for an antibody constant region, etc.). Moreover, it may be cloned from mRNA by means of RT-PCR.

An antibody may be obtained by expressing the antibody gene thus constructed by a known method. In the case of mammalian cells, the antibody gene may be expressed by means of an expression vector containing a normally used useful promoter/enhancer, the antibody gene to be expressed and, downstream of the 3' side, DNA having a polyA signal functionally binding thereto. For example, examples of the promoter/enhancer include human cytomegalovirus immediate early promoter/enhancer. Other than the above, a promoter/enhancer of a virus such as retrovirus, poliomavirus, adenovirus, or simian virus 40 (SV40) or a promoter/enhancer derived from mammalian cells such as human elongation factor-1α may be used. For example, when SV40 promoter/enhancer is used, an antibody gene can be easily expressed in accordance with a method by Mulling et al., (Mulling RC et al., Nature (1979) 277: 108-14). When human elongation factor-1α is used, an antibody gene can be easily expressed in accordance with a method by Mizushima (Mizushima, Nucleic Acids Res (1990) 18: 5322). In the case of E. coli, an antibody gene may be expressed by means of an expression vector containing a generally used useful promoter, a signal sequence for secreting an antibody, and DNA to which the antibody gene to be expressed functionally binds. For example, examples of the promoter include LacZ promoter and araB promoter.

An antibody obtained by culturing and growing of a hybridoma or by gene recombination may be purified until it becomes homogeneous. Separation and purification of an antibody may be carried out by separation and purification methods used for normal proteins. For example, separation and purification of an antibody may be carried out by appropriately selecting and combining a chromatography column such as an affinity chromatography column, filtration, ultrafiltration, salting out using ammonium sulfate or sodium sulfate, dialysis, SDS polyacrylamide gel electrophoresis, isoelectric focusing, etc. (Antibodies: A Laboratory Manual. Ed Harlow and David Lane, Cold Spring Harbor Laboratory, 1988), but should not be construed as being limited thereto. Examples of a column used in affinity chromatography include a protein A column, a protein G column, and a protein L column. Whether or not an antibody thus obtained has an effector function may be determined by a method known to a person skilled in the art, for example, it can be determined by a method described in Examples.

As a more specific example of acquiring the polypeptide of the present invention, a method for producing a humanized antibody is explained below, and other polypeptides may be obtained in the same manner by this method.

### (1) Construction of humanized antibody expression vector

A humanized antibody expression vector is an animal cell expression vector into which has been incorporated a gene coding for a light chain (L chain) C region of a human antibody and a heavy chain (H chain) C region of a human antibody in which an amino acid residue selected from the group consisting of, as Kabat EU index numbers, the 293rd amino acid, 294th amino acid, 296th amino acid, 297th amino acid, 298th amino acid, and 300th amino acid is replaced by cysteine, and may be constructed by cloning into an animal cell expression vector genes coding for an L chain C region of a human antibody and an H chain C region of a human antibody in which an amino acid residue selected from the group consisting of, as Kabat EU index numbers, the 293rd amino acid, 294th amino acid, 296th amino acid, 297th amino acid, 298th amino acid, and 300th amino acid is replaced by cysteine.

The C region of a human antibody may be a C region of an H chain and an L chain of any human antibody, and examples thereof include a C region (hereinafter referred to as hCγ1) of the IgG1 subclass of an H chain of a human antibody and a C region (hereinafter referred to as hCκ) of the κ class of an L chain of a human antibody. As a gene coding for H chain and L chain C regions of a human antibody, chromosomal DNA consisting of exons and introns may be used, and cDNA may also be used.

As an animal cell expression vector, any animal cell expression vector may be used as long as a gene coding for a C region of a human antibody can be inserted and expressed. Examples thereof include pAGE107 (Cytotechnology, 3, 133 (1990)), pAGE103 (J. Biochem., 101, 1307 (1987)), pHSG274 (Gene, 27, 223 (1984)), pKCR (Proc. Natl. Acad. Sci. USA, 78, 1527 (1981)), and pSG1βd2-4 (Cytotechnology, 4, 173 (1990)). As a promoter and enhancer used in an animal cell expression vector, SV40 early promoter and enhancer (J. Biochem., 101, 1307 (1987)), Moloney mouse leukemia virus LTR (Biochem. Biophys. Res. Commun., 149, 960 (1987)), immunoglobulin H chain promoter (Cell, 41, 479 (1985)) and enhancer (Cell, 33, 717 (1983)), etc. can be cited.

With regard to the humanized antibody expression vector, either a type in which an H chain and an L chain of an antibody are present in separate vectors or a type in which they are present in the same vector (hereinafter referred to as a tandem type) may be used, but from the viewpoint of ease of construction of a humanized antibody expression vector, ease of introduction thereof into an animal cell, balance between the amounts of antibody H chain and L chain expressed within the animal cell, etc. a tandem type humanized antibody expression vector is preferable (J. Immunol. Methods, 167, 271 (1994)). Examples of the tandem type humanized antibody expression vector include pKANTEX93 (Mol. Immunol., 37, 1035 (2000)) and pEE18 (Hybridoma, 17, 559 (1998)).

The humanized antibody expression vector thus constructed may be used for expression of a human type chimera antibody and a human type CDR-grafted antibody in an animal cell.

### (2) Acquiring cDNA coding for V region of nonhuman animal antibody

cDNA coding for H chain and L chain V regions of a nonhuman animal antibody, for example a mouse antibody, may be obtained as follows.

mRNA is extracted from a hybridoma cell producing a target mouse antibody, and cDNA is synthesized. The cDNA thus synthesized is cloned into a vector such as a phage or a plasmid to thus prepare a cDNA library. A recombinant phage or recombinant plasmid having cDNA coding for H chain V region and a recombinant phage or recombinant plasmid having cDNA coding for L chain V region are isolated from the above library using as a probe a C region portion or a V region portion of an existing mouse antibody. The entire base sequences of H chain and L chain V regions of a target mouse antibody on the recombinant phage or recombinant plasmid are determined, and the entire amino acid sequences of the H chain and L chain V regions are inferred from the base sequences.

As a nonhuman animal, any nonhuman animal such as mouse, rat, hamster, or rabbit may be used as long as a hybridoma cell can be prepared.

As a method for preparing total RNA from a hybridoma cell, a guanidine thiocyanate-cesium trifluoroacetate method (Methods in Enzymol., 154, 3 (1987)) can be cited, and as a method for preparing mRNA from total RNA, an oligo (dT)-immobilized cellulose column method (Molecular Cloning: A Laboratory Manual, Cold Spring Harbor Lab. Press New York, 1989), etc. can be cited. Furthermore, as a kit for preparing mRNA from a hybridoma cell, a Fast Track mRNA Isolation Kit (Invitrogen), a Quick Prep mRNA Purification Kit (Pharmacia), etc. can be cited. As a cDNA synthesis and cDNA library preparation method, a standard method (Molecular Cloning: A Laboratory Manual, Cold Spring Harbor Lab. Press New York, 1989; Current Protocols in Molecular Biology, Supplement 1-34), a method using a commercial kit, for example, a Super Script™ Plasmid System for cDNA Synthesis and Plasmid Cloning (GIBCO BRL) or a ZAP-cDNA Synthesis Kit (Stratagene), etc. can be cited.

With regard to a vector into which cDNA that has been synthesized using mRNA extracted from a hybridoma cell as a template is inserted when preparing a cDNA library, any vector may be used as long as the cDNA can be inserted. For example, ZAP Express (Strategies, 5, 58 (1992)), pBluescript II SK(+) (Nucleic Acids Research, 17, 9494 (1989)), λzap II (Stratagene), λgt10, λgt11 (DNA Cloning: A Practical Approach, I, 49 (1985)), Lambda BlueMid (Clontech), λExCell, pT7T3 18U (Pharmacia), pcD2 (Mol. Cell. Biol., 3, 280 (1983)), pUC18 (Gene, 33, 103 (1985)), etc. may be used.

With regard to E. coli into which a cDNA library constructed by a phage or plasmid vector is introduced, any E. coli may be used as long as the cDNA library can be introduced, expressed, and maintained. For example, XL1-Blue MRF' (Strategies, 5, 81 (1992)), C600 (Genetics, 39, 440 (1954)), Y1088, Y1090 (Science, 222, 778 (1983)), NM522 (J. Mol. Biol, 166, 1 (1983)), K802 (J. Mol. Biol., 16, 118 (1966)), JM105 (Gene, 38, 275 (1985)), etc. may be used.

As a method for selecting a cDNA clone coding for H chain and L chain V regions of a nonhuman animal antibody from the cDNA library, selection may be carried out by a colony hybridization method or plaque hybridization method using an isotope- or fluorescently-labeled probe (Molecular Cloning: A Laboratory Manual, Cold Spring Harbor Lab. Press New York, 1989). Furthermore, cDNA coding for H chain and L chain V regions may be prepared by Polymerase Chain Reaction (hereinafter referred to as a PCR method; Molecular Cloning: A Laboratory Manual, Cold Spring Harbor Lab. Press New York, 1989; Current Protocols in Molecular Biology, Supplement 1-34) by preparing a primer and using as a template a cDNA library or cDNA synthesized from mRNA.

cDNA selected by the method above is cleaved by an appropriate restriction enzyme, etc., cloned into a plasmid such as pBluescript SK(-) (Stratagene), subjected to a normally used base sequence analysis method, for example, a reaction such as a dideoxy method by Sanger (Sanger) et al. (Proc. Natl. Acad. Sci. USA, 74, 5463 (1977)), etc., and analyzed using an automatic base sequence analyzer, for example, A.L.F.DNA sequencer (Pharmacia), thus determining the base sequence of the cDNA.

By inferring the total amino acid sequences of the H chain and L chain V regions from the base sequences thus determined, and comparing them with the whole amino acid sequences of H chain and L chain V regions of a known antibody (Sequences of Proteins of Immunological Interest, US Dept. Health and Human Services, 1991), it can be confirmed whether or not the obtained cDNA codes for the complete amino acid sequence, including a secretion signal sequence, of H chain and L chain V regions of the antibody.

Furthermore, when the amino acid sequence of an antibody variable region or the base sequence of DNA coding for the variable region is already known, production is carried out using the methods below.
When the amino acid sequence is known, the amino acid sequence is converted into a DNA sequence while taking into consideration codon usage (Sequences of Proteins of Immunological Interest, US Dept. Health and Human Services, 1991), several synthetic DNAs having a length of approximately 100 bases are synthesized based on the DNA sequence thus designed, and DNA can be obtained by carrying out a PCR method using them. When the base sequence is known, several synthetic DNAs having a length of approximately 100 bases are synthesized based on the information, and DNA can be obtained by carrying out a PCR method using them.

### (3) Analysis of amino acid sequence of V region of nonhuman animal antibody

With regard to a complete amino acid sequence, containing a secretion signal sequence, of H chain and L chain V regions of an antibody, by comparing it with the whole amino acid sequence of H chain and L chain V regions of a known antibody (Sequences of Proteins of Immunological Interest, US Dept. Health and Human Services, 1991), the length and N-terminal amino acid sequence of the secretion signal sequence can be inferred, and the subgroup to which it belongs can be learned. Furthermore, the amino acid sequence of a CDR of each of H chain and L chain V regions can also be learned by comparing with the amino acid sequences of H chain and L chain V regions of a known antibody (Sequences of Proteins of Immunological Interest, US Dept. Health and Human Services, 1991).

### (4) Construction of human type chimera antibody expression vector

cDNA coding for H chain and L chain V regions of an antibody of a nonhuman animal may be cloned upstream of a gene coding for H chain and L chain C regions of a human antibody of the humanized antibody expression vector described in (1), thus constructing a human type chimera antibody expression vector. For example, cDNAs coding for H chain and L chain V regions of an antibody of a nonhuman animal are linked to a synthetic DNA that is formed from a 3' terminal side base sequence of nonhuman animal antibody H chain and L chain V regions and a 5' terminal side base sequence of H chain and L chain C regions of a human antibody, and has appropriate restriction enzyme recognition sequences at opposite ends, and they are cloned upstream of genes coding for H chain and L chain C regions of a human antibody of the humanized antibody expression vector described in (1) so that they are expressed in appropriate forms, thus constructing a human type chimera antibody expression vector.

### (5) Construction of cDNA coding for human type CDR-grafted antibody V region

cDNAs coding for human type CDR-grafted antibody H chain and L chain V regions may be constructed as follows. First, the amino acid sequences of framework (hereinafter referred to as FR) of H chain and L chain V regions of a human antibody into which CDRs of H chain and L chain V regions of an antibody of a target nonhuman animal are to be grafted are selected. With regard to the FR amino acid sequences of H chain and L chain V regions of a human antibody, any amino acid sequence may be used as long as it is derived from a human antibody. For example, FR amino acid sequences of H chain and L chain V regions of a human antibody registered in a database such as the Protein Data Bank, an amino acid sequence that is common in subgroups of the FR of H chain and L chain V regions of a human antibody (Sequences of Proteins of Immunological Interest, US Dept. Health and Human Services, 1991), etc. can be cited, and among them, in order to prepare a human type CDR-grafted antibody having sufficient activity, it is desirable to select an amino acid sequence having a high homology (at least 60%) to an FR amino acid sequence of H chain and L chain V regions of an antibody of a target nonhuman animal.

Subsequently, the amino acid sequences of the CDRs of the H chain and L chain V regions of the antibody of the target nonhuman animal are grafted on the selected FR amino acid sequences of the H chain and L chain V regions of the human antibody, thus designing the amino acid sequences of human type CDR-grafted antibody H chain and L chain V regions. The amino acid sequences thus designed are converted into DNA sequences while taking into consideration codon usage (Sequences of Proteins of Immunological Interest, US Dept. Health and Human Services, 1991) seen in the base sequence of the antibody gene, and DNA sequences coding for the amino acid sequences of the human type CDR-grafted antibody H chain and L chain V regions are designed. Several synthetic DNAs having a length of approximately 100 bases are synthesized based on the DNA sequences thus designed, and a PCR method is carried out using them. In this case, from the reaction efficiency of PCR and the length of DNA that can be synthesized it is preferable to design 4 to 6 synthetic DNAs for both the H chain and the L chain.
Furthermore, by introducing a recognition sequence of an appropriate restriction enzyme into the 5' terminals of the synthetic DNA positioned at opposite ends, cloning into the humanized antibody expression vector constructed in (1) can easily be carried out. After PCR, an amplification product is cloned into a plasmid such as pBluescript SK(-) (Stratagene), the base sequence is determined by a method described in (2), and a plasmid having a DNA sequence coding for the amino acid sequences of desired human type CDR-grafted antibody H chain and L chain V regions is obtained.

### (6) Construction of human type CDR-grafted antibody expression vector

The cDNA coding for human type CDR-grafted antibody H chain and L chain V regions constructed in (5) may be cloned upstream of genes coding for human antibody H chain and L chain C regions of the humanized antibody expression vector described in (1), thus constructing a human type CDR-grafted antibody expression vector. For example, among synthetic DNAs that are used when constructing the human type CDR-grafted antibody H chain and L chain V regions in (5), by introducing a recognition sequence of an appropriate restriction enzyme into the 5' terminals of synthetic DNAs positioned at opposite ends, cloning is carried out upstream of a gene coding for human antibody H chain and L chain C regions of the humanized antibody expression vector described in (1) so that their expression is carried out in an appropriate form, thus constructing a human type CDR-grafted antibody expression vector.

### (7) Stable production of humanized antibody

By introducing the humanized antibody expression vector described in (4) and (6) into an appropriate animal cell, a transformant that stably produces a human type chimera antibody and a human type CDR-grafted antibody (hereinafter, collectively called a humanized antibody) may be obtained. As a method for introducing a humanized antibody expression vector into an animal cell, an electroporation method (JP, A, 2-257891; Cytotechnology, 3, 133 (1990)), etc. can be cited. As an animal cell into which a humanized antibody expression vector is to be introduced, any animal cell may be used as long as it can be made to produce a humanized antibody.

Specific examples thereof include mouse myeloma cell NSO cells, SP2/0 cells, Chinese hamster ovary cell CHO/dhfr-cells, CHO/DG44 cells, rat myeloma YB2/0 cells, IR983F cells, Syrian hamster kidney-derived BHK cells, and human myeloma cell Namalwa cells, and preferred examples thereof include Chinese hamster ovary cell CHO/DG44 cells and rat myeloma YB2/0 cells.

A transformant that stably produces a humanized antibody after introduction of a humanized antibody expression vector may be selected by means of an animal cell culture medium containing an agent such as G418 sulfate
(hereinafter referred to as G418; SIGMA) in accordance with a method disclosed in JP, A, 2-257891. As an animal cell culture medium, RPMI1640 medium (Nissui Pharmaceutical Co.), GIT medium (Nihon Pharmaceutical Co., Ltd.), EX-CELL302 medium (JRH), IMDM medium (GIBCO BRL), Hybridoma-SFM medium (GIBCO BRL), or a medium obtained by adding various types of additives such as fetal calf serum (hereinafter referred to as FCS) to the above media may be used. By culturing the transformant obtained in a medium, a humanized antibody may be produced and accumulated in the culture supernatant. The amount produced and the antigen binding activity of a humanized antibody in the culture supernatant may be measured by an enzyme immunoassay (hereinafter referred to as an ELISA method; Antibodies: A Laboratory Manual, Cold Spring Harbor Laboratory, Chapter 14, 1998, Monoclonal Antibodies: Principles and Practice, Academic Press Limited, 1996), etc. Furthermore, with regard to the transformant, the amount of humanized antibody produced may be increased by utilizing a DHFR gene amplification system, etc. in accordance with a method disclosed in JP, A, 2-257891.

The humanized antibody may be purified from the culture supernatant of the transformant using a protein A column (Antibodies: A Laboratory Manual, Cold Spring Harbor Laboratory, Chapter 8, 1988, Monoclonal Antibodies: Principles and Practice, Academic Press Limited, 1996). Furthermore, other than the above, a purification method that is usually used for purification of a protein may be used. For example, purification may be carried out by combining gel filtration, ion exchange chromatography, ultrafiltration, etc. The molecular weight of the H chain or L chain of a purified humanized antibody or of an entire antibody molecule may be measured by polyacrylamide gel electrophoresis (hereinafter referred to as SDS-PAGE; Nature, 227, 680 (1970)), a western blotting method (Antibodies: A Laboratory Manual, Cold Spring Harbor Laboratory, Chapter 12, 1988, Monoclonal Antibodies: Principles and Practice, Academic Press Limited, 1996), etc.

A method for producing a polypeptide using an animal cell as a host is explained above and, as described above, a polypeptide may be produced in a yeast, insect cells, plant cells, an animal individual, or a plant individual by the same method as for an animal cell.

When a host cell already has the ability to express a polypeptide, after preparing a cell that expresses a polypeptide using a known method in the present invention, the cell is cultured, and the target polypeptide is purified from the culture, thus producing the polypeptide of the present invention.

Furthermore, the present invention provides a method for producing a chimera molecule mutant having an improved effector function, the method including a step of subjecting an amino acid residue in the Fc region of a chimera molecule containing a cell binding portion and an Fc region and having an effector function to substitution with a cysteine residue, the amino acid residue being selected from the group consisting of, as Kabat EU index numbers, the 293rd amino acid, 294th amino acid, 296th amino acid, 297th amino acid, 298th amino acid, and 300th amino acid. More specifically, the present invention provides a method for producing a chimera molecule mutant having an improved effector function, the method including the steps below.
(1) A step of subjecting an amino acid residue in the Fc region of a chimera molecule containing a cell binding portion and an Fc region and having an effector function to substitution with a cysteine residue, the amino acid residue being selected from the group consisting of, as Kabat EU index numbers, the 293rd amino acid, 294th amino acid, 296th amino acid, 297th amino acid, 298th amino acid, and 300th amino acid,
(2) a step of introducing DNA coding for a chimera molecule mutant having a cell binding portion and an Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue into a host cell or a host organism, and expressing the DNA, and
(3) a step of recovering an expression product.

The chimera molecule used in the method for producing a chimera molecule mutant of the present invention may contain a peptide region between the cell binding portion and the Fc region as long as it has an effector function. As the peptide region, (1) to (3) below can be cited as examples, but the region should not be construed as being limited thereto. (1) A linker, (2) an antibody hinge region, and (3) a linker and an antibody hinge region. The 'antibody hinge region' includes not only a full length antibody hinge region, but also part of an antibody hinge region as long as it contributes to the effector function (or does not inhibit the effector function).

In the method for producing a chimera molecule mutant of the present invention, first an amino acid residue in the Fc region of a chimera molecule containing a cell binding portion and an Fc region and having an effector function is subjected to substitution with a cysteine residue, the amino acid residue being selected from the group consisting of, as Kabat EU index numbers, the 293rd amino acid, 294th amino acid, 296th amino acid, 297th amino acid, 298th amino acid, and 300th amino acid. A method for subjecting the amino acid residue selected from the group consisting of, as Kabat EU index numbers, the 293rd amino acid, 294th amino acid, 296th amino acid, 297th amino acid, 298th amino acid, and 300th amino acid to substitution with a cysteine residue is not particularly limited and, for example, it may be carried out by the above-mentioned site-directed mutagenesis method, etc.

In the method for producing a chimera molecule mutant of the present invention, DNA coding for a chimera molecule mutant containing a cell binding portion and an Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue is subsequently introduced into a host cell or a host organism by utilizing a method known to a person skilled in the art, and the DNA is expressed. By utilizing a method known to a person skilled in the art, an expression product (chimera molecule mutant) may be recovered.

The present invention also provides a method for producing a chimera molecule mutant having an improved effector function, the method including the steps below.
(1) A step of producing a chimera molecule containing a cell binding portion and an Fc region,
(2) a step of determining whether or not the chimera molecule produced has an effector function,
(3) a step of subjecting an amino acid residue in the Fc region of a chimera molecule that has been determined to have an effector function to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid,
(4) a step of introducing DNA coding for a chimera molecule mutant containing a cell binding portion and an Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue into a host cell or a host organism, and expressing the DNA, and
(5) a step of recovering an expression product.
   In this method, first, a chimera molecule containing a cell binding portion and an Fc region is produced. The chimera molecule produced has at least a cell binding portion and an Fc region; examples thereof include, but are not limited to, a chimera molecule having a peptide region between a cell binding portion and an Fc region. Production of the chimera molecule can be carried out as follows.
   Firstly, DNA coding for the cell binding portion and DNA coding for the Fc region are cloned by a method known to a person skilled in the art. Furthermore, as necessary, DNA coding for a peptide other than the above is cloned. The origin of the Fc region is not particularly limited; it may be derived from an antibody having an effector function or may be derived from an antibody that does not have an effector function.
   Subsequently, DNA coding for the cell binding portion and DNA coding for the Fc region are linked by a method known to a person skilled in the art (DNA coding for the cell binding portion, DNA coding for the Fc region, and DNA coding for the peptide other than the above are linked as necessary), thus preparing DNA coding for a chimera molecule containing a cell binding portion and an Fc region.
   Subsequently, the DNA coding for the chimera molecule containing the cell binding portion and the Fc region is introduced into a host cell or a host organism by a method known to a person skilled in the art, thus expressing the DNA. By utilizing a method known to a person skilled in the art, an expression product (a chimera molecule) may be recovered.

In this method, it is then determined whether or not the chimera molecule produced above has an effector function. Determination of whether or not the chimera molecule has an effector function may be carried out by a method known to a person skilled in the art, and it may be determined by for example a method described in Examples.

In this method, an amino acid residue in the Fc region of the chimera molecule that has been determined to have an effector function is then replaced by a cysteine residue, the amino acid residue being selected from the group consisting of, as Kabat EU index numbers, the 293rd amino acid, 294th amino acid, 296th amino acid, 297th amino acid, 298th amino acid, and 300th amino acid. A method for subjecting the amino acid residue selected from the group consisting of, as Kabat EU index numbers, the 293rd amino acid, 294th amino acid, 296th amino acid, 297th amino acid, 298th amino acid, and 300th amino acid to substitution with a cysteine residue is not particularly limited and, for example, it may be carried out by the above-mentioned site-directed mutagenesis method, etc.
In the method for producing a chimera molecule mutant of the present invention, the DNA coding for the chimera molecule mutant containing the cell binding portion and the Fc region in which an amino acid residue selected from the group consisting of, as Kabat EU index numbers, the 293rd amino acid, 294th amino acid, 296th amino acid, 297th amino acid, 298th amino acid, and 300th amino acid is replaced by a cysteine residue is subsequently introduced into a host cell or a host organism by utilizing a method known to a person skilled in the art, and the DNA is expressed. By utilizing a method known to a person skilled in the art, an expression product (a chimera molecule mutant) may be recovered.

As methods for measuring the amount of protein of a purified polypeptide, the activity of binding to an antigen or an FcR, or an effector function, known methods described in Monoclonal Antibodies: Principles and Practice, Third Edition, Acad. Press, 1993, Antibodies, A Laboratory manual, Cold Spring Harbor Laboratory, 1988, etc. may be used. As a specific example, when a polypeptide is a humanized antibody, the activity of binding to an antigen or an FcR and the activity of binding to an antigen-positive cultured cell line or an FcR-expressing cell may be measured by an ELISA method, a fluorescent antibody method (Cancer Immunol. Immunother., 36, 373 (1993)), a flow cytometry method, etc. Cytotoxicity of an antigen-positive cultured cell line may be evaluated by measuring CDC activity, ADCC activity, etc. (Cancer Immunol. Immunother., 36, 373 (1993)). Furthermore, the safety of a polypeptide in humans and the therapeutic effect may be evaluated using an appropriate model of an animal species that is relatively close to humans such as Macaca fascicularis.

The polypeptide of the present invention can give an effect of improving ADCC activity defined by the above-mentioned method compared with a polypeptide that has not been subjected to cysteine substitution. Because of this, when it is used for antibody therapy, the amount of drug used can be reduced, it is economical, and side effects can be suppressed.

Since an effector function can be greatly improved as hereinbefore described, the polypeptide of the present invention can be used in various applications such as treatment, diagnosis, and evaluation.

The pharmaceutical composition of the present invention contains the above-mentioned polypeptide of the present invention. Because of this, ADCC activity is improved, and a sufficient pharmacological effect can be obtained by the administration of a small amount, and it is very advantageous in excellent antibody therapy applications. Moreover, in another embodiment, the pharmaceutical composition of the present invention contains a nucleic acid coding for the polypeptide of the present invention or an Fc receptor binding portion thereof, a vector containing the nucleic acid, and/or a host cell or host organism having the vector. Such a composition may be used in, for example, a gene therapy in which an Fc receptor binding portion of any antibody or a specific antibody present in a living individual is modified and its effector function is improved. In yet another embodiment, the pharmaceutical composition of the present invention contains a polypeptide-producing cell that has been transformed by a vector containing a nucleic acid coding for the polypeptide of the present invention, or an antibody-producing cell in which a gene coding for an Fc region has been modified so that an amino acid selected from the group consisting of, as Kabat EU index numbers, the 293rd amino acid, 294th amino acid, 296th amino acid, 297th amino acid, 298th amino acid, and 300th amino acid in the Fc region is a cysteine residue. Since such a composition can continuously release, for example, an antibody having a high effector function within a body, it can be used in various cell therapies for the purpose of treatment of a tumor or enhancing immune strength. The above-mentioned pharmaceutical composition may contain, other than the above-mentioned components, for example, a nucleic acid, an amino acid, a peptide, a protein, another formulation additive such as an organic compound, an inorganic compound, or an excipient, and a combination thereof. The pharmaceutical composition of the present invention may be formulated using shape forming means as necessary and administered orally or parenterally. The pharmaceutical composition of the present invention may be particularly suitably used in a method such as parenteral administration by means of an injection, a drip, a percutaneous absorption agent, etc.

More specifically, the present invention provides a pharmaceutical composition that contains the polypeptide of the present invention, a nucleic acid coding for the polypeptide or an Fc receptor binding portion thereof, a vector containing the nucleic acid, and/or a host cell or host organism having the vector, and a pharmaceutically acceptable carrier. The present invention also provides a method for treating a mammal, the method including administering the polypeptide of the present invention, a nucleic acid coding for the polypeptide or an Fc receptor binding portion thereof, a vector containing the nucleic acid, and/or a host cell or host organism having the vector. Examples of the mammal include human and nonhuman mammals (e.g. mouse, rat, monkey, etc.).

The pharmaceutical composition of the present invention may be formulated by a known method by incorporating a pharmaceutically acceptable carrier in addition to the antibody. For example, it may be used parenterally in the form of an injection of a sterile solution or a suspension in water or a pharmaceutically acceptable liquid other than water. For example, formulation can be expected to be carried out by appropriately combining with a pharmacologically acceptable carrier or medium, specifically sterile water or physiological saline, a vegetable oil, an emulsifying agent, a suspending agent, a surfactant, a stabilizer, a flavoring agent, an excipient, a vehicle, a preservative, a binder, etc., and mixing in a configuration of a generally recognized unit dose required for carrying out formulation. The amount of active ingredient in these formulations is set so as to give an appropriate volume within a designated range.

A sterile composition for injection may be formulated in accordance with a standard formulation implementation using a vehicle such as distilled water for injection.
As an aqueous solution for injection, for example, physiological saline, an isotonic solution containing glucose or another adjuvant such as for example D-sorbitol, D-mannose, D-mannitol, or sodium chloride can be cited, and it may be used in a combination with an appropriate solubilizing agent such as for example an alcohol, specifically ethanol, a polyalcohol such as for example propylene glycol or polyethylene glycol, or a nonionic surfactant such as for example polysorbate 80™ or HCO-50.

As an oleaginous liquid sesame oil and soybean oil can be cited, and as a solubilizing agent benzyl benzoate or benzyl alcohol may be used in combination. Furthermore, it may be combined with a buffer such as a phosphate buffer or a sodium acetate buffer, a soothing agent such as procaine hydrochloride, a stabilizer such as benzyl alcohol or phenol, or an antioxidant. An injection thus prepared is usually filled into an appropriate ampoule.

Administration is preferably parenteral administration, and specific examples thereof include injection form, nasal administration form, pulmonary administration form, and percutaneous administration form. An injection form may be administered systemically or locally by for example intravenous injection, intramuscular injection, intraperitoneal injection, subcutaneous injection, etc.

Furthermore, the method of administration may be selected appropriately according to the age or symptoms of a patient. The amount of pharmaceutical composition containing the polypeptide of the present invention administered may be selected from, for example, a range of about 1 µg to 100 mg (e.g. about 1 µg to 15 mg, about 10 µg to 20 mg, about 0.1 mg to 20 mg) per kg of weight per administration. The pharmaceutical composition of the present invention may be administered to one site or a plurality of different sites. Furthermore, the pharmaceutical composition of the present invention may be administered continuously. Repetitive administration over several days may be continued according to conditions until disease conditions are desirably suppressed. The amount administered and the method of administration depend on the weight, age, symptoms, etc. of a patient, and a person skilled in the art may select it appropriately.

The pharmaceutical composition of the present invention may be used for the treatment of a wide range of diseases to which antibody therapy can be applied. As diseases/symptoms to which antibody therapy is applied, for example, metastatic breast cancer (anti-HER2 antibody), CD20-positive B cell non-Hodgkin lymphoma, thrombocytopenia, Sjogren syndrome, ankylosing spondylitis, sensory impairment, inflammatory bowel disease (IBD), scleroderma, rheumatoid arthritis, multiple sclerosis, lymphocytic leukemia, type 1 diabetes, cirrhosis of the liver, lymphoma, graft rejection, nephritis, multiple myeloma, vasculitis, autoimmune disease, blood cancer, various CD20-positive cell tumors (anti-CD20 antibody), rheumatoid arthritis, Crohn's disease (anti-TNFα antibody), acute rejection after kidney transplant (anti-CD3 antibody, anti-CD25 antibody), etc. can be cited. Antibodies used for treatment are shown in parentheses. By utilizing the pharmaceutical composition of the present invention instead of a conventional antibody for these diseases, etc., the effect of treatment can be greatly improved, thus alleviating the economic and physical burden on the patient. Furthermore, it may be suitably utilized as an antibody medicine that can be used in antibody therapy in the future. Moreover, the pharmaceutical composition of the present invention can be utilized for various immunotherapies and disease prevention in which an effector function of an antibody present within the body of a patient is enhanced and the immune strength of the patient is increased.

Furthermore, the present invention provides a method for improving an effector function of an antibody, the method including a step of subjecting an amino acid residue in an antibody having an effector function to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid.
Moreover, the present invention provides a method for improving an effector function of a chimera molecule, the method including a step of subjecting an amino acid in the Fc region of a chimera molecule containing a cell binding portion and an Fc region and having an effector function to substitution with a cysteine residue, the amino acid residue being selected from the group consisting of, as Kabat EU index numbers, the 293rd amino acid, 294th amino acid, 296th amino acid, 297th amino acid, 298th amino acid, and 300th amino acid.
In the above-mentioned method, substitution of the amino acid residue selected from the group consisting of, as Kabat EU index numbers, the 293rd amino acid, 294th amino acid, 296th amino acid, 297th amino acid, 298th amino acid, and 300th amino acid may be carried out as appropriate by the above-mentioned method.

Furthermore, the present invention provides a method for improving an effector function of a chimera molecule, the method including the steps below.
(1) A step of producing a chimera molecule containing a cell binding portion and an Fc region,
(2) a step of determining whether or not the chimera molecule produced has an effector function, and
(3) a step of subjecting an amino acid residue in the Fc region of a chimera molecule that has been determined to have an effector function to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid.
   In the above method, production of a chimera molecule, determination of whether or not the chimera molecule produced has an effector function, and substitution of the amino acid residue selected from the group consisting of, as Kabat EU index numbers, the 293rd amino acid, 294th amino acid, 296th amino acid, 297th amino acid, 298th amino acid, and 300th amino acid may be carried out appropriately by the above-mentioned methods.

Furthermore, the present invention relates to a method for producing an Fc region-containing polypeptide having a high effector function, the method including a step of subjecting an amino acid in the Fc region of an Fc region-containing polypeptide to substitution by a cysteine residue, the amino acid being selected from the group consisting of, as Kabat EU index numbers, the 293rd amino acid, 294th amino acid, 296th amino acid, 297th amino acid, 298th amino acid, and 300th amino acid, and to an Fc region-containing polypeptide produced by this method.
Moreover, the present invention relates to a method for enhancing an effector function of an antibody, the method including a step of subjecting an amino acid in an Fc region of an antibody to substitution by a cysteine residue, the amino acid being selected from the group consisting of, as Kabat EU index numbers, the 293rd amino acid, 294th amino acid, 296th amino acid, 297th amino acid, 298th amino acid, and 300th amino acid, and to an antibody obtained by this method.

In the present invention, the Fc region-containing polypeptide is not particularly limited as long as it is a polypeptide containing an Fc region of an antibody, and examples thereof include an antibody and a chimera molecule containing a cell binding portion and an Fc region. Substitution of a predetermined amino acid residue by a cysteine residue in the above-mentioned method may be carried out using any known method as described above. Specifically, it may be achieved by, but is not limited to, for example isolating a nucleic acid coding for an Fc region-containing polypeptide or an antibody, introducing a predetermined substitution sequence into the nucleic acid by a site-directed mutagenesis method, etc., introducing the nucleic acid thus obtained into a host cell, and carrying out expression. The above-mentioned method is typically carried out in vitro, but may also be carried out in vivo by subjecting a gene of an Fc region-containing polypeptide present within a living body, for example a gene of an antibody, to substitution within the living body with a mutant gene in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue. A method for subjecting in vivo a predetermined gene to substitution with a predetermined mutant gene is known to a person skilled in the art.

The present invention further relates to a method for preparing a high effector function antibody-producing cell, the method including a step of mutating a gene coding for an Fc region of an antibody-producing cell so that an amino acid in the Fc region is replaced by a cysteine residue, the amino acid being selected from the group consisting of, as Kabat EU index numbers, the 293rd amino acid, 294th amino acid, 296th amino acid, 297th amino acid, 298th amino acid, and 300th amino acid, and to an antibody-producing cell prepared by this method.

Here, the antibody-producing cell is not particularly limited as long as it is a cell having the ability to produce an antibody, and examples thereof include naturally occurring cells such as B cells or plasma cells, a hybridoma of such a naturally occurring cell and another cell such as a tumor cell, and any cell into which an antibody gene has been introduced in an expressible manner, preferably in a secretable manner. Mutagenesis of a gene coding for an Fc region of an antibody-producing cell may be carried out by any known method such as transfection with a vector containing a desired mutant gene. The above-mentioned method is typically carried out in vitro, but can also be carried out in vivo by substituting within a living body a gene of an antibody-producing cell present within the living body such as a B cell or a plasma cell with a mutant gene in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue. A method for subjecting in vivo a predetermined gene to substitution with a predetermined mutant gene is known to a person skilled in the art.

An antibody-producing cell prepared by the above-mentioned method can be used not only in production of an antibody but also in cell therapy involving transfer of the cell to a living body, etc. When it is used in such a therapy, the cell is preferably allogeneic to the animal to which it is to be transferred, and is more preferably an autologous cell collected from the individual to which it is to be transferred. An antibody-producing cell prepared by the above-mentioned method may be proliferated as necessary before transfer. Various techniques related to cell therapy such as cell culturing or cell administration methods are known to a person skilled in the art.
All prior art documents cited in the present description are incorporated into the present description by reference.

### Examples

The present invention is explained in detail below by reference to Examples. The present invention should not be construed as being limited to these Examples.

### Example 1 Preparation of H chain constant region amino acid mutants (Glu293Cys, Glu294Cys, Tyr296Cys, Asn297Cys, Ser298Cys, and Tyr300Cys mutants) of anti-CD20 chimera antibody

### 1-1. Cloning of gene coding for anti-CD20 chimera antibody (wild type)

### 1-1-1. Anti-CD20 mouse L chain variable region gene

mRNA was obtained from a hybridoma cell producing anti-CD20 mouse monoclonal antibody using a QuickPrep micro mRNA purification kit (Amersham Biosciences, product code 27-9255-01), and based thereon cDNA was prepared using a First-Strand cDNA Synthesis kit (Amersham Biosciences, code 27-9261-01). By a PCR reaction using this cDNA as a template with a combination of a sense primer selected from any one of MKV1 to 11 below and MKC antisense primer, an L chain variable region gene was amplified. The PCR reaction was carried out using a total of 50 µL of a reaction liquid containing cDNA 4 µL, 2.5 mM dNTPs 4 µL, sense primer (20 µM) 2.5 µL, antisense primer (20 µM) 2.5 µL, DMSO 2.5 µL, x10 pfu polymerase buffer 5 µL, pfu polymerase 1 µL, and sterile water 28.5 µL at 94°C 2 min; 94°C 1 min, 55°C 2 min, 72°C 2 min (30 cycles); and 72°C 4 min, and the amplification product was stored at 4°C.

The DNA sequences of the primers were as follows. MKV1 primer: ATGAAGTTGCCTGTTAGGCTGTTGGTGCTG (SEQ ID No: 95) MKV2 primer: ATGGAGWCAGACACACTCCTGYTATGGGTG (SEQ ID No: 96) MKV3 primer: ATGAGTGTGCTCACTCAGGTCCTGGSGTTG (SEQ ID No: 97) MKV4 primer: ATGAGGRCCCCTGCTCAGWTTYTTGGMWTCTTG (SEQ ID No: 98) MKV5 primer: ATGGATTTWCAGGTGCAGATTWTCAGCTTC (SEQ ID No: 99) MKV6 primer: ATGAGGTKCYYTGYTSAGYTYCTGRGG (SEQ ID No: 100) MKV7 primer: ATGGGCWTCAAGATGGAGTCACAKWYYCWGG (SEQ ID No: 101) MKV8 primer: ATGTGGGGAYCTKTTTYCMMTTTTTCAATTG (SEQ ID No: 102) MKV9 primer: ATGGTRTCCWCASCTCAGTTCCTTG (SEQ ID No: 103) MKV10 primer: ATGTATATATGTTTGTTGTCTATTTCT (SEQ ID No: 104) MKV11 primer: ATGGAAGCCCCAGCTCAGCTTCTCTTCC (SEQ ID No: 105) MKC primer: ACTGGATGGTGGGAAGATGG (SEQ ID No: 106)
(In the sequences above, M = A or C, R = A or G, W = A or T, S = C or G, Y = C or T, K = G or T.)
By using a combination of MKV5 primer and MKC primer above, an anti-CD20 mouse L chain variable region gene was amplified, and this gene was inserted into pCR2.1 vector (Invitrogen), thus giving pCR2.1-MLV.

### 1-1-2. Cloning of anti-CD20 mouse H chain variable region gene

An anti-CD20 mouse H chain variable region gene was amplified in the same manner as in 1-1-1. above except that any one of MHV1 to 12 primers below was used as a sense primer and MHCG2b primer below was used as an antisense primer.

### Sense primers

MHV1 primer: ATGAAATGCAGCTGGGGCATSTTCTTC (SEQ ID No: 107)
MHV2 primer: ATGGGATGGAGCTRTATCATSYTCTT (SEQ ID No: 108)
MHV3 primer: ATGAAGWTGTGGTTAAACTGGGTTTTT (SEQ ID No: 109)
MHV4 primer: ATGRACTTTGGGYTCAGCTTGRTTT (SEQ ID No: 110)
MHV5 primer: ATGGACTCCAGGCTCAATTTAGTTTTCCTT (SEQ ID No: 111)
MHV6 primer: ATGGCTGTCYTRGSGCTRCTCTTCTGC (SEQ ID No: 112)
MHV7 primer: ATGGRATGGAGCKGGRTCTTTMTCTT (SEQ ID No: 113)
MHV8 primer: ATGAGAGTGCTGATTCTTTTGTG (SEQ ID No: 114)
MHV9 primer: ATGGMTTGGGTGTGGAMCTTGCTATTCCTG (SEQ ID No: 115)
MHV10 primer: ATGGGCAGACTTACATTCTCATTCCTG (SEQ ID No: 116)
MHV11 primer: ATGGATTTTGGGCTGATTTTTTTTATTG (SEQ ID No: 117)
MHV12 primer: ATGATGGTGTTAAGTCTTCTGTACCTG (SEQ ID No: 118)
   Antisense primer
   MHCG2b primer: CAGTGGATAGACTGATGGGGG (SEQ ID No: 119)
   (In the sequences above, M, R, W, S, Y, and K have the same meanings as in 1-1-1. above.)
   By using a combination of MHV7 primer and MHCG2b primer above, an anti-CD20 mouse H chain variable region gene was amplified, and this gene was inserted into pCR2.1 vector (Invitrogen), thus giving pCR2.1-MHV.

### 1-1-3. Cloning of human IgG1 L chain constant region gene

Lymphocytes were isolated from human blood using Lymphoprep (Axis Shield). mRNA was obtained from these lymphocytes using a QuickPrep micro mRNA purification kit (Amersham Biosciences, code No. 27-9255-01), and based thereon cDNA was prepared using a First-Strand cDNA Synthesis kit (Amersham Biosciences, code No. 27-9261-01). A human IgG1 L chain constant region gene was amplified in the same manner as in 1-1-1. above except that this cDNA was used as a template and those below were used as primers. The gene thus obtained was inserted into a pCR2.1 vector (Invitrogen), thus giving pCR2.1-LC.

### Sense primer

hIgG1 LCF primer: ACTGTGGCTGCACCATCTGTCTTC (SEQ ID No: 120)
   Antisense primer
hIgG1 LCR primer: TTAACACTCTCCCCTGTTGAAGCTCTT (SEQ ID No: 121)

### 1-1-4. Human IgG1 H chain constant region gene

A human IgG1 H chain constant region gene was amplified in the same manner as in 1-1-3. above except that those below were used as primers. The gene thus obtained was inserted into pCR2.1 vector (Invitrogen), thus giving pCR2.1-HC (wild type).
Sense primer
hIgG1 HCF primer: GCCTCCACCAAGGGCCCATCGGTC (SEQ ID No: 122)
Antisense primer
hIgG1 HCR primer: TTATTTACCCGGAGACAGGGAGAGGCT (SEQ ID No: 123)

### 1-1-5. Construction of anti-CD20 chimera antibody L chain expression vector

An anti-CD20 chimera antibody L chain expression vector (p chimera LC) was prepared by incorporating an anti-CD20 mouse L chain variable region gene and a human IgG1 L chain constant region gene into an expression vector in tandem. That is, an anti-CD20 mouse L chain variable region gene and a human IgG1 L chain constant region gene were inserted into expression vector BCMGneo XhoI and NotI sites. A fragment obtained by carrying out the PCR reaction below using as a template the pCR2.1-MLV prepared in 1-1-1. above was used as the anti-CD20 mouse L chain variable region gene, and a fragment obtained by carrying out the PCR reaction below using as a template the pCR2.1-LC prepared in 1-1-3. above was used as the human IgG1 L chain constant region gene.

An anti-CD20 mouse L chain variable region gene fragment was obtained by a reaction using a total of 50 µL of a reaction liquid containing pCR2.1-MLV (20 ng/µL) 2 µL, 2.5 mM dNTPs 4 µL, sense primer (20 µM) 2.5 µL, antisense primer (20 µM) 2.5 µL, DMSO 2.5 µL, x10 pfu polymerase Buffer 5 µL, pfu polymerase 1 µL, and sterile water 30.5 µL at 94°C 2 min; 94°C 1 min, 55°C 2 min, 72°C 2 min (20 cycles) ; and 72°C 4 min, and was stored at 4°C.

### Sense primer

L1 primer: ACCGCTCGAGATGGATTTTCAGGTGCAGATTATCAGC (SEQ ID No: 124)

### Antisense primer

L2 primer: TTTCAGCTCCAGCTTGGTCCCAGCACC (5'-phosphorylated) (SEQ ID No: 125)
   Furthermore, a human IgG1 L chain constant region gene fragment was obtained in the same manner as for the anti-CD20 mouse L chain variable region gene except that pCR2.1-LC was used as a template and those below were used as primers.

### Sense primer

L3 primer: ACTGTGGCTGCACCATCTGTCTTCATC (5'-phosphorylated) (SEQ ID No: 126)

### Antisense primer

L4 primer: ATAGTTTAGCGGCCGCTTAACACTCTCCCCTGTTGAAGCTCTTTGT (SEQ ID No: 127)

The anti-CD20 mouse L chain variable region gene fragment and the human IgG1 L chain constant region gene fragment obtained by the above-mentioned PCR reactions were cleaved by means of the restriction enzymes XhoI (Takara) and NotI (Takara) respectively and then purified using a Wizard^{®} SV Gel and PCR Clean-up System (Promega). Subsequently, a fragment obtained by cleaving BCMGneo vector by means of XhoI and NotI and purifying using a Wizard^{®} SV Gel and PCR Clean-up System (Promega), and the above-mentioned purified anti-CD20 mouse L chain variable region gene fragment and purified human IgG1 L chain constant region gene fragment were mixed and ligated, thus giving an anti-CD20 chimera antibody L chain expression vector, p chimera LC. The vector thus obtained was sequenced, thus confirming that the target fragments were inserted. The sequences of the inserted L chain gene and the amino acid coded thereby are shown in SEQ ID Nos: 66 and 65 respectively.

### 1-1-6. Construction of anti-CD20 chimera antibody H chain expression vector

An anti-CD20 mouse H chain variable region gene and a human IgG1 H chain constant region gene were incorporated into an expression vector in tandem, thus preparing an anti-CD20 chimera antibody H chain expression vector (p chimera HC (wild type)). That is, an anti-CD20 mouse H chain variable region gene and a human IgG1 H chain constant region gene were inserted into expression vector BCMGneo XhoI and NotI sites. For the anti-CD20 mouse H chain variable region gene, a fragment obtained by carrying out the PCR reaction below using the pCR2.1-MHV prepared in 1-1-2. above as a template was used, and for the anti-CD20 mouse H chain constant region gene, a fragment obtained by carrying out the PCR reaction below using the pCR2.1-HC (wild type) prepared in 1-1-2. above as a template was used. The anti-CD20 mouse H chain variable region gene was obtained in the same manner as for the anti-CD20 mouse L chain variable region gene of 1-1-5. above except that pCR2.1-MHV was used as a template and those below were used as primers.

### Sense primer

H1 primer: ACCGCTCGAGATGGGATGGAGCTGGGTCTTTCTCTTC (SEQ ID No: 128)

### Antisense primer

H2 primer: TGAGGAGACGGTGACCGTGGTCCC (5'-phosphorylated) (SEQ ID No: 129)

Furthermore, the human IgG1 H chain constant region gene was obtained in the same manner as for the anti-CD20 mouse L chain variable region gene of 1-1-5. above except that pCR2.1-HC (wild type) was used as a template and those below were used as primers.

### Sense primer

H3 primer: GCCTCCACCAAGGGCCCATCGGTC (5'-phosphorylated) (SEQ ID No: 130)

### Antisense primer

H4 primer: ATAGTTTAGCGGCCGCTTATTTACCCGGAGACAGGGAGAGGCTCTT (SEQ ID No: 131)

The anti-CD20 mouse H chain variable region gene fragment and the human IgG1 H chain constant region gene fragment obtained by the above-mentioned PCR reactions were cleaved by means of the restriction enzymes XhoI (Takara) and NotI (Takara) respectively, and then purified using a Wizard (registered trademark) SV Gel and PCR Clean-up System (Promega). Subsequently, a fragment formed by cleaving BCMGneo vector by means of XhoI and NotI and purifying using a Wizard^{®} SV Gel and PCR Clean-up System (Promega), and the purified anti-CD20 mouse H chain variable region gene fragment and the purified human IgG1 H chain constant region gene fragment were mixed and ligated, thus giving an anti-CD20 chimera antibody H chain expression vector, p chimera HC (wild type). The vector thus obtained was sequenced, thereby confirming that the target fragments were inserted. The sequences of the wild type H chain gene thus inserted and the amino acids coded thereby are shown in SEQ ID Nos: 50 and 49 respectively.

### 1-2. Construction of anti-CD20 chimera antibody expression vector

An L chain cDNA and an H chain cDNA were separated from the p chimera LC and p chimera HC (wild type) obtained in 1-1. above using EcoRI and NotI restriction enzymes, and inserted into a pIRESneo2 vector and a pIRESpuro2 vector respectively by a standard method, thus giving an L chain expression vector, anti-CD20 mab L chain/pIRESneo2, and an H chain expression vector, anti-CD20 mab H chain/pIRESpuro2. The vectors thus obtained were sequenced using an ABI 3100-Avant (Applied Biosystems) in accordance with the instruction manual, and the DNA base sequences were confirmed.

### 1-3. Acquiring cDNA coding for H chain constant region amino acid mutant and construction of amino acid-modified H chain expression vector

For the purpose of acquiring an antibody in which 293Glu, 294Glu, 296Tyr, 297Asn, 298Ser, or 300Tyr of an H chain constant region was changed to Cys, site-directed mutagenesis was carried out using QuikChange (Stratagene) in accordance with the instruction manual with the p chimera HC (wild type) obtained in 1-1-6. as a template. For Glu293Cys mutagenesis, Glu293Cys sense primer (5'-GCCAAGACAAAGCCGCGGTGCGAGCAGTACAACAGCACGTACCGGGTGGTC-3', SEQ ID No: 132) and Glu293Cys antisense primer (5'-GACCACCCGGTACGTGCTGTTGTACTGCTCGCACCGCGGCTTTGTCTTGGC-3', SEQ ID No: 133) were used, thus giving p chimera Glu293Cys. For Glu294Cys mutagenesis, Glu294Cys sense primer (5'-GACAAAGCCGCGGGAGTGCCAGTACAACAGCACGTACCGGGTGGTC-3', SEQ ID No: 134) and Glu294Cys antisense primer (5'-GACCACCCGGTACGTGCTGTTGTACTGGCACTCCCGCGGCTTTGTC-3', SEQ ID No: 135) were used, thus giving p chimera Glu294Cys. For Tyr296Cys mutagenesis, Tyr296Cys sense primer (5'-AAGCCGCGGGAGGAGCAGTGCAACAGCACGTACCGGGT-3', SEQ ID No: 136) and Tyr296Cys antisense primer (5'-ACCCGGTACGTGCTGTTGCACTGCTCCTCCCGCGGCTT-3', SEQ ID No: 137) were used, thus giving p chimera Tyr296Cys. For Asn297Cys mutagenesis, Asn297Cys sense primer (5'-AGGAGCAGTACTGCAGCACGTACCGGGT-3', SEQ ID No: 138) and Asn297Cys antisense primer (5'-ACCCGGTACGTGCTGCAGTACTGCTCCT-3', SEQ ID No: 139) were used, thus giving p chimera Asn297Cys. For Ser298Cys mutagenesis, Ser298Cys sense primer (5'-GAGGAGCAGTACAACTGCACGTACCGGGTGG-3', SEQ ID No: 140) and Ser298Cys antisense primer (5'-CCACCCGGTACGTGCAGTTGTACTGCTCCTC-3', SEQ ID No: 141) were used, thus giving p chimera Ser298Cys. For Tyr300Cys mutagenesis, Tyr300Cys sense primer (5'-AGCAGTACAACAGCACGTGCCGGGTGGTCAGCGT-3', SEQ ID No: 142) and Tyr300Cys antisense primer (5'-ACGCTGACCACCCGGCACGTGCTGTTGTACTGCT-3', SEQ ID No: 143) were used, thus giving p chimera Tyr300Cys. Confirmation of the sequence of the inserted cDNA was carried out using an ABI 3100-Avant (Applied Biosystems) in accordance with the instruction manual.

Using EcoRI and NotI restriction enzymes H chain cDNAs were separated from the p chimera Glu293Cys, p chimera Glu294Cys, p chimera Tyr296Cys, p chimera Asn297Cys, p chimera Ser298Cys, and p chimera Tyr300Cys obtained by the above-mentioned site-directed mutagenesis, and were inserted into pIRESpuro2 vectors by a standard method, thus giving the amino acid-modified H chain expression vectors anti-CD20 mab H_Glu293Cys/pIRESpuro2, anti-CD20 mab H_Glu294Cys/pIRESpuro2, anti-CD20 mab H_Tyr296Cys/pIRESpuro2, anti-CD20 mab H_Asn297Cys/pIRESpuro2, anti-CD20 mab H_Ser298Cys/pIRESpuro2, and anti-CD20 mab H_Tyr300Cys/pIRESpuro2 respectively. The DNA base sequences of the vectors thus obtained were confirmed using an ABI 3100-Avant (Applied Biosystems) in accordance with the instruction manual. The SEQ ID Nos of the gene of each of the amino acid-modified antibody H chains and the amino acid coded thereby are shown in the Table below.

**Table 8**

| Mutant | Nucleic acid | Amino acid |
|---|---|---|
| Glu293Cys | SEQ ID No: 43 | SEQ ID No: 37 |
| Glu294Cys | SEQ ID No: 44 | SEQ ID No: 38 |
| Tyr296Cys | SEQ ID No: 45 | SEQ ID No: 39 |
| Asn297Cys | SEQ ID No: 46 | SEQ ID No: 40 |
| Ser298Cys | SEQ ID No: 47 | SEQ ID No: 41 |
| Tyr300Cys | SEQ ID No: 48 | SEQ ID No: 42 |

### 1-4. Preparation of anti-CD20 chimera antibody-producing cell line using CHO cells

The L chain expression vector anti-CD20 mab L chain/pIRESneo2, and the wild type H chain expression vector anti-CD20 mab H/pIRESpuro2 or the constant region amino acid-modified H chain expression vector anti-CD20 mab H_Glu293Cys/pIRESpuro2, anti-CD20 mab H_Glu294Cys/pIRESpuro2, anti-CD20 mab H_Tyr296Cys/pIRESpuro2, anti-CD20 mab H_Asn297Cys/pIRESpuro2, anti-CD20 mab H_Ser298Cys/pIRESpuro2, or anti-CD20 mab H_Tyr300Cys/pIRESpuro2, obtained in 1-2. and 1-3. above, were introduced into CHO-K1 cells (ATCC catalog No. CCL-61) using FuGENE^{®} (Roche) in accordance with the instruction manual. That is, transduced into CHO-K1 cells were anti-CD20 mab L chain/pIRESneo2 and anti-CD20 mab H/pIRESpuro2 when preparing wild type antibody-producing cells, anti-CD20 mab L chain/pIRESneo2 and anti-CD20 mab H_Glu293Cys/pIRESpuro2 when preparing Glu293Cys-modified antibody-producing cells, anti-CD20 mab L chain/pIRESneo2 and anti-CD20 mab H_Glu294Cys/pIRESpuro2 when preparing Glu294Cys-modified antibody-producing cells, anti-CD20 mab L chain/pIRESneo2 and anti-CD20 mab H_Tyr296Cys/pIRESpuro2 when preparing Tyr296Cys-modified antibody-producing cells, anti-CD20 mab L chain/pIRESneo2 and anti-CD20 mab H_Asn297Cys/pIRESpuro2 when preparing Asn297Cys-modified antibody-producing cells, anti-CD20 mab L chain/pIRESneo2 and anti-CD20 mab H_Ser298Cys/pIRESpuro2 when preparing Ser298Cys-modified antibody-producing cells, and anti-CD20 mab L chain/pIRESneo2 and anti-CD20 mab H_Tyr300Cys/pIRESpuro2 when preparing Tyr300Cys-modified antibody-producing cells, thus coexpressing the L chain and the H chain.

The gene-transferred CHO-K1 cells were cultured by means of RPMI1640 medium (SIGMA) containing 10% FCS (Hyclone) and penicillin-streptomycin (SIGMA), and drug selection was carried out by further adding 5 µg/mL Puromycin (SIGMA) and 0.6 mg/mL G418 (Wako Pure Chemical Industries, Ltd.). Cells for which drug resistance was confirmed were subjected to limiting dilution, and screened with respect to single cell clone using antibody production capability as an index. Screening of antibody-expressing cells was carried out by sandwich ELISA using anti-human Igγ chain antibody (SIGMA) and HRP-labeled anti-human IgFc antibody (Cappel), and an anti-CD20 chimera antibody-producing cell line with high production of each antibody was established.

### 1-4-1. Preparation of FUT8 knockdown CHO-K1 cells

In order to produce an antibody containing no fucose, fucosyl transferase 8 (FUT8)-knocked-down CHO-K1 cells were prepared. By introducing an antibody gene into these cells in accordance with 1-4. above, an antibody containing no fucose could be prepared.
Specifically, in accordance with a standard method, FUT8 siRNA sense primer (5'-gatccccgctgagtctctccgaatacttcaagagagtattcggagagactcagcttttta-3', SEQ ID No: 144) and FUT8 siRNA antisense primer (5'-tcgataaaaagctgagtctctccgaatactctcttgaagtattcggagagactcagcggg-3', SEQ ID No: 145) were heated in the presence of NaCl at 99°C for 2 min, and then annealed by cooling from 72°C to 4°C over 2 hours. The DNA fragment thus obtained was inserted into pSuper gfp+neo (OligoEngine). The base sequence of the inserted DNA was confirmed using an ABI 3100-Avant (Applied Biosystems), thus constructing the FUT8 siRNA expression vector FUT8 SiRNA/pSuper gfp+neo.

The FUT8 siRNA expression vector FUT8 siRNA/pSuper gfp+neo thus obtained was introduced into CHO-K1 cells (ATCC catalog No. CCL-61) using FuGENE^{®} (Roche) in accordance with the instruction manual. The gene-transferred CHO-K1 cells were cultured by means of RPMI1640 medium (SIGMA) containing 10% FCS (Hyclone) and penicillin-streptomycin (SIGMA), and drug selection was carried out by further adding 0.6 mg/mL G418 (Wako Pure Chemical Industries, Ltd.). Cells that had acquired drug resistance were subjected to sorting using a cell sorter (EPICS ALTRA, Beckman Coulter) with the amount of GFP expressed as an index. Cells thus sorted were subjected to limiting dilution, thus giving high GFP expression cells, that is, cells with high expression of FUT8 siRNA. The amount of FUT8 mRNA expressed in these cells was quantified using a real time PCR method employing an FUT8 expression analysis sense primer (5'-TGGTCTACTGCTTCATGATTGCA-3', SEQ ID No: 146), an FUT8 expression analysis antisense primer (5'-GCATAGCGCCAATTCTGAGAT-3', SEQ ID No: 147), and an ABI PRISM 7000 (Applied Biosystems), and the amount of mRNA expressed was found to be 22.8% of that of cells prior to gene transfer, thus confirming suppression of FUT8 expression. The cells having high siRNA expression thus obtained were used in experiments as FUT8 knockdown CHO-K1 cells.

### 1-5. Production and purification of antibody

The anti-CD20 chimera antibody-producing cell line obtained in 1-4. above was cultured in RPMI1640 medium containing 5 µg/mL Puromycin, 0.6 mg/mL G418, and 10% FCS, and proliferated up to 60% of the maximum cell density of the culture dish. Subsequently, washing was carried out twice with phosphate buffered physiological saline containing no Ca²⁺ or Mg²⁺ (PBS(-)) to thus remove the growth medium, it was substituted with 0.1% BSA-containing RPMI1640, culturing was carried out for 7 days, and the supernatant was recovered. After the supernatant was filtered with a 0.2 µm filter, it was adsorbed on a Protein L column (PIERCE) by a standard method and eluted with 0.1 M glycine buffer (pH 2.8), and the pH was made neutral using 0.75 M Tris-HCl (pH 9.0). Subsequently, dialysis was carried out against PBS(-) by a standard method, thus giving an antibody for evaluation.

### Example 2 AILIM/ICOS-IgFc and AILIM/ICOS-IgFc mutant 1. Preparation of AILIM/ICOS-IgFc chimera molecule expression vector

### 1-1. Construction of cDNA coding for AILIM/ICOS extracellular domain region

cDNA coding for an extracellular domain region of AILIM/ICOS was isolated from mRNA of activated T cells by a PCR method in accordance with a standard method using a primer designed based on known sequence information for
AILIM/ICOS.

Specifically, T cells were purified from the human peripheral blood mononuclear cells obtained in Example 1 1-1-3. using a PanT-Isolation Kit (Myltenyi) in accordance with the instruction manual. Purified T cells were seeded at 10⁵ cells/well on an ELISA plate that had been coated for 1 hour at 37°C with a solution formed by diluting anti-CD3 antibody (clone OKT3, Ortho Biotech) to a final concentration of 1 µg/mL and anti-CD28 antibody (clone 28.2, BD) to a final concentration of 5 µg/mL with PBS containing no Ca²⁺ or Mg²⁺ (PBS(-)), and cultured in a CO₂ incubator containing 5% CO₂ at 37°C for 24 hours, thus forming activated T cells. RNA was extracted from the activated T cells by means of TRIzol Reagent (Invitrogen), and cDNA was synthesized with 1 µg of this RNA as a template using ReverTra Ace-a (Toyobo Co., Ltd.) in accordance with the instruction manual. A PCR reaction was carried out for 35 cycles using 0.2 µL of the cDNA solution thus obtained and 50 pmole each of the sense primer (5'-tgttgctagcaaacatgaagtcaggcctc-3', NheI site sequence added to AILIM/ICOS sequence, SEQ ID No: 148) and the antisense primer (5'-aacggatccttcagctggcaacaaag-3', BamHI site sequence added to AILIM/ICOS sequence, SEQ ID No: 149) as synthetic oligonucleotides in a reaction system having a final capacity of 50 µL by adding 0.25 µL of Ex Taq polymerase (TaKaRa) and 5 µL of the Buffer included with the Ex Taq polymerase.

After the PCR reaction, an approximately 0.45 kbp cDNA fragment was recovered using 1% agarose gel electrophoresis in accordance with a standard method, and purified using a Wizard SV Gel and PCR Clean-up System (Promega) in accordance with the instruction manual. cDNA concentration was calculated from the absorbance at 260 nm (1 O.D.260 = 50 µg/mL). 1 µg of this fragment was cleaved at 37°C for 3 hours by means of 10 units each of NheI restriction enzyme and BamHI restriction enzyme in accordance with the instruction manual, and an approximately 0.45 kbp AILIM/ICOS extracellular region cDNA fragment having terminals cleaved by the restriction enzymes NheI and BamHI was obtained by the same method as above.

### 1-2. Construction of cDNA coding for human IgFc

On the other hand, cDNA coding for the Fc region (IgFc) of human immunoglobulin 1 was constructed using a PCR method by the procedures below from cDNA coding for the IgFc amino acid sequence described in GenBank accession No. J00228. Firstly, base sequences for the binding of primer for amplification during the PCR reaction (including BamHI restriction enzyme sequence for the 5' terminal and NotI restriction enzyme sequence for the 3' terminal of IgFc cDNA) were added to the 5' and 3' terminals of cDNA coding for the IgFc amino acid sequence of GenBank accession No. J00228. The base sequence thus designed was divided into a total of eight base sequences from the 5' terminal side with about 100 bases each (adjacent base sequences having an approximately 20 base overlap sequence at the terminals) and, oligonucleotides consisting of the sense strand and the antisense strand thereof in alternating order, the sense primer (5'-tgaaggatcccgaggagcccaaatcttgtgacaa-3', BamHI site sequence added to IgFc sequence, SEQ ID No: 150) and the antisense primer (5'-gaagcggccgctcatttacccggagacagggagaggctc-3', NotI site sequence added to IgFc sequence, SEQ ID No: 151) were synthesized. Each synthetic oligonucleotide was added to a PCR reaction liquid so as to give a final concentration of 0.1 µM, and a PCR reaction was carried out for 30 cycles in a reaction system with a final capacity of 50 µL by adding 0.25 µL of Ex Taq polymerase (TaKaRa) and 5 µL of the Buffer included with the Ex Taq polymerase.

After the PCR reaction, 1% agarose gel electrophoresis was carried out in accordance with a standard method, and an approximately 0.45 kbp cDNA fragment was recovered and purified using a Wizard SV Gel and PCR Clean-up System (Promega) in accordance with the instruction manual. cDNA concentration was calculated from the absorbance at 260 nm (1 O.D.260 = 50 µg/mL). 1 µg of this fragment was cleaved at 37°C for 3 hours by means of 10 units each of BamHI restriction enzyme and NotI restriction enzyme in accordance with the instruction manual, and a cDNA fragment with an approximately 0.76 kbp IgFc region having terminals cleaved by means of BamHI and NotI restriction enzymes was then obtained by the same method as above.

### 1-3. Construction of AILIM/ICOS-IgFc chimera molecule expression vector

In order to construct an expression vector having these cDNA fragments incorporated thereinto, 1 µg of the expression vector pIRES-puro2 (Clontech) was cleaved at 37°C for 3 hours by means of 10 units each of NheI restriction enzyme and NotI restriction enzyme in accordance with the instruction manual, and a pIRES-puro2 cDNA fragment having terminals cleaved by the NheI and NotI restriction enzymes was then obtained by the same method as above. This fragment, a cDNA fragment of an approximately 0.45 kbp AILIM/ICOS extracellular region having terminals cleaved by NheI and BamHI restriction enzymes, and a cDNA fragment of an approximately 0.76 kbp IgFc region having terminals cleaved by BamHI and NotI restriction enzymes were ligated using Ligation High (Toyobo Co., Ltd.) in accordance with the instruction manual, and then used for transforming E. coli DH5α for transformation (Toyobo Co., Ltd.). The colony thus obtained was liquid-cultured in NZY broth for 16 hours. A plasmid was purified from the transformed E. coli by means of a Wizard plus SV Minipreps DNA Purification Kit (Promega), thus giving a pIRESpuro2-AILIM/ICOS-IgFc vector expressing cDNA of a chimera molecule in which AILIM/ICOS extracellular region and IgFc were ligated. Following this, a reaction was carried out using an ABI 3100-Avant (Applied Biosystems) in accordance with the instruction manual, thus confirming the DNA sequence.

### 2. Preparation of AILIM/ICOS-IgFc mutant expression vector

A reaction was carried out by means of a QuikChange^{®} Site-Directed Mutagenesis Kit (Stratagene) in accordance with the instruction manual using pIRESpuro2-AILIM/ICOS-IgFc as a template and each of the primers described in Example 1 1-3. in order to mutate 221Glu, 222Glu, 224Tyr, 225Asn, 226Ser, or 228Tyr of the AILIM/ICOS-IgFc molecule (corresponding to IgG 293Glu, 294Glu, 296Tyr, 297Asn, 298Ser, or 300Tyr in the Kabat EU index) into Cys.

After mutagenesis, a colony obtained by transforming competent E. coli was liquid-cultured in NZY broth for 16 hours. A plasmid was purified from the transformed E. coli by means of a Wizard plus SV Minipreps DNA Purification Kit (Promega), and expression vectors pIRES-puro2 AILIM-IgFc Glu22lCys, pIRES-puro2 AILIM-IgFc Glu222Cys, pIRES-puro2 AILIM-IgFc Tyr224Cys, pIRES-puro2 AILIM-IgFc Asn225Cys, pIRES-puro2 AILIM-IgFc Ser226Cys or pIRES-puro2 AILIM-IgFc Tyr228Cys of mutants AILIM-IgFc Glu221Cys, AILIM-IgFc Glu222Cys, AILIM-IgFc Tyr224Cys, AILIM-IgFc Asn225Cys, AILIM-IgFc Ser226Cys, or AILIM-IgFc Tyr228Cys, having Glu221Cys, Glu222Cys, Tyr224Cys, Asn225Cys, Ser226Cys or Tyr228Cys mutations, were obtained. Following this, a reaction was carried out using an ABI 3100-Avant (Applied Biosystems) in accordance with the instruction manual, thus confirming the DNA sequence. The SEQ ID Nos of the base sequences coding for the mutants and the amino acid sequences formed by the base sequences are shown in the table below.

### [Table 9]

**Table 9**

| Mutant | Nucleic acid | Amino acid |
|---|---|---|
| AILIM-IgFc Glu221Cys | SEQ ID No: 81 | SEQ ID No: 75 |
| AILIM-IgFc Glu222Cys | SEQ ID No: 82 | SEQ ID No: 76 |
| AILIM-IgFc Tyr224Cys | SEQ ID No: 83 | SEQ ID No: 77 |
| AILIM-IgFc Asn225Cys | SEQ ID No: 84 | SEQ ID No: 78 |
| AILIM-IgFc Ser226Cys | SEQ ID No: 85 | SEQ ID No: 79 |
| AILIM-IgFc Tyr228Cys | SEQ ID No: 86 | SEQ ID No: 80 |

The 144^{th} to 375^{th} amino acids in the amino acid sequences described in SEQ ID Nos: 75 to 80 above correspond to the 216^{th} to 447^{th} amino acids as Kabat EU index number. The amino acid sequence of the wild type chimera molecule before a mutation is introduced is shown in SEQ ID No: 87, and the base sequence coding therefor is shown in SEQ ID No: 88.

### 3. Preparation of CHO-K1 cell expressing AILIM/ICOS-IgFc and AILIM/ICOS-IgFc mutant

1 x 10⁶ CHO-K1 cells were transduced using 36 µL of FuGENE^{®} and 12 µg of pIREpuro2-AILIM/ICOS-IgFc or each of the mutant expression vectors in accordance with the instruction manual. On the second day after transduction, Puromycin was added to 10% fetal calf serum (FCS)-containing RPMI1640 medium at a final concentration of 0.5 to 5 µg/mL, and culturing was then carried out for 9 days. After drug resistant CHO-K1 cells were selected by means of Puromycin, the cells were cloned by a limiting dilution method, and cells with high expression of AILIM/ICOS-IgFc or an AILIM/ICOS-IgFc mutant were screened and selected by ELISA using an anti-AILIM/ICOS antibody.

### 4. Production and purification of AILIM/ICOS-IgFc and AILIM/ICOS-IgFc mutant

Cells with high expression of AILIM/ICOS-IgFc or the AILIM/ICOS-IgFc mutants were cultured in 10% FCS-containing RPMI1640 medium and proliferated up to 60% of the maximum cell density of the culture dish. Washing was carried out twice with PBS containing no Ca²⁺ or Mg²⁺ (PBS(-)) to thus remove the growth medium, it was replaced with 0.1% BSA-containing RPMI1640, culturing was carried out for 3 days, and the supernatant was recovered. After the supernatant was filtered with a 0.2 µm filter, it was adsorbed on a Protein G column (Amersham Biosciences) by a standard method, and eluted with 0.1 M glycine buffer (pH 2.8), and the pH was made neutral using 0.75 M Tris-HCl (pH 9.0). Subsequently, dialysis was carried out using PBS(-) by a standard method. The concentration of AILIM/ICOS-IgFc or the AILIM/ICOS-IgFc mutants in these samples was measured by an ELISA method using an anti-AILIM/ICOS antibody.

### Example 3 Evaluation of anti-CD20 chimera antibody H chain constant region amino acid-modified antibody

With regard to the antibody obtained in Example 1, reactivity with an antigen (CD20 binding activity), reactivity with NK cell Fcγ receptor III (CD16 binding activity), and ADCC induction activity were measured.

### 1. Separation and preparation of human peripheral blood mononuclear cells

Human blood was collected in an anticoagulant (CPD)-containing blood collecting bag (Terumo Corporation, Terumo CPD blood bag). This blood was layered on 15 mL of Lymphoprep (AXIS-SHIELD) dispensed into a 50 mL centrifugal tube (Falcon) and centrifuged using a swing type cell separation centrifuge at room temperature and 1,600 rpm for 30 min in accordance with the instruction manual. After centrifugation, a mononuclear cell layer was recovered in accordance with the instruction manual, washed three times with PBS containing no calcium or magnesium and to which bovine serum albumin (BSA, Sigma) had been added at a final concentration of 0.5% (w/v), and then dispersed in RPMI1640 medium containing fetal calf serum (FCS) at a final concentration of 10% (10% FCS-containing RPMI1640), thus giving a cell suspension of human peripheral blood-derived mononuclear cells.

### 2. Analysis of CD20 binding reactivity

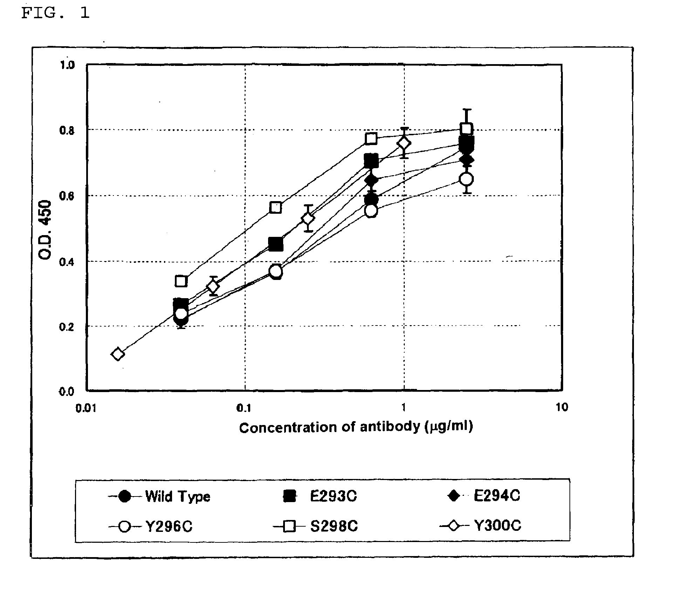

Daudi cells (ATCC catalog No. CCL-213) were dispensed into a 96 well plate at a density of 6 × 10⁴ cells/well, each of the antibody mutants (Glu293Cys, Glu294Cys, Tyr296Cys, Ser298Cys, or Tyr300Cys) obtained in Example 1 or wild type antibody was added thereto, and a reaction was carried out at 4°C for 1 hour. Following this, washing was carried out with 3 mM EDTA-0.5% BSA-PBS(-) (washing buffer). Subsequently, 50 µL/well of an HRP-labeled anti-human IgG antibody (Zymed) diluted 500 times with the washing buffer was added, and a reaction was carried out at 4°C for 1 hour. Cells after the reaction were washed with the washing buffer, 100 µL/well of TMB substrate (KPL) was added thereto so as to cause coloration, the reaction was stopped by adding 100 µL/well of 1 M HCl (Wako), and the OD value at 450 nm was measured using a plate reader (VersaMax, Molecular Devices). The results are shown in FIG. 1. From the figure, it is clear that all the mutants showed CD20 binding reactivity at the same level as that of the wild type, thus determining that amino acid substitution in the mutants does not affect antigen-recognizing capability.

### 3. Analysis of CD16 binding reactivity

### 3-1. Preparation of CD16 expressing CHO-K1 cells

In accordance with a standard method, RNA was extracted from human peripheral blood mononuclear cells using Trizol (Invitrogen), and cDNA synthesis was carried out using ReverTra Ace (TOYOBO). A CD16 sense primer (5'-TTTGAATTCatgtggcagctgctcct-3', SEQ ID No: 152) and a CD16 antisense primer (5'-AAAGCGGCCGCCCagtctcttgttgagcttc-3', SEQ ID No: 153), which bind to the CD16 5' terminal and 3' terminal respectively, were synthesized, and PCR was carried out using Ex Taq polymerase (TaKaRa) with the cDNA above as a template. That is, a total of 50 µL of a reaction liquid containing template cDNA (10 ng/µL) 1 µL, 2.5 mM dNTPs 4 µL, primer liquid mixture (50 µM each) 1 µL, ×10 Ex Taq polymerase Buffer 5 µL, Ex Taq polymerase 0.25 µL, and sterile water 38.75 µL was used, and a reaction was carried out at 94°C 1 min; 94°C 45 sec, 55°C 1 min, 72°C 2 min (35 cycles); 72°C 10 min. After the PCR reaction product was subjected to 1% agarose gel electrophoresis, the cDNA fragment was purified using a Wizard SV Gel and PCR Clean-up System (Promega), and further treated with restriction enzymes (EcoRI and NotI) so as to insert it into pIRESpuro2, thus giving the CD16 expression vector CD16/pIRESpuro2. The DNA base sequence of the vector thus obtained was confirmed using an ABI 3100-Avant (Applied Biosystems).

The CD16/pIRESneo2 thus obtained was transduced into CHO-K1 cells (ATCC catalog No. CCL-61) using FuGENE^{®} (Roche) in accordance with the instruction manual. The CHO-K1 cells thus gene-transferred were cultured in 10% FCS (Hyclone)- and penicillin-streptomycin (SIGMA)-containing RPMI1640 medium (SIGMA), and drug selection was carried out by further adding 0.6 mg/mL G418 (Wako Pure Chemical Industries, Ltd.). Cells for which drug resistance was confirmed were subjected to limiting dilution, and the amount of CD16 expression of the single cell clone thus obtained was analyzed by means of a flow cytometer (FACS Caliber, Beckton Dickinson) using anti-CD16 antibody. Cells having high CD16 expression were used as CD16-expressing CHO-K1 cells in the experiments below.

### 3-2. Analysis of CD16 binding reactivity

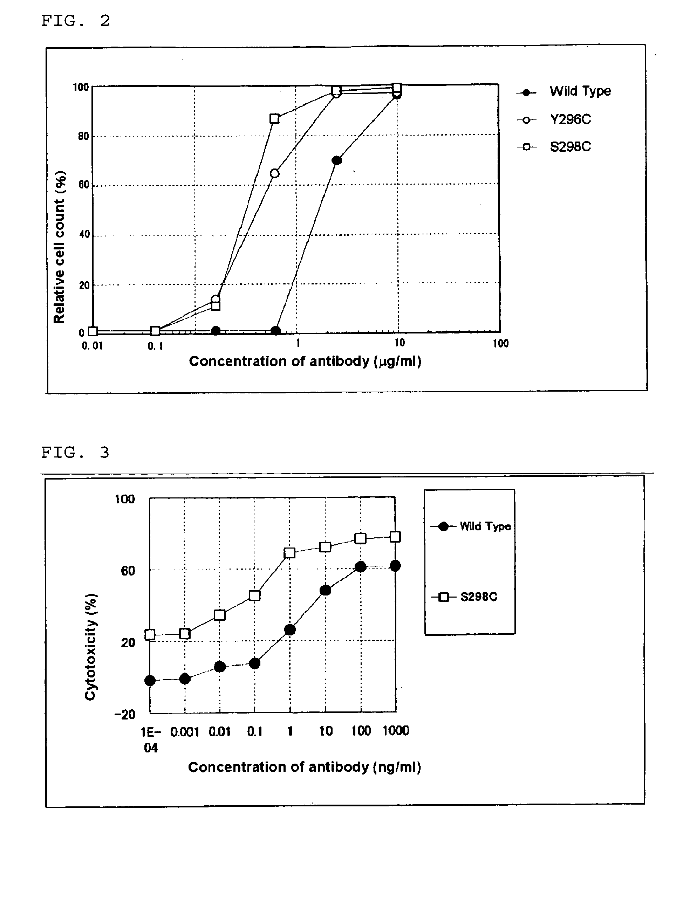

CD16-expressing CHO-K1 cells were taken at 6 × 10⁴ cells/tube, each of the antibody mutants obtained in Example 1 (Tyr296Cys or Ser298Cys) or wild type antibody was added thereto to give a total amount of 50 µL/tube, and a reaction was carried out at 4°C for 1 hour. Following this, washing was carried out with 3 mM EDTA-0.5% BSA-PBS(-) (washing buffer). Subsequently, a biotin-labeled mouse anti-human IgGκ L chain antibody (Vector Lab) diluted 200 times with the washing buffer was added at 100 µL/tube. After a reaction was carried out at 4°C for 30 min, washing was carried out with 3 mM EDTA-0.5% BSA-PBS(-), PE-labeled streptavidin (BD-Pharmingen) diluted 400 times with the washing buffer was added at 100 µL/tube, and a reaction was carried out at 4°C for 30 min. After the reaction was completed, washing was carried out twice with 3 mM EDTA-0.5% BSA-PBS(-), and a CD16-binding anti-CD20 antibody was analyzed using a flow cytometer (FACS Caliber, BecktonDickinson). The results are shown in FIG. 2. From this figure, it is clear that CD16 binding reactivity of all of the mutants was greatly improved compared with that of the wild type. That is, when comparison was made for concentrations that gave the same effect, the Tyr296Cys mutant showed an activity about 5 times that of the wild type, and the Ser298Cys mutant about 10 times that of the wild type. This result shows that the polypeptide of the present invention has a high effector function.

### 4. Analysis of ADCC induction activity

For measurement of ADCC activity, Daudi cells (ATCC catalog No. CCL-213) were used as target cells, and human peripheral blood mononuclear cells obtained in 1. above were used as effector cells. In accordance with a standard method, Daudi cells were suspended in 10% FCS- and 10% WEHI-3 culture supernatant (IL-3)-containing RPMI1640 medium at a concentration of 4 × 10⁵ cells/mL, and 25 µL of each thereof was seeded on a U-bottomed 96-well plate (Falcon). Anti-CD20 chimera antibody amino acid mutant (Ser298Cys) or wild type anti-CD20 chimera antibody was diluted with 10% FCS-containing RPMI1640 medium so as to give a predetermined concentration, 25 µL each thereof was further added to the U-bottomed 96-well plate (Falcon), and a reaction was carried out at 37°C for 1 hour. Following this, peripheral blood mononuclear cells were added at 5 × 10⁵ cells/well and cultured at 37°C for 16 hours. After culturing, the plate was subjected to centrifugation, and 50 µL of supernatant was collected from each well and transferred to a new flat-bottomed 96-well plate. 50 µL of Assay Buffer (CytoTox96^{®} Non-Radioactive Cytotoxicity Assay, Promega) was added to each well, a reaction was carried out at room temperature for 30 min shielded from light, subsequently 50 µL of Stop Buffer (CytoTox96^{®} Non-Radioactive Cytotoxicity Assay) was added, and the OD value at 490 nm was measured.

As a basis for calculating cytotoxicity, wells containing Daudi cells alone or human peripheral blood mononuclear cells alone as a cellular component in a final solution amount of 100 µL were prepared, 10 µL of Lysis Buffer (CytoTox96 Non-Radioactive Cytotoxicity Assay) was added to half of the wells containing Daudi cells alone, and a reaction was carried out for 45 min, thus lysing the cells. Following this, in the same manner as for the experimental conditions group, 50 µL of supernatant was transferred to a new flat-bottomed 96-well plate, 50 µL of Assay Buffer (CytoTox96^{®} Non-Radioactive Cytotoxicity Assay) was added, a reaction was carried out at room temperature for 30 min shielded from light, subsequently 50 µL of Stop Buffer (CytoTox96 Non-Radioactive Cytotoxicity Assay) was added, and the OD value at 490 nm was measured. Here, the total of the OD values for wells containing Daudi cells alone and wells containing human peripheral blood mononuclear cells alone was defined as a cytotoxicity of 0%, a value obtained by subtracting the OD value for wells containing Daudi cells alone to which Lysis Buffer had not been added from the OD value for wells containing Daudi cells alone to which Lysis Buffer had been added was defined as 100%, and a standard line for calculating cytotoxic activity was determined. Cytotoxic activity under various conditions was calculated using this standard line. An experiment was carried out for 3 or more wells for each experimental condition, and the average value and standard error thereof were calculated. The results are shown in FIG. 3. From this figure, it is clear that the polypeptide of the present invention has remarkably high ADCC induction activity compared with the wild type antibody. When comparison was made for concentrations that gave the same cytotoxicity, the polypeptide of the present invention in fact showed an activity of about 200-fold.

## Claims

(1) A polypeptide having an effector function, the polypeptide containing a polypeptide consisting of an amino acid sequence represented by any one of SEQ ID Nos: 1 to 6 as an Fc receptor binding portion, or (2) a polypeptide having an enhanced effector function and containing an Fc receptor binding portion selected from the group consisting of (i) a polypeptide that has one or more mutations in a polypeptide consisting of an amino acid sequence represented by any one of SEQ ID Nos: 1 to 6, (ii) a polypeptide that is coded by a nucleic acid having one or more mutations in the base sequence of a nucleic acid having a base sequence represented by any one of SEQ ID Nos: 7 to 12 or of a nucleic acid coding for the same polypeptide as that coded by the above nucleic acid, (iii) a polypeptide that is coded by a nucleic acid hybridizing under stringent conditions with a complementary strand, or a fragment thereof, of a nucleic acid coding for a polypeptide consisting of an amino acid sequence represented by any one of SEQ ID Nos: 1 to 6 or mutant (i) or (ii), and (iv) a polypeptide that is coded by a nucleic acid having at least 60% homology to a base sequence represented by any one of SEQ ID Nos: 7 to 12.

2. The polypeptide according to Claim 1, wherein the Fc receptor binding portion is an Fc region in which an amino acid selected from the group consisting of, as Kabat EU index numbers, the 293rd amino acid, 294th amino acid, 296th amino acid, 297th amino acid, 298th amino acid, and 300th amino acid is replaced by a cysteine residue.

3. The polypeptide according to Claim 1 or 2, further including a cell binding portion.

4. The polypeptide according to Claim 3, wherein the cell binding portion recognizes or binds to at least one molecule selected from the group consisting of a cytokine receptor, a cell adhesion molecule, a cancer cell surface molecule, a cancer stem cell surface molecule, a blood cell surface molecule, and a virus-infected cell surface molecule.

5. The polypeptide according to Claim 4, wherein the cell binding portion recognizes or binds to at least one molecule selected from the group consisting of antigens CD3, CD11a, CD20, CD22, CD25, CD28, CD33, CD52, Her2/neu, EGF receptor, EpCAM, MUC1, GD3, CEA, CA125, HLA-DR, TNFα receptor, VEGF receptor, CTLA-4, AILIM/ICOS, and an integrin molecule.

6. An isolated nucleic acid coding for the polypeptide according to any one of Claims 1 to 5.

7. A vector containing the nucleic acid according to Claim 6.

8. A host cell or host organism having the vector according to Claim 7.

9. A method for producing a polypeptide, the method comprising culturing the host cell or host organism according to Claim 8 so as to express the polypeptide coded by the nucleic acid.

10. A method for producing in vitro an Fc region-containing polypeptide having a high effector function, the method comprising a step of subjecting an amino acid in the Fc region of the Fc region-containing polypeptide to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid.

11. A polypeptide produced by the method according to Claim 10.

12. An in vitro method for enhancing an effector function of an antibody, the method comprising a step of subjecting an amino acid in an Fc region of the antibody to substitution with a cysteine residue, the amino acid being selected from the group consisting of, as Kabat EU index numbers, the 293rd amino acid, 294th amino acid, 296th amino acid, 297th amino acid, 298th amino acid, and 300th amino acid.

13. An antibody obtained by the method according to Claim 12.

14. A method for preparing in vitro a high effector function antibody-producing cell, the method comprising a step of mutating a gene that codes for an Fc region of the antibody-producing cell so as to replace an amino acid in the Fc region by a cysteine residue, the amino acid being selected from the group consisting of, as Kabat EU index numbers, the 293rd amino acid, 294th amino acid, 296th amino acid, 297th amino acid, 298th amino acid, and 300th amino acid.

15. An antibody-producing cell prepared by the method according to Claim 14.

16. A pharmaceutical composition containing the polypeptide according to any one of Claims 1 to 5 and 11, the antibody according to Claim 13, the nucleic acid according to Claim 6, the vector according to Claim 7, the host cell or host organism according to Claim 8, and/or the cell according to Claim 15.
